# EUROPÄISCHE PATENTSCHRIFT

(11) **EP 3 753 521 B1**
(45) Veröffentlichungstag und Bekanntmachung des Hinweises auf die Patenterteilung: **26.11.2025**
(21) Anmeldenummer: 19181353.4
(22) Anmeldetag: 19.06.2019
(51) Int. Cl.: A61B 34/30, A61B 34/35, A61B 90/25, A61B 90/35, A61B 90/50, B25J 9/16, B25J 13/04, B25J 13/02, B25J 13/06, A61B 90/30, B25J 19/06, A61B 34/00, A61B 90/00

(54) **MEDIZINISCHE HANDHABUNGSVORRICHTUNG ZUR STEUERUNG EINER HANDHABUNGSVORRICHTUNG**
MEDICAL HANDLING DEVICE FOR CONTROLLING A HANDLING DEVICE
DISPOSITIF DE MANIPULATION MÉDICAL D'UN DISPOSITIF DE MANIPULATION

(43) Veröffentlichungstag der Anmeldung: 23.12.2020
(73) Patentinhaber: Karl Storz SE & Co. KG, 78532 Tuttlingen (DE)
(72) Erfinder: Schulze, Marco, 78532 Tuttlingen (DE); Kim, Chang-Hae, 78532 Tuttlingen (DE); Köhler, Benedikt, 78532 Tuttlingen (DE); Schrader, Stephan, 78532 Tuttlingen (DE)
(74) Vertreter: Witte, Weller & Partner Patentanwälte mbB

(56) Entgegenhaltungen:
- EP-A1- 3 119 334
- EP-A1- 3 142 594
- AT-U1- 10 676
- GB-A- 2 568 989
- US-A1- 2018 177 523
- US-A1- 2018 263 710

## Beschreibung

Die vorliegende Offenbarung betrifft eine medizinische Handhabungsvorrichtung und ein Verfahren zur Steuerung einer Handhabungsvorrichtung, wobei die Handhabungsvorrichtung eine robotische Handhabungseinheit aufweist, die einen Instrumentenhalter zur Aufnahme eines Instruments trägt, beispielsweise eines Beobachtungsinstruments, und wobei eine Handhabungssteuereinrichtung zur Steuerung der robotischen Handhabungseinheit vorgesehen ist, die über ein Eingabegerät steuerbar ist.

Aus der US 2018/263710 A1 sind ein Verfahren und eine Vorrichtung zur Verarbeitung chirurgischer Informationen bekannt. Das Verfahren umfasst ein Erfassen einer ersten Positionsinformation einer chirurgischen Bildgebungseinrichtung, wobei die erste Positionsinformation eine Verlagerung der chirurgischen Bildgebungseinrichtung aus einer vorbestimmten Position anzeigt, ein Erzeugen einer zweiten Positionsinformation bezüglich der Bildgebungseinrichtung auf Basis erster Bildinformationen, die in einem Registrierungsmodus von der Bildgebungseinrichtung erlangt werden, und Bestimmen der Position einer chirurgischen Komponente bezüglich der vorbestimmten Position auf Basis der ersten Positionsinformation und der zweiten Positionsinformation. In einem Bildgebungsmodus werden auf Basis der bestimmten Position zweite Bildinformationen der chirurgischen Komponente von der Bildgebungseinrichtung erlangt.

Aus der AT 10 676 U1 sind ein Verfahren zum Betreiben eines mobilen Handbediengerätes, das für die Abgabe oder Freischaltung von potentiell gefahrbringenden Steuerkommandos an eine steuerbare technische Einrichtung vorgesehen ist, sowie ein entsprechend ausgebildetes, mobiles Handbediengerät bekannt. Die AT 10 676 U1 bezieht sich insbesondere auf Werkzeugmaschinen und industrielle Robotern. Es ist eine Sicherheitsschalteinrichtung mit einer Auswerteschaltung und zumindest einem Zustimmtaster vorgesehen, der drei Zustände aufweisen kann, nämlich einen Ruhezustand, einen Zustimmungs-Betätigungszustand sowie einen Panik-Betätigungszustand.

Aus der GB 2 568 989 A ist ein automatisches Positioniersystem bekannt, mit einem mehrgelenkigen Positionierarm; einem mit einem Griff versehenen Endeffektor an einem distalen Ende des Positionierungsarms; einem mit dem Endeffektor gekoppelten Kraft-Momenten-Sensor; und mit einer Steuerung, die mit dem Positionierarm und dem Kraft-Momenten-Sensor kommuniziert, um unter Verwendung von Sensor-Signalen zumindest eine externe Kraft oder ein externes Drehmoment auf den Endeffektor zu bestimmen, auf dieser Basies eine Antriebsgeschwindigkeit zum Bewegen des Endeffektors zu bestimmen, Gelenkbewegungen des Positionierarms zum Bewegen des Endeffektors gemäß der Antriebsgeschwindigkeit zur berechnen, und den Positionierarm zu veranlassen, sich gemäß den berechneten Gelenkbewegungen zu bewegen, wobei die Antriebsgeschwindigkeit gemäß einem gewählten Steuerungsmodus bestimmt wird.

Aus der US 2018/177523 A1 ist eine Armbaugruppe zur Verwendung bei einer medizinischen Prozedur bekannt. Die Armbaugruppe umfasst eine Basis und einen mit der Basis gekoppelten Mehrgelenkarm mit einem distalen Ende, mit dem ein Endeffektor koppelbar ist. Der Mehrgelenkarm ist manuell bewegbar, wobei zu diesem Zweck ein Griff vorgesehen ist. Die Armbaugruppe umfasst ferner einen Bildaufnehmer, der mit dem Endeffektor koppelbar ist. Ferner ist ein Gegengewicht vorgesehen, das mit dem Mehrgelenkarm gekoppelt ist, wobei das Gegengewicht einstellbar ist, um Gewichtsänderungen des Mehrgelenkarms bzw. des Bildaufnehmers zu kompensieren.

Aus der WO 2015/142512 A1 ist ein Chirurgieroboter bekannt, der über eine Bedienkonsole steuerbar ist, die als Fußpedale gestaltete Aktivierungsschalter umfasst. Aus der WO 2015/175278 A1 ist ein Chirurgieroboter bekannt, der über eine Bedienkonsole steuerbar ist, die einen Auswahlschalter umfasst, der wahlweise als Fußpedal gestaltet sein kann.

Aus der DE 10 2013 110 543 A1 ist ein als Beobachtungsinstrument gestaltetes medizinisches Instrument bekannt, das zum Aufnehmen eines Bildes eines Objektfelds an einem menschlichen oder tierischen Körper von außerhalb des Körpers ausgebildet ist, wobei das Instrument einen Schaft und eine an einem distalen Ende des Schafts angeordnete Beobachtungsoptik zum Aufnehmen des Bildes des Objektfelds aufweist, wobei die Beobachtungsoptik als Stereooptik mit mindestens einem elektronischen Bildaufnehmer zum Aufnehmen eines Stereobilds des Objektfelds ausgebildet ist, und wobei das Instrument eine Optikeinheit aufweist, die die Beobachtungsoptik umfasst, und die um eine zu einer Blickrichtung der Beobachtungsoptik näherungsweise parallele erste Drehachse drehbar ist.

Ein solches Beobachtungsinstrument zur Beobachtung eines Objektfeldes von außerhalb des Körpers wird als Exoskop bezeichnet. Ferner sind Instrumente, insbesondere Beobachtungsinstrumente, bekannt, die als Endoskope zum Aufnehmen eines Bildes innerhalb des menschlichen oder tierischen Körpers gestaltet sind.

Aus der DE 10 2015 121 017 A1 ist eine Beobachtungsvorrichtung bekannt, die ein Beobachtungsinstrument und ein als Mehrachs-Eingabemodul gestaltetes Eingabegerät zur Steuerung des Beobachtungsinstruments aufweist. Das Eingabegerät ist ähnlich einer sogenannten Space-Mouse gestaltet. Die DE 10 2015 121 017 A1 lehrt, das Eingabegerät sowohl zur Steuerung von Bildaufnahmeparametern als auch zur Steuerung von Bildwiedergabeparametern zu verwenden. Ein Bildaufnahmeparameter betrifft etwa eine Fokusverstellung. Ein Bildwiedergabeparameter betrifft etwa einen digitalen Zoom.

Im Sinne der vorliegenden Offenbarung ist ein distales Ende eines Elements ein einem Beobachtungsobjekt, etwa einem Patienten, zugewandtes Ende. Demgegenüber ist ein proximales Ende des Elements ein dem distalen Ende und folglich auch dem Beobachtungsobjekt abgewandtes Element. Bei einem handgeführten Instrument ist das proximale Ende regelmäßig dem Bediener des Instruments zugewandt. Bei einem mittels einer Handhabungseinheit geführten Instrument ist das Instrument zuweilen - aber nicht zwangsläufig - im Bereich seines proximalen Endes an der Handhabungseinheit aufgenommen, etwa an einem Gehäuse.

Ferner sind sogenannte Teleoperationssysteme oder Telemanipulationssysteme bekannt, etwa aus der US 5,696,837 A, bei denen ein Instrument in Form eines Beobachtungsinstruments oder dergleichen über einen Manipulator gehalten und ferngesteuert wird.

Medizinische Instrumente, etwa Beobachtungsinstrumente in Form eines Endoskops oder eines Exoskopes, können grundsätzlich handgehalten bzw. handgeführt sein. Dies hat den Vorteil, dass der Benutzer intuitiv und unmittelbar die Blickrichtung, das Objektfeld bzw. den Bildausschnitt und weitere Parameter der Bilderfassung anpassen kann, indem das Instrument entsprechend im Raum positioniert wird.

Es sind jedoch auch Systeme bekannt, bei denen Instrumente nicht handgehalten oder handgeführt werden, sondern an einem Stativ oder einem Ausleger aufgenommen sind. Dies hat den Vorteil, dass kein Bediener erforderlich ist, der das Instrument händisch in der gewünschten Position und Orientierung halten muss. Hierbei ist es vorstellbar, dass das Instrument ortsunveränderlich angeordnet ist, um beispielsweise während einer Operation permanent ein- und denselben Bildausschnitt in einem vorgewählten Objektfeld zu beobachten.

Darüber hinaus ist es auch vorstellbar, das Instrument an einer Handhabungseinheit bzw. einem Manipulator (auch als motorisches Haltesystem bzw. Roboter bezeichnet) anzuordnen, um Bewegungsfreiheitsgrade der Handhabungseinheit zur Bewegung und Ausrichtung des Instruments zu nutzen.

Auf diese Weise kann auch bei einem nicht unmittelbar handgehaltenen oder handgeführten Instrument eine Änderung der Position, Orientierung und/oder des Bildausschnitts erfolgen. Hierfür ist jedoch eine Bedienung erforderlich, um die gewünschte Bewegung des Instruments zu veranlassen.

Häufig sind jedoch bereits Bedienelemente für die Instrumente als solches vorgesehen, etwa Bedienelemente zur Steuerung von Bildaufnahmeparametern und/oder Bildwiedergabeparametern eines Bilderfassungssystems, welches ein Beobachtungsinstrument mit Bildaufnehmer und eine entsprechende Anzeige zur Bildwiedergabe aufweist. Das heißt, selbst ohne zusätzliche Bewegung des Instruments sind bereits diverse Bedienoperationen vorstellbar, für welche Bedienelemente vorgesehen sind.

Bei der Automatisierung bzw. maschinellen Unterstützung medizinischer Tätigkeiten ist jedoch darauf zu achten, dass die Systeme als solches noch intuitiv, einfach und sicher bedienbar sind. Insbesondere bei telemedizinischen Systemen bzw. robotischen Systemen muss bedacht werden, dass häufig keine unmittelbare Rückkopplung an den Bediener möglich ist. Dies führt etwa im Vergleich zur rein manuellen handgeführten Betätigung unter Umständen zu Fehlbedienungen, wenn dem Bediener nicht unmittelbar klar ist, welche Aktivität mit dem aktuell vorgenommenen Bedienbefehl ausgelöst wird.

Vor diesem Hintergrund liegt der vorliegenden Offenbarung die Aufgabe zugrunde, eine medizinische Handhabungsvorrichtung sowie ein Verfahren zur Steuerung einer Handhabungsvorrichtung anzugeben, die eine intuitive und fehlerarme Steuerung einer Mehrzahl von Funktionen ermöglichen. Vorzugsweise kann mit einer überschaubaren Anzahl an Eingabegeräten bzw. Eingabemöglichkeiten eine Mehrzahl unterschiedlicher Funktionen gesteuert werden. Vorzugweise kann dies derart erfolgen, dass es keine nachteiligen Wechselwirkungen/Überschneidungen gibt. Die Bedienung ist vorzugsweise hinreichend eindeutig, um die Gefahr von Fehlbedienungen zu verringern.

Vorzugsweise ist die Handhabungsvorrichtung derart gestaltet, dass bei der Bedienung/Steuerung keine übermäßige Ablenkung von der eigentlichen Tätigkeit gegeben ist.

Ferner soll im Rahmen der vorliegenden Offenbarung eine Handhabungsvorrichtung angegeben werden, welche die Arbeitsbedingungen für den Benutzer/Operateur optimiert und dazu beiträgt, dass der Benutzer die Übersicht behält.

Gemäß einem ersten Aspekt der vorliegenden Offenbarung wird diese Aufgabe durch eine medizinische Handhabungsvorrichtung gelöst, die Folgendes aufweist:
- einen Instrumentenhalter zur Aufnahme eines Instruments,
- eine robotische Handhabungseinheit, die den Instrumentenhalter trägt,
- eine Steuereinrichtung mit einer Handhabungssteuereinheit zur Steuerung der robotischen Handhabungseinheit und einer Instrumentensteuereinheit zur Steuerung des Instruments,
   wobei die Steuereinrichtung eine Schnittstelle für zumindest ein Eingabegerät aufweist, und
- ein mit der Steuereinrichtung koppelbares Eingabegerät, das mit der Schnittstelle gekoppelt ist,
   wobei das Eingabegerät in einem ersten Betriebsmodus zur Steuerung des Instruments und in einem zweiten Betriebsmodus zur Steuerung der robotischen Handhabungseinheit nutzbar ist,
   wobei die Steuereinrichtung mit dem Instrument und mit der robotischen Handhabungseinheit koppelbar ist,
   wobei die Steuereinrichtung dem Eingabegerät und dem am Instrumentenhalter aufgenommenen Instrument zwischengeschaltet ist, und
   wobei die Steuereinrichtung dazu ausgebildet ist, im zweiten Betriebsmodus über das Eingabegerät erfasste Steuerbefehle für die robotische Handhabungseinheit der Handhabungssteuereinheit über die Instrumentensteuereinheit bereitzustellen,
- einen als Fußschalter gestalteten Freigabeschalter zur Aktivierung des zweiten Betriebsmodus, in dem die robotische Handhabungseinheit in Reaktion auf Eingabebefehle am Eingabegerät motorisch verfahrbar ist, um das Instrument motorisch zu bewegen,
   wobei der Freigabeschalter zwei Zustände aufweist, wobei ein erster Zustand in Ursprungszustand ist, und wobei ein zweiter Zustand ein Aktivierungszustand für den zweiten Betriebsmodus ist,
   wobei der Freigabeschalter einen dritten Zustand aufweist, wobei der Freigabeschalter bei Aktivierung ausgehend vom ersten Zustand durch Aufbringen einer Betätigungskraft zunächst in den zweiten Zustand und danach bei erhöhter Betätigungskraft in den dritten Zustand überführbar ist, und
   wobei sowohl im ersten Zustand als auch im dritten Zustand des Freigabeschalters die Steuerung der robotischen Handhabungseinheit gesperrt ist.

Die der Offenbarung zugrunde liegende Aufgabe wird auf diese Weise vollkommen gelöst.

Erfindungsgemäß wird nämlich durch den Freigabeschalter und die Gestaltung der Steuereinrichtung gewährleistet, dass der Bediener die Handhabungsvorrichtung nur beabsichtigt und nicht unbewusst im zweiten Betriebsmodus betreibt. Dies erhöht die Sicherheit beim Betrieb der Handhabungsvorrichtung. Im ersten Betriebsmodus kann der Bediener im Wesentlichen Funktionen des am Instrumentenhalter aufgenommenen Instruments steuern. Dies kann etwa bei einem Beobachtungsinstrument Bildaufnahmeparameter und/oder Bildwiedergabeparameter betreffen, welche unabhängig von der robotischen Handhabungseinheit steuerbar sind.

Im zweiten Betriebsmodus wird hingegen die robotische Handhabungseinheit benutzt, beispielsweise um die Position und/oder Orientierung des Instruments im Raum zu verändern. Dies kann bei beispielhaften Nutzungen zur medizinischen Diagnose und/oder Behandlung in unmittelbarer Nähe eines Patienten eben auch Relativbewegungen des Instruments in Bezug auf den Patienten umfassen. Hingegen umfasst der erste Betriebsmodus keine oder weitgehend keine Relativbewegung zwischen dem Instrument und dem Patienten. Demgemäß besteht primär im zweiten Betriebsmodus ein gewisses Gefährdungspotenzial durch Bewegungen des Instruments. Der zweite Betriebsmodus wird beispielsweise genutzt, um bei Verwendung eines Beobachtungsinstruments einen angezeigten Bildausschnitt durch Bewegung des Instruments relativ zum Beobachtungsobjekt zu verändern.

Vor diesem Hintergrund ist daher vorgesehen, dass der Bediener den zweiten Betriebsmodus nur bewusst durch Betätigung des Freigabeschalters aktivieren kann. Auf diese Weise wird verhindert, dass der Bediener im ersten Betriebsmodus unbewusst die robotische Handhabungseinheit zur Steuerung (Bewegung) des Instruments ansteuert. Das Sicherheitsniveau bei der Bedienung der Handhabungsvorrichtung erhöht sich.

Gleichwohl kann der Bediener mit ein und demselben Eingabegerät diverse Funktionen der robotischen Handhabungsvorrichtung steuern. Dies gilt für Funktionen der robotischen Handhabungseinheit, die über die Handhabungssteuereinheit gesteuert werden. Dies gilt jedoch auch für Funktionen des Instruments, die die durch die Instrumentensteuereinheit gesteuert werden.

Auf diese Weise wird eine Trennung zwischen Funktionen, bei denen die Handhabungseinheit womöglich oder auf jeden Fall bewegt wird, und Funktionen, bei denen die Handhabungseinheit nicht bewegt wird, möglich. Dies erhöht die Sicherheit. Dies gilt insbesondere dann, wenn der Bediener die Handhabungseinheit mittelbar (zum Beispiel ohne direkte Sicht auf das Instrument) steuert und sich dabei an einem angezeigten Bild und nicht unbedingt an den konkreten Bewegungen von Elementen der Handhabungseinheit und dem Instrument orientiert.

Die robotische Handhabungseinheit kann auch als telemedizinische Handhabungseinheit bezeichnet werden. Auch wenn es grundsätzlich vorstellbar ist, die Handhabungseinheit voll- oder teilautomatisiert zu betreiben, so ist jedoch zumindest in beispielhaften Ausführungsformen eine Steuerung durch den Bediener/Operateur vorgesehen.

Generell kann das Eingabegerät bei der Verwendung eines Beobachtungsinstruments dazu genutzt werden, um über Bedienereingaben den angezeigten Bildausschnitt zu manipulieren. Dies kann eine Bewegung (Verschiebung oder Schwenkbewegung) umfassen. Vorstellbar ist jedoch auch eine Drehung (Rotation) sowie eine Vergrößerung/Verkleinerung (Zoom bzw. Veränderung des Abbildungsmaßstabs). Ferner ist es vorstellbar, über das Eingabegerät einen Fokus-Antrieb zur Verstellung einer Fokusebene (Schärfeebene) anzusteuern.

Das Eingabegerät ist zur Steuerung des Instruments aber auch zur Steuerung der robotischen Handhabungseinheit nutzbar. Dies vereinfacht die Bedienung für den Bediener deutlich. Es ist nicht unbedingt nötig, zwei separate Eingabegeräte zu nutzen.

Die Instrumentensteuereinheit kann als CCU/Controller/Konsole bezeichnet werden. Das Eingabegerät für die Manipulation (Bewegung) des Bildausschnitts ist in beispielhaften Ausgestaltungen über eine Schnittstelle an die Instrumentensteuereinheit angekoppelt. Dies heißt mit anderen Worten, Steuerbefehle für die Handhabungssteuereinheit werden (signaltechnisch) vom Eingabegerät über die Instrumentensteuereinheit an die Handhabungseinheit übergeben. Ferner kann der Status der Bildeinstellung (Zoom, Focus, ROI Position und Bildorientierung) der zu bewegenden Einheit (z.B. Exoskop) über die CCU an die Handhabungseinheit weitergegeben werden. Die Handhabungseinheit kann dann entsprechend darauf reagieren, beispielsweise durch Änderung der Bewegungsrichtung bei geänderter Orientierungseinstellung, oder durch Änderung des Pivotierungsradius bei geänderter Fokuseinstellung.

Die Steuereinrichtung kann verteilt sein und demgemäß eine Instrumentensteuereinheit zur Steuerung des Instruments und eine separate Handhabungssteuereinheit zur Steuerung der robotischen Handhabungseinheit umfassen, die miteinander kommunizieren. Es versteht sich, dass die verteilte Gestaltung auch virtuell (softwaremäßig) realisiert werden kann. Gleichwohl ist zumindest in beispielhaften Ausführungsformen eine hardwaremäßige Trennung von Instrumentensteuereinheit und Handhabungssteuereinheit vorstellbar. Auf diese Weise bleibt die Instrumentensteuereinheit (CCU/Konsole) universell nutzbar, also auch für handgehaltene/handgeführte Instrumente.

Gemäß einer beispielhaften Ausgestaltung der Handhabungsvorrichtung ist das Instrument als Beobachtungsinstrument ausgestaltet. Beispielsweise handelt es sich bei dem Beobachtungsinstrument um ein Instrument zur Beobachtung des Körpers von außerhalb des Körpers. Dies ist nicht einschränkend zu verstehen. Gleichwohl ist es auch vorstellbar, andere Instrumente, die keine Beobachtungsinstrumente sind, am Instrumentenhalter der robotischen Handhabungseinheit aufzunehmen.

Das Eingabegerät ist mit der Steuereinrichtung koppelbar, wobei die Steuereinrichtung mit dem Instrument und mit der robotischen Handhabungseinheit koppelbar ist, und wobei die Steuereinrichtung dem Eingabegerät und dem am Instrumentenhalter aufgenommenen Instrument zwischengeschaltet ist. Mit anderen Worten ist die Steuereinrichtung in beispielhaften Ausgestaltungen dem Eingabegerät und dem Instrument signaltechnisch zwischengeordnet. In dieser Ausgestaltung erfolgt der Signalaustausch zwischen dem Eingabegerät und dem Instrument unter Nutzung der Steuereinrichtung.

Sowohl der erste Betriebsmodus als auch der zweite Betriebsmodus nutzen gleiche Leitungen und Signalwege, zumindest in beispielhaften Ausgestaltungen.

Gemäß einer weiteren beispielhaften Ausgestaltung der Handhabungsvorrichtung ist die Steuereinrichtung signaltechnisch dem Eingabegerät und der robotischen Handhabungseinheit zwischengeordnet. Ferner ist in dieser Ausgestaltung die Instrumentensteuereinheit - signaltechnisch - zwischen dem Beobachtungsinstrument und dem Eingabegerät angeordnet. Signale werden über die Instrumentensteuereinheit weitergeleitet bzw. gegebenenfalls sogar durchgeschliffen.

In einer beispielhaften Ausgestaltung ist ferner vorgesehen, dass das Eingabegerät (für den Fall der Steuerung eines Bildaufnahmeparameters direkt am Beobachtungsinstrument) signaltechnisch über die robotische Handhabungseinheit mit dem Beobachtungsinstrument verbindbar ist.

Ein Aspekt der vorliegenden Offenbarung beruht darauf, dass die Handhabungssteuereinheit die robotische Handhabungseinheit in Abhängigkeit von Parametern des Beobachtungsinstruments steuert. Dies kann insbesondere Parameter eines Beobachtungskopfes/Kamerakopfes des Beobachtungsinstruments betreffen. Beispielsweise kann die Handhabungssteuereinheit die Geschwindigkeit der Bewegung der Glieder der robotischen Handhabungseinheit in Abhängigkeit von einer gegebenen Vergrößerungsstufe (Zoom-Faktor, Fokusabstand bzw. Objektabstand). So kann etwa bei einem großen Zoom-Faktor bzw. einem kleinen Objektabstand (entsprechend einer Detaildarstellung) die Verfahrgeschwindigkeit der robotischen Handhabungseinheit verringert werden. Demgemäß kann etwa bei einem kleinen Zoom-Faktor bzw. einem großen Objektabstand (entsprechend einer Übersichtsdarstellung) die Verfahrgeschwindigkeit der robotischen Handhabungseinheit erhöht werden.

Ein Aspekt der vorliegenden Offenbarung betrifft die Nutzung von Parametern oder Kennwerten, die das Beobachtungsinstrument und dessen Betrieb kennzeichnen, für die Steuerung der robotischen Handhabungseinheit durch die Handhabungssteuereinheit. Es gibt also bei den genutzten Signalen und Informationen Überschneidungen und gegebenenfalls eine wechselseitige Beeinflussung.

Gleichwohl ist zumindest in beispielhaften Ausgestaltungen vorgesehen, dass das Beobachtungsinstrument und die Instrumentensteuereinheit gelöst von der Handhabungsvorrichtung separat nutzbar sind. Dies gilt insbesondere für sogenannte handgehaltene/handgeführte Anwendungen. Somit können einerseits Synergien zwischen der Handhabungseinheit und dem Beobachtungsinstrument bzw. zwischen der Handhabungssteuereinheit und der Instrumentensteuereinheit genutzt werden. Andererseits kann das Beobachtungsinstrument weiterhin autark benutzt werden, ohne dass zwingend die Handhabungseinheit vorgesehen sein muss.

Der Freigabeschalter weist zwei Zustände 0 und I auf, wobei ein erster Zustand 0 ein Ursprungszustand ist, und wobei ein zweiter Zustand I ein Aktivierungszustand für den zweiten Betriebsmodus ist. Demgemäß weist der Freigabeschalter zumindest ein Eingabeelement mit zwei (oder mehr) Schaltstufen auf. Der erste Zustand 0 entspricht beispielhaft einem Zustand, in dem keine Einwirkung/Betätigung durch den Bediener auf den Freigabeschalter erfolgt.

Durch eine Einwirkung auf das Eingabeelement des Freigabeschalters kann der Freigabeschalter vom ersten Zustand 0 in den zweiten Zustand I gebracht werden. Im zweiten Zustand ist der zweite Betriebsmodus aktiviert, sodass der Bediener die Handhabungseinheit über das Eingabegerät steuern kann.

Der Freigabeschalter weist ferner einen dritten Zustand II auf, wobei der Freigabeschalter bei Aktivierung ausgehend vom ersten Zustand 0 durch Aufbringen einer Betätigungskraft zunächst in den zweiten Zustand I und danach bei erhöhter Betätigungskraft in den dritten Zustand II überführbar ist, und wobei sowohl im ersten Zustand 0 als auch im dritten Zustand II des Freigabeschalters die Steuerung der robotischen Handhabungseinheit gesperrt ist. Auf diese Weise wird die Betriebssicherheit weiter erhöht. Diese Funktion kann auch als Panik-Funktion bezeichnet werden. Beispielsweise dann, wenn der Bediener eine zu hohe Betätigungskraft aufbringt, wird der Freigabeschalter in den dritten Zustand II überführt, in dem der zweite Betriebsmodus (zur Steuerung der Handhabungseinheit über das Eingabegerät) deaktiviert ist.

Damit werden beispielsweise solche Situationen berücksichtigt, in denen der Bediener unbeabsichtigt auf den Freigabeschalter einwirkt. Dies kann etwa bei einem Fußschalter dadurch geschehen, dass der Bediener ohne Absicht auf das Eingabeelement des Fußschalters tritt. Mit anderen Worten muss der Bediener den Freigabeschalter im zweiten Zustand I halten, um die Handhabungseinheit zum Verfahren des Instruments über das Eingabegerät steuern zu können. Wenn also eine höhere Kraft aufgebracht wird, so erfolgt eine Deaktivierung des zweiten Betriebsmodus. Dies erfolgt eben auch dann, wenn unbewusst mit hoher Kraft auf den Freigabeschalter eingewirkt wird.

Mit anderen Worten wird beispielsweise ein Kraftbereich mit einer oberen Grenze und einer unteren Grenze definiert, in dem der Freigabeschalter im zweiten Zustand I ist, um den zweiten Betriebsmodus freizugeben. Wenn der Bediener mit einer geringeren Kraft auf den Freigabeschalter einwirkt, so verbleibt dieser im ersten Zustand 0. Der zweite Betriebsmodus ist nicht freigegeben. Umgekehrt, wenn der Bediener mit einer höheren Kraft auf den Freigabeschalter einwirkt, so wird die obere Grenze des Kraftbereichs überschritten. Der Freigabeschalter wird in den dritten Zustand II überführt. Der zweite Betriebsmodus ist nicht freigegeben bzw. der zweite Betriebsmodus wird deaktiviert. Auf diese Weise kann die Gefahr von Fehlbedienungen weiter reduziert werden.

Gemäß einer weiteren beispielhaften Ausgestaltung der Handhabungsvorrichtung liegt der zweite Zustand I des Freigabeschalters, bezogen auf Schaltstellungen eines Eingabeelements des Freigabeschalters, zwischen dem ersten Zustand 0 und dem dritten Zustand II.

Gemäß einer weiteren beispielhaften Ausgestaltung der Handhabungsvorrichtung ist der Freigabeschalter direkt mit der Handhabungssteuereinheit der Steuereinrichtung verbunden, die der robotischen Handhabungseinheit zugeordnet ist. Auf diese Weise wird eine proprietäre Sicherheitseinrichtung (Freigabesteuerung) geschaffen, die nicht einfach softwaremäßig umgangen/deaktiviert werden kann. Dies erhöht die Sicherheit weiter.

Beispielhaft ist der Freigabeschalter fest mit der Steuereinrichtung der Handhabungsvorrichtung verdrahtet, insbesondere mit einer dortigen Sicherheitseinrichtung. Der Freigabeschalter ist - signaltechnisch - direkt mit der Sicherheitseinrichtung/Freigabesteuerung gekoppelt. Dies verringert die Gefahr von Manipulationen. Mit anderen Worten ist es vorstellbar, den Freigabeschalter und/oder die Sicherheitseinrichtung diskret zu gestalten (durch diskrete und gegebenenfalls proprietäre Elemente).

Die Steuereinrichtung ist dazu ausgebildet, im zweiten Betriebsmodus über das Eingabegerät erfasste Steuerbefehle für die robotische Handhabungseinheit der Handhabungssteuereinheit über die Instrumentensteuereinheit bereitzustellen. Damit wird dem Umstand Rechnung getragen, dass das Instrument, beispielsweise das Beobachtungsinstrument, grundsätzlich auch ohne die robotische Handhabungseinheit verwendbar ist, etwa für handgehaltene/handgeführte Anwendungen. In einem solchen Fall wird das Eingabegerät primär zur Instrumentensteuerung genutzt. Insofern ist in beispielhaften Ausgestaltungen eine Kopplung des Eingabegerätes mit der Instrumentensteuereinheit vorgesehen. Die Nutzung dieses Eingabegerätes zur Steuerung der Handhabungseinheit, also zur Erzeugung von Bediensignalen, die durch die Handhabungssteuereinheit verarbeitet werden, findet im zweiten Betriebsmodus statt und wird durch den Freigabeschalter ermöglicht.

Gemäß einer weiteren beispielhaften Ausgestaltung der Handhabungsvorrichtung werden die Steuerbefehle für die robotische Handhabungseinheit im zweiten Betriebsmodus über die Instrumentensteuereinheit übermittelt, welche die Steuerbefehle weiterleitet. Auch damit wird dem Umstand Rechnung getragen, dass das Instrument zumindest in beispielhaften Ausgestaltungen auch autark ohne die robotische Handhabungseinheit nutzbar ist. Gleichwohl ergibt sich ein einfacher Aufbau der Steuereinrichtung, wobei die Bediensicherheit und die Vermeidung von Fehlbedienungen hinreichend berücksichtigt werden.

Gemäß einer weiteren beispielhaften Ausgestaltung der Handhabungsvorrichtung ist das Eingabegerät als Mehrachs-Eingabegerät gestaltet, wobei das Eingabegerät Bedienbewegungen in Form von Schubbewegungen oder Schwenkbewegungen in zumindest zwei Achsen erfasst, und wobei die Bedienbewegungen in Steuerbefehle für die robotische Handhabungseinheit überführt werden.

Gemäß einer weiteren beispielhaften Ausgestaltung der Handhabungsvorrichtung umfasst die robotische Handhabungseinheit eine mehrgliedrige Kinematik mit einer Mehrzahl von Koppelgliedern, wobei die Steuereinrichtung dazu ausgebildet ist, die Bewegungsvorgaben mittels Interpolation in Steuerbefehle für Bewegungsachsen der Koppelglieder zu überführen. Auf diese Weise kann auch eine mehrachsige Handhabungseinheit in einfacher Weise durch Bedienung am Eingabegerät gesteuert werden. Es handelt sich bei der mehrgliedrigen Kinematik beispielsweise um eine serielle Kinematik. Es versteht sich, dass auch eine parallele oder gemischt seriell-parallele Kinematik verwendbar ist.

Gemäß einer weiteren beispielhaften Ausgestaltung der Handhabungsvorrichtung umfasst die robotische Handhabungseinheit eine serielle Kinematik mit einer Mehrzahl von Koppelgliedern, welche von der Handhabungssteuereinheit der Steuereinrichtung gesteuert werden. Somit kann das Beobachtungsinstrument im gegebenen Raum mit großer Bewegungsfreiheit kontrolliert verfahren werden.

Gemäß einer weiteren beispielhaften Ausgestaltung der Handhabungsvorrichtung ist die Steuereinrichtung dazu ausgebildet, eine aktuelle Position und Orientierung des Instruments zu speichern und bedarfsweise aufzurufen. Diese Funktion kann auch als Save-and-Recall (Speichern-und-Abrufen) Funktion bezeichnet werden. Auf diese Weise kann der Bediener zumindest einen Wegpunkt setzen, der bedarfsweise aus einer zwischenzeitlich eingenommenen Position des Instruments wieder heranfahrbar ist. Es ist vorstellbar, hierfür am Eingabegerät spezielle Funktionstasten vorzusehen, um die Position zu speichern und später abzurufen.

Diese Funktion kann auch als Recall-Funktion bezeichnet werden. Zudem ist es vorstellbar, dass bei gleichzeitiger Verwendung eines Beobachtungsinstrumentes neben der räumlichen Position des Instruments auch dessen Bildparameter (beispielsweise Zoom, Fokus-Wert und ROI-Position) gespeichert werden und bedarfsweise bei entsprechendem Befehl ("Recall") wieder abgerufen und eingestellt werden. Dies gilt auch für Parameter solcher Instrumente, die keine Beobachtungsinstrumente sind.

Generell kann die Recall-Funktion weitere Parameter, beispielsweise Parameter mit Bezug auf Fokusstellung, Zoom, Bildausschnitt (bei Pan-Funktion), sowie weitere Bildeinstellungen wie Kontrast, Helligkeit und Filter betreffen. Das heißt, dass auch andere Parameter des Instrumentes oder Beobachtungsinstruments mit dieser Funktion gespeichert und bedarfsweise abgerufen werden können.

Diese Funktion kann an die Freigabe über den Freigabeschalter gekoppelt sein. Es ist also vorstellbar, die Save-and-Recall nur dann zuzulassen, wenn der Freigabeschalter den zweiten Betriebsmodus der Handhabungsvorrichtung freigibt.

In einer beispielhaften Ausgestaltung umfasst diese Funktion das bedarfsweise Speichern und Anfahren bestimmter Positionen und Orientierungen, ohne dass der hierbei zurückgelegte Pfad definiert ist. In einer weiteren beispielhaften Ausgestaltung umfasst diese Funktion gewissermaßen eine "Rückwärtsfahrt", bei der die Handhabungseinheit nicht nur die Position/Orientierung anfährt, sondern auch den zwischenzeitlichen Bewegungspfad in entgegengesetzter Richtung abfährt. Etwas Derartiges kann beispielsweise in einem sogenannten Direktsteuermodus verwendet werden.

Gemäß einer weiteren beispielhaften Ausgestaltung der Handhabungsvorrichtung ist die Steuereinrichtung dazu ausgebildet, eine Mehrzahl von vorgegebenen Positionen und Orientierungen des Instruments abzuspeichern, um diese bedarfsweise aufzurufen. Auf diese Weise kann die Save-and-Recall Funktion mehrere Positionen/Orientierungen bedarfsweise anfahren. Die Steuerung kann über ein Eingabegerät erfolgen. Grundsätzlich ist es vorstellbar, mehrere Eingabegeräte zu verwenden, also beispielsweise auch einen Touch-Monitor und/oder eine Tastatur. Auf diese Weise können auch Auswahlentscheidungen mit mehreren Wahlmöglichkeiten einfach und direkt getroffen werden.

Gemäß einer weiteren beispielhaften Ausgestaltung der Handhabungsvorrichtung ist die Steuereinrichtung dazu ausgestaltet, die robotische Handhabungseinheit in einem Direktsteuermodus zu betreiben, um das Instrument im Raum zu bewegen und auszurichten, wobei Bedienbefehle an der robotischen Handhabungseinheit durch Einwirkung auf ein dem Instrument benachbartes Element der Handhabungseinheit erzeugbar sind, und wobei die Handhabungssteuereinheit dazu ausgestaltet ist, die robotische Handhabungseinheit derart anzusteuern, dass das Beobachtungsinstrument der induzierten Bewegung folgt.

Ein solcher Direktsteuermodus ("Direct Drag Mode") kann insbesondere für die Grobpositionierung des Beobachtungsinstruments in Bezug auf das Objekt bzw. das Objektfeld genutzt werden. Sofern die robotische Handhabungseinheit in geeigneter Weise angesteuert wird, ergibt sich eine quasi-manuelle Verstellung direkt am Instrumentenhalter oder zumindest in der Nachbarschaft des Beobachtungsinstruments.

In einem solchen Modus kann folglich der Instrumentenhalter mit dem daran aufgenommenen Beobachtungsinstrument quasi-manuell im Raum bewegt und ausgerichtet werden, wobei die Steuereinrichtung dazu ausgebildet ist, die robotische Handhabungseinheit jeweils selbsttätig in der aktuellen Position/Orientierung zu halten, aber eine manuelle (quasi-manuell) Bewegung durch direktes Greifen und Bewegen zu ermöglichen

Es ist vorstellbar, die Antriebe der Elemente/Glieder der kinematischen Kette der robotischen Handhabungseinheit im Direktsteuermodus derart zu überwachen, dass die Bedienbefehle erfasst werden. Somit kann besagte "Folgebewegung" erzeugt werden. Mit anderen Worten dient die robotische Handhabungseinheit im Direktsteuermodus selbst als Eingabegerät.

In diesem Modus ist es aus Sicht der robotischen Handhabungseinheit nicht unbedingt notwendig, Informationen betreffend das Beobachtungsinstrument umfangreich abzufragen, da ja die Bewegung direkt an der robotischen Handhabungseinheit manuell induziert und gesteuert wird.

Der Direktsteuermodus kann einen direkten Kraftangriff durch den Bediener an einem Glied der robotischen Handhabungseinheit umfassen, wobei das Glied vorzugsweise dem Instrumentenhalter benachbart ist. Auf diese Weise dient die robotische Handhabungseinheit selbst als Eingabegerät.

Gemäß einer weiteren beispielhaften Ausgestaltung der Handhabungsvorrichtung ist ein Bedienelement zur Steuerung der robotischen Handhabungseinheit im Direktsteuermodus vorgesehen, wobei das Bedienelement einen Sensor zur Erzeugung eines Freigabesignals für den Direktsteuermodus aufweist. Das heißt mit anderen Worten, für den Direktsteuermodus ist nicht unbedingt eine Freigabe über den Freigabeschalter für den zweiten Betriebsmodus erforderlich. Beispielsweise ist der Sensor als eine Art Näherungssensor oder Berührsensor gestaltet.

Vorzugsweise dient ein und dasselbe Eingabeelement (etwa ein pilzartiger Knopf oder ein Hebel ähnlich einem Joystick) zur Erfassung der induzierten Bewegungen im Direktsteuermodus, aber auch (etwa über einen integrierten Sensor) zur Freigabe des Direktsteuermodus.

Es versteht sich, dass gemäß einer alternativen Ausgestaltung die Freigabe des Direktsteuermodus auch über ein separates Element, also einen separaten Freigabeschalter erfolgen kann, wobei der Freigabeschalter für den Direktsteuermodus nicht zwangsläufig mit dem Freigabeschalter für den zweiten Betriebsmodus übereinstimmen muss.

Gemäß einer weiteren beispielhaften Ausgestaltung ist beim Wechsel zwischen dem Direktsteuermodus und der Steuerung unter Verwendung des separaten Eingabegeräts eine erneute Freigabe über den Freigabeschalter vorgesehen. Auf diese Weise ist sichergestellt, dass der Wechsel zwischen der direkten Steuerung im Direktsteuermodus und der mittelbaren Steuerung über das Eingabegerät eindeutig stattfindet.

In einer beispielhaften Weiterbildung dieser Ausgestaltung ist ein Eingabegerät mit einem Eingabeelement vorgesehen, um die Handhabungseinheit und damit das Beobachtungsinstrument im Direktsteuermodus zu steuern. Das Eingabegerät ist beispielhaft direkt an einem Element der Handhabungseinheit angeordnet und vorzugsweise dem Instrumentenhalter bzw. dem aufgenommenen Instrument benachbart. Das Eingabegerät bzw. dessen Eingabeelement selbst kann grundsätzlich einfach gehalten sein. Es kann sich hierbei um einen Griff handeln, über den der Bediener die Handhabungseinheit durch Ziehen, Drücken oder in ähnlicher Weise bewegen/manipulieren kann.

Gemäß einer weiteren beispielhaften Ausgestaltung der Handhabungsvorrichtung ist die robotische Handhabungseinheit auf einem Wagen montiert, wobei eine Ansteuerung der robotischen Handhabungseinheit zur Bewegung des Instruments nur dann ermöglicht ist, wenn der Wagen (bzw. eine Bewegung des Wagens) blockiert ist. Diese Maßnahme erhöht die Sicherheit weiter. Auf diese Weise werden Relativbewegungen des Wagens gegenüber dem Patienten vermieden, welche auch zu einer Relativbewegung des Instruments gegenüber dem Patienten führen würden.

Die Blockade kann über eine Sperre bzw. eine Abstützung herbeigeführt werden. Beispielhaft ist eine Abstützung vorstellbar, die den Wagen (oder alternativ ein Fahrgestell) so weit anhebt, dass der Wagen feststeht. Die Funktion der Sperre/Abstützung kann über einen Feststell-Sensor überwacht werden. Die Abstützung kann allgemein auch als Fahrsperre bezeichnet werden.

In einer weiteren beispielhaften Ausgestaltung der Handhabungsvorrichtung ist ferner ein Sensor (Blockadesensor) vorgesehen, der die Fahrsperre des Wagens überwacht, um ein entsprechendes Signal an die Steuereinrichtung bzw. eine Sicherheitseinrichtung zu ermitteln. In einer beispielhaften Ausgestaltung ist der Blockadesensor über eine diskrete Schaltung mit einer Sicherheitseinrichtung der Steuereinrichtung gekoppelt. Beispielhaft ist also der Blockadesensor fest mit der Sicherheitseinrichtung vertrat. Es bietet sich eine Gestaltung mit proprietären Elementen an. Auf diese Weise kann die Überwachung der Blockade des Wagens manipulationssicher überwacht werden.

Es ist vorstellbar, den Freigabeschalter mit dem Blockadesensor schaltungstechnisch zu kombinieren, um die Sicherheitsfunktionen zu bündeln. Auch hier bietet sich eine diskrete Schaltung, Gestaltung und Ankopplung der Elemente an, zumindest in beispielhaften Ausführungsformen.

Gemäß einer weiteren beispielhaften Ausgestaltung der Handhabungsvorrichtung ist die Schnittstelle mit einer Mehrzahl von Eingabegeräten koppelbar, über die die robotische Handhabungseinheit steuerbar ist, wobei die Eingabegeräte unterschiedlich priorisierbar sind. Auf diese Weise können gewünschte Redundanzen geschaffen werden. Beispielsweise kann in einem Notfall ein Master-Eingabegerät ein anderes überstimmen bzw. dessen Befehle aufheben.

Ferner kann die Handhabungsvorrichtung durch verschiedene Personen gesteuert werden, die zusammenarbeiten. So ist es vorstellbar, dass die Befehlsgewalt für die Steuerung der Handhabungsvorrichtung während einer medizinischen Prozedur phasenweise von verschiedenen Personen wahrgenommen wird. Ferner ist es vorstellbar, dass verschiedene Eingabegeräte an verschiedenen Orten/Positionen in Bezug auf den Patienten bzw. dessen Auflage angeordnet sind. Dies ermöglicht die Steuerung aus verschiedenen Perspektiven.

Wie vorstehend bereits angedeutet, sind verschiedene Betriebsmodi vorstellbar. Dies umfasst grundsätzlich auch die Bedienung mit verschiedenartigen Eingabegeräten. So ist es vorstellbar, die Handhabungsvorrichtung über ein Einhand-Eingabegerät zu steuern. Ferner ist es vorstellbar, die Handhabungsvorrichtung über einen Touch-Monitor und/oder eine Tastatur in Kombination mit einem Monitor zu steuern. Daneben ist es vorstellbar, die Handhabungsvorrichtung, insbesondere deren robotische Handhabungseinheit, zumindest in einem Direktsteuermodus über einen Krafteingriff an der Handhabungseinheit selbst zu steuern. Es versteht sich, dass je nach gewählter Art der Steuerung nicht unbedingt sämtliche Funktionen der Handhabungsvorrichtung steuerbar sind.

Gemäß einer weiteren beispielhaften Ausgestaltung der Handhabungsvorrichtung ist die Steuereinrichtung dazu ausgebildet, eine gegebene Ausrichtung des Bildaufnehmers zu erfassen und in Abhängigkeit davon eine Zuordnung zwischen einem Eingabegerät-Koordinatensystem und einem Koordinatensystem der Handhabungseinheit vorzunehmen, das die Orientierung des Bildaufnehmers widerspiegelt. Demgemäß beschreibt die Orientierung den Horizont bzw. die Drehlage des Bildaufnehmers.

Mit anderen Worten ist es zumindest in einigen Ausführungsbeispielen vorstellbar, dass eine Rechts-Links-Achse des Eingabegerätes eine Rechts-Links-Bewegung des angezeigten Bildausschnitts bewirkt. Gleichermaßen kann eine Vor-Zurück- Achse des Eingabegerätes eine Vor-Zurück-Bewegung bzw. Hoch-Runter-Bewegung des angezeigten Bildausschnitts bewirken. Dies gilt beispielsweise auch für entsprechende Schwenkbewegungen. Die Information betreffend die aktuelle Orientierung des Bildaufnehmers wird beispielsweise vom Beobachtungsinstrument an die Steuereinrichtung übertragen.

Die Steuerung der robotischen Handhabungseinheit erfolgt nun unter der Prämisse, dass die Orientierung des Bildaufnehmers (der künstliche Horizont des angezeigten Bildausschnitts) gewahrt wird. Eine entsprechende Interpolation der Bewegung über verschiedene Achsen der Handhabungseinheit trägt dazu bei, dass diese Zuordnung beibehalten wird.

Gemäß einer weiteren beispielhaften Ausgestaltung der Handhabungsvorrichtung weist das Beobachtungsinstrument einen Orientierungssensor zur Erfassung der Orientierung des Bildaufnehmers auf. Auf diese Weise kann der künstliche Horizont bzw. das dem Bildaufnehmer eigene Koordinatensystem signaltechnisch erfasst werden.

In einer weiteren beispielhaften Ausgestaltung erfolgt die Erfassung der gegebenen Orientierung des Bildaufnehmers mittelbar über die Anzeige, in dem anhand des angezeigten Bildausschnitts die gewünschte Orientierung (Horizontlage) definiert wird. Es ist grundsätzlich auch vorstellbar, die Orientierung des Bildaufnehmers mittelbar über eine Steuerung eines Antriebs zur Rotation/Verdrehung des Bildaufnehmers zu erfassen. Demgemäß wird die Orientierung nicht über einen Sensor erfasst sondern von den Soll-Vorgaben für den Antrieb hergeleitet.

Insbesondere bei einem Bildaufnehmer mit einem Beobachtungskanal (Mono-Bildaufnehmer) ist auch eine rein digitale Drehung und Erfassung des Horizonts vorstellbar.

Gemäß einer weiteren beispielhaften Ausgestaltung der Handhabungsvorrichtung ist die Steuereinrichtung dazu ausgebildet, die robotische Handhabungseinheit unter Berücksichtigung der gegebenen Ausrichtung des Beobachtungsinstruments derart anzusteuern, dass das Beobachtungsinstrument entlang einer Kugelschalenfläche oder einer Kugelschalenabschnittsfläche verfahrbar ist. Die Bewegung erfolgt entlang einer Kugelschale oder einem Kugelschalenabschnitt mit zumindest im Wesentlichen konstantem Radius in Bezug auf den Pivotpunkt.

Gemäß einer weiteren beispielhaften Ausgestaltung der Handhabungsvorrichtung ist die Steuereinrichtung dazu ausgebildet, bei der Verfahrbewegung des Beobachtungsinstruments die Ausrichtung des Bildaufnehmers unter Berücksichtigung der Bewegungsbahn anzupassen. Auf diese Weise kann die Ausrichtung der optischen Achse auf den Pivotpunkt (zumindest auf einen Bereich in der Nähe davon) herbeigeführt werden. Das Beobachtungsinstrument, insbesondere dessen Bildaufnehmer, ist/sind radial zum Zentrum der umkreisenden Bewegung ausgerichtet.

Gemäß einer weiteren beispielhaften Ausgestaltung der Handhabungsvorrichtung ist die Steuereinrichtung dazu ausgebildet, die robotische Handhabungseinheit unter Berücksichtigung der gegebenen Ausrichtung des Beobachtungsinstruments derart anzusteuern, dass das Beobachtungsinstrument entlang einer Ebene parallel zur Beobachtungsebene verfahrbar ist. Dies erfolgt vorzugsweise unter Berücksichtigung der Ausrichtung des Beobachtungsinstruments, insbesondere eines Bildaufnehmers davon.

Gemäß einer weiteren beispielhaften Ausgestaltung der Handhabungsvorrichtung ist die Steuereinrichtung dazu ausgestaltet, die Handhabungseinheit derart anzusteuern, dass das Instrument durch interpolierte Bewegung der Handhabungseinheit um eine virtuelle Kippachse verschwenkbar ist, die parallel zum Bildaufnehmer angeordnet ist. Auf diese Weise kann ein Instrument (Beobachtungsinstrument) mit variabler Blickrichtung "simuliert" werden. Instrumente ohne integrierten Schwenkantrieb können somit auch einen solchen Freiheitsgrad bzw. eine solche Funktion bereitstellen. Es versteht sich, dass diese Funktion insbesondere bei Instrumenten vorstellbar ist, welche außerhalb des Körpers des Patienten angeordnet sind.

Es ist vorstellbar, dass in alternativen Ausführungsformen Instrumente mit variabler Blickrichtung und entsprechenden (internen) Antrieben vorgesehen sind, wobei ebenso die Steuerung der Antriebe über das Eingabegerät erfolgt. Auf diese Weise kann eine intuitive Steuerung des Schwenkantriebs erfolgen.

Gemäß einer weiteren beispielhaften Ausgestaltung der Handhabungsvorrichtung ist die Steuereinrichtung dazu ausgebildet, einen Typ des Instruments zu erfassen und die robotische Handhabungseinheit in Abhängigkeit vom erfassten Typ anzusteuern. Dies umfasst beispielhaft eine Steuerung der Handhabungseinheit durch die Handhabungssteuereinheit und eine Erfassung/Ermittlung des Typs des Instruments durch die Instrumentensteuereinheit.

Beispielhaft ist die Steuereinrichtung dazu ausgestaltet, unterschiedliche Typen von Instrumenten zu erkennen, beispielsweise anhand einer Identifikationsinformation (ID), wobei durch die Steuereinrichtung eine an den jeweiligen Typ des Instruments angepasste Konfiguration zur Steuerung der Handhabungseinheit und des Instruments nutzt. Gemäß einer weiteren beispielhaften Ausgestaltung ist der Instrumentenhalter dazu ausgestaltet, unterschiedliche Instrumente aufzunehmen. Am Instrumentenhalter ist beispielhaft eine Schnittstelle angeordnet, an die das Instrument signaltechnisch angekoppelt, so dass über diese Schnittstelle Konfigurationsinformationen und/oder Identifikationsinformationen abgefragt werden können.

Gemäß einer weiteren beispielhaften Ausgestaltung der Handhabungsvorrichtung ist der Bildaufnehmer im als Beobachtungsinstrument gestalteten Instrument drehbar. Dies bezieht sich insbesondere auf eine Achse senkrecht zur Bildebene des Bildaufnehmers. Beispielhafte Ausführungsformen mit Stereo-Bildaufnehmer nutzen eine solche Funktion. Es ist grundsätzlich vorstellbar, den Bildaufnehmer manuell verdrehbar zu gestalten. Es ist jedoch auch vorstellbar, einen Drehantrieb/Rotationsantrieb für den Bildaufnehmer vorzusehen. Die Verdrehbarkeit des Bildaufnehmers ermöglicht eine Bildaufrichtung.

Gemäß einer weiteren beispielhaften Ausgestaltung der Handhabungsvorrichtung weist das Beobachtungsinstrument einen Stereo-Bildaufnehmer auf, insbesondere mit zwei Bildsensoren. In einem solchen Fall ist die Bildaufrichtung über die Verdrehbarkeit des Bildaufnehmers von Vorteil. Auf diese Weise kann die Ausrichtung der Beobachtungskanäle mit der menschlichen Augenpartie bewerkstelligt werden. Es sind auch Ausführungsformen mit mehr als zwei Bildsensoren vorstellbar, etwa wenn jeweils zwei Sensoren einem Spektralbereich zugeordnet sind (sichtbares Licht, Infrarot, etc.).

Gemäß einer weiteren beispielhaften Ausgestaltung der Handhabungsvorrichtung ist die Steuereinrichtung dazu ausgebildet, eine Zuordnung zwischen der Orientierung des Bildaufnehmers und Bewegungsachsen für die Eingabe am Eingabegerät derart vorzunehmen, dass Bewegungsrichtungen des durch die Wiedergabeeinheit angezeigten Bildausschnitts mit Richtungsvorgaben am Eingabegerät in Übereinstimmung gebracht werden. Auf diese Weise ist es für den Bediener nicht erforderlich, eine gedankliche Ausrichtung zwischen den unterschiedlichen Koordinatensystemen/Orientierungen vorzunehmen. Der Bediener kann sich primär am angezeigten Bildausschnitt orientieren, um diesen in gewünschter Weise zu bewegen.

Demgemäß umfasst die Umsetzung der Bedienbefehle (Richtungsbefehle und Verfahrbefehle) durch die Steuereinrichtung eine Koordinatentransformation, die bei der Ansteuerung der Handhabungseinheit berücksichtigt wird. Die Bewegungsachsen entsprechen beispielhaft entsprechenden Bewegungsfreiheitsgraden (vor, zurück, rechts, links, etc.).

Gemäß einer weiteren beispielhaften Ausgestaltung der Handhabungsvorrichtung ist das Eingabegerät als Mehrachs-Eingabegerät gestaltet, vorzugsweise als Einhand-Mehrachs-Eingabegerät, wobei das Eingabegerät Bedienbewegungen in Form von Schubbewegungen oder Schwenkbewegungen in zumindest zwei Achsen erlaubt, um Bewegungssignale zur Bewegung des Bildausschnitts entlang einer sphärischen Fläche zu erfassen. Das Eingabegerät kann etwa als sogenannte 3D-Maus gestaltet sein. Durch geeignete Einwirkung auf das Eingabegerät, insbesondere auf ein Betätigungselement des Eingabegerätes, kann die Bewegung des Bildausschnitts beispielsweise entlang einer Kugelfläche oder Kugelabschnittsfläche gesteuert werden. Aus Sicht des Betrachters einer Anzeige einer Wiedergabeeinheit, also etwa eines Monitors, wird das Eingabegerät mit dem Bildaufnehmer zweidimensional entlang einer Abwicklung der gekrümmten Fläche bewegt. Es versteht sich, dass die reale Bewegung im dreidimensionalen Raum erfolgt. Ferner ist es in einem weiteren Modus vorstellbar, die Bewegung des Bildausschnitts entlang einer Ebene parallel zur Objektebene über die Bedienbewegungen zu steuern. Aus Sicht des Betrachters der Wiedergabeeinheit verschiebt sich der angezeigte Bildausschnitt.

Es ist also vorstellbar, beim Eingabegerät ein Eingabeelement vorzusehen, welches in verschiedenen Achsen bewegbar ist, wobei die Bewegungen (beispielsweise Translationsbewegung entlang zweier oder mehr Achsen, sowie Rotationsbewegung bzw. Schwenkbewegung entlang zweier oder mehr Achsen) über geeignete Sensoren erfasst und in Steuerbefehle überführt werden.

Gemäß einer weiteren beispielhaften Ausgestaltung der Handhabungsvorrichtung richtet die Steuereinrichtung die zwei oder mehr Bewegungsachsen des Eingabegerätes mit der gegebenen Orientierung des Bildaufnehmers aus, so dass Bedienbewegungen eines Eingabeelements des Eingabegerätes in richtungsgleichen Bewegungen des angezeigten Bildausschnitts resultieren. Dies sorgt dafür, dass der Bediener intuitiv über Bewegungen nach links, rechts, oben/vorne oder unten/hinten die gewünschten Bewegungen steuern kann. Der Bediener muss sich keine Gedanken um die Koordinatentransformation machen. Dies wird durch die Steuereinrichtung bewerkstelligt.

Mit anderen Worten kann also etwa eine Schwenkbewegung nach rechts eine Rechtsbewegung des Beobachtungsinstruments entlang der gekrümmten Fläche unter Beibehaltung des Objektabstands und der Zentrierung des Bildaufnehmers bewirken. Gleichermaßen bewirkt eine Schwenkbewegung nach links eine Bewegung des Beobachtungsinstruments nach links entlang der gekrümmten Fläche. Ferner kann beispielsweise eine Linearbewegung nach rechts eine Rechtsbewegung des Beobachtungsinstruments entlang der zur Objektebene parallelen Ebene bewirken. Gleichermaßen bewirkt eine Linearbewegung nach links eine Bewegung des Beobachtungsinstruments nach links entlang der parallelen Ebene. Der Objektabstand in Bezug auf die Objektebene bleibt gleich.

Gemäß einer weiteren beispielhaften Ausgestaltung der Handhabungsvorrichtung ist das Eingabegerät als Einhand-Eingabegerät gestaltet, wobei das Eingabegerät Bedienbewegungen zumindest in Form einer Rotation um eine Längsachse oder einer Translation entlang der Längsachse erfasst, um Bewegungssignale zur Steuerung einer Zoom-Funktion sowie für eine Fokuseinstellung zu erfassen. Demgemäß kann das Eingabegerät weitere Funktionen erfüllen. In einfacher Weise kann in nur einem Betriebsmodus eine Mehrzahl von Funktionen (Bewegung, Vergrößerung, etc.) gesteuert werden. Es ist jedoch auch vorstellbar, verschiedene Betriebsmodi vorzusehen, um eine eindeutige Steuerung zu erlauben.

Gemäß einer weiteren beispielhaften Ausgestaltung der Handhabungsvorrichtung ist die Steuereinrichtung dazu ausgestaltet, eine Initialisierungsprozedur durchzuführen, um Konfigurationsinformationen betreffend das gehaltene Beobachtungsinstrument zu erfassen, wobei die Initialisierung vorzugsweise eine Abfrage über die Instrumentensteuereinheit aufweist, und wobei die Konfigurationsinformationen an die Handhabungssteuereinheit übermittelt und bei der Steuerung der Handhabungseinheit berücksichtigt werden.

Auf diese Weise kann die Steuereinrichtung beispielsweise ermitteln, welche Art von Beobachtungsinstrument momentan an der robotischen Handhabungseinheit befestigt ist. Die Art des Beobachtungsinstruments betrifft beispielsweise dessen Abmessungen, Parameter von dessen Bildaufnehmer, die Fähigkeit zum Datenaustausch, eine etwaige Rotationsposition zum Drehen des Bildaufnehmers, und einen etwaigen Sensor zur Erfassung der Drehlage des Bildaufnehmers, usw.

Der Begriff Initialisierungsprozedur ist nicht so zu verstehen, dass es sich lediglich um eine einmalige Prozedur handelt. Die Initialisierungsprozedur kann wiederholt ausgeführt werden, etwa bei jeder konkreten Behandlung oder Diagnoseaufgabe, also beispielsweise beim Hochfahren der Steuereinrichtung, oder bei jeder Umrüstung der Handhabungsvorrichtung. Die Initialisierungsprozedur auch kann gezielt und automatisiert wiederholt werden, etwa durch periodische Wiederholungen. Umgekehrt ist es vorstellbar, dass der Bediener die Initialisierungsprozedur bewusst auslösen kann.

Mit anderen Worten kann während der Initialisierungsprozedur beispielsweise eine Art Offset bestimmt werden, wobei die Steuereinrichtung (Handhabungssteuereinheit) für die robotische Handhabungseinheit diesen Offset bei der Steuerung der robotischen Handhabungseinheit nutzt. Dieser Offset definiert beispielsweise die Lage des Bildaufnehmers in Relation zu den Elementen der Handhabungseinheit. Der Offset kann die geometrische Gestalt/Ausdehnung des Beobachtungsinstruments beschreiben. Auf diese Weise kann der Bildaufnehmer des Beobachtungsinstruments genau gesteuert werden, um den Bildausschnitt wie gewünscht zu verfahren.

Beispielhaft ist es vorstellbar, die der Initialisierungsprozedur innewohnende Abfrage über eine Betätigung des Freigabeschalters zur Aktivierung des zweiten Betriebsmodus zu starten. Die Initialisierungsprozedur kann auch als Setup-Prozedur bezeichnet werden. Demgemäß ist es vorstellbar, verschiedene Kamerasysteme/Beobachtungsinstrumente zu nutzen. Die Daten (Konfigurationsinformationen) können durch das Beobachtungsinstrument direkt bereitgestellt werden. Alternativ kann das Beobachtungsinstrument über seine ID erkannt werden, wobei anhand der ID Daten aus einer Datenbank abzufragen sind.

Gemäß einer weiteren beispielhaften Ausgestaltung der Handhabungsvorrichtung ist die Steuereinrichtung dazu ausgestaltet, den angezeigten Bildausschnitt bedarfsweise zu spiegeln, wobei die Umsetzung von Bedienbefehlen am Eingabegerät die Spiegelung berücksichtigt. Auf diese Weise kann etwa ein Flip-Modus bereitgestellt werden.

Eine Spiegelung erfolgt beispielsweise um eine horizontale oder vertikale Achse. Etwas Derartiges kann beispielsweise dann erfolgen, wenn ein anderer Bediener die Steuerung der Handhabungseinheit übernimmt, der aus Sicht des zuvor aktiven Bedieners auf einer gegenüberliegenden Seite des Patienten steht.

Gemäß einem weiteren Aspekt wird die Aufgabe der vorliegenden Offenbarung durch ein nichtchirurgisches Verfahren zur Steuerung einer Handhabungsvorrichtung gelöst, wobei die Handhabungsvorrichtung eine robotische Handhabungseinheit mit einem Instrumentenhalter und einem daran aufgenommenen Instrument mit einem Bildaufnehmer zur Erfassung eines Bildausschnitts einer Objektebene umfasst, wobei das Verfahren die folgenden Schritte aufweist:
- Bereitstellung einer robotischen Handhabungseinheit mit einem Instrumentenhalter und einem Eingabegerät,
- Bereitstellung des Instruments am Instrumentenhalter,
   wobei das Eingabegerät in einem ersten Betriebsmodus zur Steuerung des Instruments und in einem zweiten Betriebsmodus zur Steuerung der robotischen Handhabungseinheit nutzbar ist, und wobei die Steuerung des Instruments und der robotischen Handhabungseinheit unter Nutzung des Eingabegerätes über eine Steuereinrichtung erfolgt, mit der das Eingabegerät koppelbar ist,
- Aktivierung des zweiten Betriebsmodus, in dem die robotische Handhabungseinheit in Reaktion auf Eingabebefehle am Eingabegerät motorisch verfahrbar ist, um das Instrument motorisch zu bewegen, durch Betätigung eines als Fußschalter gestalteten Freigabeschalters,
   wobei der Freigabeschalter zwei Zustände aufweist, wobei ein erster Zustand ein Ursprungszustand ist, und wobei ein zweiter Zustand ein Aktivierungszustand für den zweiten Betriebsmodus ist,
   wobei der Freigabeschalter einen dritten Zustand aufweist, wobei der Freigabeschalter bei Aktivierung ausgehend vom ersten Zustand durch Aufbringen einer Betätigungskraft zunächst in den zweiten Zustand und danach bei erhöhter Betätigungskraft in den dritten Zustand überführbar ist,
   wobei sowohl im ersten Zustand als auch im dritten Zustand des Freigabeschalters die Steuerung der robotischen Handhabungseinheit gesperrt ist,
   wobei die Steuereinrichtung mit dem Instrument und mit der robotischen Handhabungseinheit koppelbar ist,
   wobei die Steuereinrichtung dem Eingabegerät und dem am Instrumentenhalter aufgenommenen Instrument zwischengeschaltet ist, und
   wobei die Steuereinrichtung dazu ausgebildet ist, im zweiten Betriebsmodus über das Eingabegerät erfasste Steuerbefehle für die robotische Handhabungseinheit der Handhabungssteuereinheit über die Instrumentensteuereinheit bereitzustellen.

Die Aufgabe der Offenbarung wird auch auf diese Weise vollständig gelöst.

Gemäß einer weiteren beispielhaften Ausgestaltung des Verfahrens ist der Freigabeschalter in Abhängigkeit von einer aufgebrachten Betätigungskraft in einem ersten Zustand 0, einem zweiten Zustand I und einem dritten Zustand II betreibbar, wobei der zweite Zustand I ein Aktivierungszustand für den zweiten Betriebsmodus ist, wobei im ersten Zustand 0 und im dritten Zustand II der zweite Betriebsmodus deaktiviert ist, und wobei beim Schalten des Freigabeschalters der zweite Zustand I dem ersten Zustand 0 und dem dritten Zustand II zwischengeordnet ist.

Es ist vorstellbar, das Verfahren zur Steuerung einer medizinischen Handhabungsvorrichtung zu nutzen. Es ist vorstellbar, das Verfahren für chirurgische und/oder diagnostische Prozesse zu nutzen. Es ist jedoch auch vorstellbar, das Verfahren für andere als chirurgische und/oder diagnostische Prozesse zu nutzen. Chirurgische und/oder diagnostische Verfahren fallen jedoch ausdrücklich nicht unter den Anspruchsumfang des erfindungsgemässen Verfahrens. Es sind folglich auch Ausführungsbeispiele vorstellbar, in denen das Verfahren nicht zur Durchführung einer chirurgischen/diagnostischen Prozedur am menschlichen oder tierischen Körper genutzt wird.

Es versteht sich, dass das Steuerungsverfahren analog zu den beispielhaften Ausgestaltungen der Handhabungsvorrichtung weitergebildet sein kann und umgekehrt. Mit anderen Worten kann der Gegenstand beispielhafter Ausgestaltungen und Weiterbildungen, die auf die Handhabungsvorrichtung bezogen sind, auch zum Gegenstand entsprechende Ausgestaltungen des hier beschriebenen Verfahrens werden.

Es versteht sich, dass die vorstehend genannten und die nachstehend noch zu erläuternden Merkmale der Erfindung nicht nur in der jeweils angegebenen Kombination, sondern auch in anderen Kombinationen oder in Alleinstellung verwendbar sind, ohne den Rahmen der vorliegenden Erfindung zu verlassen.

Weitere Merkmale und Vorteile der Erfindung ergeben sich aus der nachfolgenden Beschreibung mehrerer bevorzugter Ausführungsbeispiele unter Bezugnahme auf die Zeichnungen. Es zeigen:
- Fig. 1: eine perspektivische Ansicht einer Ausführungsform einer medizinischen Handhabungsvorrichtung zur Beobachtung eines Objektfeldes bei einem Patienten;
- Fig. 2: eine weitere perspektivische Ansicht der Handhabungsvorrichtung gemäß Fig. 1 in einer abweichenden Ansichtsorientierung;
- Fig. 3: eine schematische, vereinfachte Teilansicht eines Bildaufnehmers bei einem Beobachtungskopf eines Beobachtungsinstruments sowie einer Wiedergabeeinheit zur Veranschaulichung einer Bildorientierung;
- Fig. 4: eine weitere Darstellung analog zur Fig. 3 mit einer korrigierten Bildorientierung durch Rotation des Bildaufnehmers;
- Fig. 5: eine perspektivische Teilansicht einer Ausführungsform einer Handhabungsvorrichtung mit einem Beobachtungsinstrument zur Veranschaulichung einer beispielhaften Funktion der Handhabungsvorrichtung;
- Fig. 6: eine weitere perspektivische Teilansicht einer Ausführungsform einer Handhabungsvorrichtung mit einem Beobachtungsinstrument zur Veranschaulichung einer weiteren beispielhaften Funktion;
- Fig. 7: eine weitere perspektivische Teilansicht einer Ausführungsform einer Handhabungsvorrichtung mit einem Beobachtungsinstrument zur Veranschaulichung einer weiteren beispielhaften Funktion;
- Fig. 8: eine schematische Ansicht einer Anordnung, umfassend zwei Eingabegeräte, welche bei der Handhabungsvorrichtung zu Steuerungszwecken nutzbar sind;
- Fig. 9: eine perspektivische, vereinfachte Teilansicht einer Ausführungsform einer Handhabungsvorrichtung mit einem Eingabeelement, das als Freigabeschalter fungiert;
- Fig. 10: eine weitere perspektivische Teilansicht einer Ausführungsform einer Handhabungsvorrichtung mit einem Beobachtungsinstrument zur Veranschaulichung einer weiteren beispielhaften Funktion;
- Fig. 11: eine Seitenansicht eines Instrumentenhalters mit einem daran aufnehmbaren Beobachtungsinstrument, in einem nicht montierten Zustand;
- Fig. 12: eine weitere Ansicht der Anordnung gemäß Fig. 12 im montierten Zustand;
- Fig. 13: ein schematisches Blockschaltbild zur Veranschaulichung einer Prinzipgestaltung einer Ausführungsform einer Handhabungsvorrichtung;
- Fig. 14: ein schematisch vereinfachtes Blockdiagramm zur Veranschaulichung einer Ausführungsform eines Verfahrens zur Steuerung einer Handhabungsvorrichtung für ein Instrument; und
- Fig. 15: ein weiteres schematisch vereinfachtes Blockdiagramm zur Veranschaulichung einer weiteren Ausführungsform eines Verfahrens zur Steuerung einer Handhabungsvorrichtung für ein Instrument.

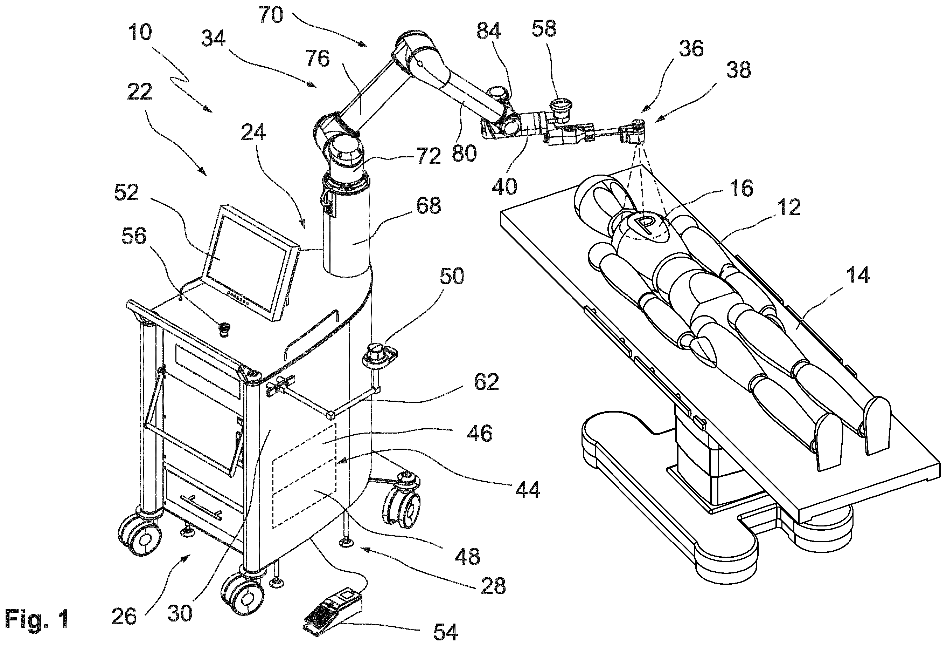

Fig. 1 veranschaulicht anhand einer perspektivischen Übersichtsdarstellung eine insgesamt mit 10 bezeichnete Handhabungsvorrichtung. Die Handhabungsvorrichtung 10 kann auch als medizinische Handhabungsvorrichtung bezeichnet werden. Die Handhabungsvorrichtung 10 ist in dem in Fig. 1 gezeigten Ausführungsbeispiel einem Patienten 12 zugeordnet, der auf einem Tisch 14 gelagert ist. Die Handhabungsvorrichtung 10 ist für therapeutische, chirurgische und/oder diagnostische Zwecke nutzbar. Es ist jedoch auch eine Verwendung für nicht-therapeutische, nicht-chirurgische und/oder nicht-diagnostische Zwecke vorstellbar. Dies kann die Verwendung bei Übungen bzw. Simulationen umfassen.

In dem veranschaulichten Ausführungsbeispiel dient die Handhabungsvorrichtung 10 zur Beobachtung eines Objektfeldes 16. Bei dem Objektfeld 16 handelt es sich beispielhaft um einen Teil des Körpers des Patienten 12. Aus Veranschaulichungszwecken ist in zumindest einigen der hierin gezeigten Figuren das Objektfeldes 16 durch den Buchstaben P gekennzeichnet. Dies ist nicht einschränkend zu verstehen.

In dem in Fig. 1 gezeigten Ausführungsbeispiel ist das Objektfeld 16 außen am Körper des Patienten 12 angeordnet. Demgemäß dient die Handhabungsvorrichtung 10 in diesem Ausführungsbeispiel zur Beobachtung des Körpers von außerhalb des Körpers.

Allgemein dient die Handhabungseinrichtung 10 zur optischen Beobachtung im Bereich des sichtbaren elektromagnetischen Spektrums bzw. in benachbarten Randbereichen. Es geht in wesentlichen Ausführungsbeispielen folglich um die Beobachtung unter Nutzung von Weißlicht, InfrarotStrahlung oder UV-Strahlung. Für das menschliche Auge sichtbare Licht (Weißlicht) liegt etwa in einem Spektralbereich zwischen 380 nm und 780 nm. Strahlung im Nah-Infrarot-Bereich liegt im Bereich von etwa 780 nm bis 1400 nm. Sogenanntes Nahes UV-Licht (auch bezeichnet als Schwarzlicht oder UV-A-Licht) liegt im Bereich von etwa 315 bis 380 nm. Sogenanntes mittleres UV-Licht (auch bezeichnet als UV-B-Licht) liegt im Bereich von etwa 280 nm bis 315 nm.

Die vorgenannten Bereiche können für die Weißlicht-Beobachtung sowie für PDD-Anwendungen (Photodynamische Diagnostik) und PDT-Anwendungen (Photodynamische Therapie) genutzt werden. Hiervon kann auch die Fluoreszenzbeobachtung umfasst sein. In diesem Zusammenhang ist auch Fluoreszenzbeobachtung unter Nutzung von Indocyaningrün (ICG) mit Fluoreszenz im Nahinfrarotbereich denkbar.

Die Handhabungsvorrichtung 10 umfasst eine Plattform 22, welche etwa als Fahrwagen oder Wagen 24 gestaltet ist. Dies ist nicht einschränkend zu verstehen. Gleichwohl ist es zumindest in beispielhaften Ausgestaltungen vorgesehen, dass die Plattform 22 verfahrbar ist. Dies erhöht die Flexibilität und Eignung für diverse Anwendungsfälle. Demgemäß ist die Plattform 22 beispielsweise als Wagen 24 mit einem Fahrgestell 26 gestaltet. In der in Fig. 1 gezeigten Ausführungsform umfasst der Wagen 24 neben dem Fahrgestell 26 auch eine sogenannte Abstützung 28 bzw. eine entsprechende Stützeinheit.

Die Abstützung 28 wird genutzt, um den Wagen 24 während des Betriebs der Handhabungsvorrichtung 10 gegen ungewolltes Verfahren zu schützen. Demgemäß kann die Abstützung 28 dazu dienen, den Wagen 24 aufzubocken. Alternativ oder zusätzlich ist es vorgesehen, Räder des Fahrgestells 26 zu blockieren, im Sinne einer Feststellbremse. Der Status des Wagens 24 (fahrbar oder aufgebockt/festgestellt) kann über geeignete Sensoren überwacht werden, um einen Betrieb der Handhabungsvorrichtung 10 eben nur dann freizugeben, wenn sichergestellt ist, dass der Wagen 24 nicht ungewollt verfahrbar ist. Es versteht sich, dass der Wagen 24 auch in anderer Weise verankert/fixiert werden kann, um einen sicheren Betrieb der Handhabungsvorrichtung 10 zu ermöglichen.

Ferner weist die Plattform 22 ein Gehäuse 30 auf, das Elemente/Einheiten der Handhabungsvorrichtung 10 beherbergt. Auf diese Weise ergibt sich eine kompakte, übersichtliche Gestaltung. Ferner vereinfacht sich die Reinigung der Handhabungsvorrichtung 10. Alternativ ist auch eine Gestaltung als Regal oder Regalwagen vorstellbar. In beispielhaften Ausgestaltungen sind wesentliche Steuereinheiten für die Handhabungsvorrichtung 10 im Gehäuse 30 des Fahrwagens 24 angeordnet. Dies führt dazu, dass die Plattform 22 mobil ist, so dass ein Einsatz an verschiedenen Orten bzw. in verschiedenen Räumen vorstellbar ist. Es versteht sich, dass die Plattform 22 bzw. der Fahrwagen 24 gleichwohl mit der Umwelt gekoppelt sind, etwa zu Zwecken der Energieversorgung, Signalversorgung und/oder Medienversorgung.

Die Plattform 22 bzw. der die Plattform bildende Fahrwagen 24 trägt eine Handhabungseinheit 34. In den veranschaulichten Ausführungsbeispielen ist die Handhabungseinheit 34 als motorische Handhabungseinheit, beispielsweise als robotische Handhabungseinheit gestaltet. Alternativ kann die Handhabungseinheit 34 als Telemanipulatoreinheit bezeichnet werden. Demgemäß bildet die Plattform 22 eine Basis für die Handhabungseinheit 34. Zumindest in beispielhaften Ausführungsformen sind Steuereinrichtungen für die Handhabungseinheit 34 an der Plattform 22 bzw. in deren Gehäuse 30 angeordnet.

Die Handhabungseinheit 34 ist dazu ausgebildet, ein Instrument 36 zu tragen/zu halten. Über die Handhabungseinheit 34 kann das Instrument 36 motorisch bewegt werden. Demgemäß kann die Handhabungseinheit 34 als Telemanipulator für das Instrument 36 bezeichnet werden. Bei dem Instrument 36 handelt sich beispielsweise um ein medizinisches Instrument. Zumindest in beispielhaften Ausgestaltungen ist das Instrument 36 als Beobachtungsinstrument 38 gestaltet. Bei den Beobachtungsinstrument 38 handelt es sich beispielhaft um ein Instrument zur Beobachtung des Patienten 12 von außerhalb des Körpers, also mit Abstand zum Körper des Patienten 12. Ein solches Beobachtungsinstrument 38 kann als Exoskop gestaltet und bezeichnet sein.

Das Instrument 36 ist an einem Instrumentenhalter 40 aufgenommen. Vorzugsweise ist das Instrument 36 lösbar am Instrumentenhalter 40 aufgenommen. Mit anderen Worten kann das Instrument 36 grundsätzlich auch vom Instrumentenhalter und folglich von der Handhabungseinheit 34 gelöst werden. Somit ist es vorstellbar, das Instrument 36 in alternativen Anwendungen auch handgeführt/handgehalten zu betreiben. Nachfolgend wird insbesondere aus Veranschaulichungsgründen davon ausgegangen, dass das Instrument 36 als Beobachtungsinstrument 38 zur Beobachtung eines Objektfeldes 16 genutzt wird, insbesondere als medizinisches Beobachtungsinstrument 38 zur Beobachtung eines Objektfeldes 16 bei einem Patienten 12.

In Fig. 1 veranschaulicht das Bezugszeichen 44 anhand gestrichelter Linien eine Steuereinrichtung 44, die an der Plattform 22 aufgenommen ist. Beispielhaft weist die Plattform 22 einen Wagen 24 mit einem Regalgestell auf, das eine Einhausung in Form eines Gehäuses 30 umfasst. Demgemäß kann die Steuerrichtung 44 an jenem Regalgestell aufgenommen und gehalten sein.

Zumindest in beispielhaften Ausführungsformen weist die Steuereinrichtung 44 eine Handhabungssteuereinheit 46 und eine Instrumentensteuereinheit 48 auf. Bei der Handhabungssteuereinheit 46 und der Instrumentensteuereinheit 48 kann es sich um diskrete, grundsätzlich separate Steuereinheiten/Steuermodule handeln. Mit anderen Worten können mehrere Geräte miteinander kombiniert werden, um die Steuereinrichtung 44 zu bilden. Es ist jedoch auch vorstellbar, die Steuereinrichtung 44 derart zu gestalten, dass die Handhabungssteuereinheit 46 und die Instrumentensteuereinheit 48 zumindest teilweise gemeinsame Hardware/Rechentechnik nutzen. Mit anderen Worten ist es vorstellbar, die Steuereinheiten 46, 48 diskret und/oder integral zu gestalten. Mischformen sind denkbar.

Zur Steuerung der Handhabungsvorrichtung 10 und insbesondere zur Interaktion mit der Steuereinrichtung 44 sind diverse Eingabegeräte für einen Bediener (etwa einen Operateur oder Assistenten) vorgesehen. Beispielsweise ist ein Eingabegerät 50 vorgesehen, welches als Einhand-Eingabegerät gestaltet ist. Beispielhaft ist das Eingabegerät 50 als sogenannte 3D-Maus gestaltet, zumindest ähnlich einer 3D-Maus. Mit anderen Worten kann das Eingabegerät 50 dazu ausgebildet sein, Bedienereingaben und folglich Steuerbefehle in mehreren Raumachsen zu erfassen, wobei die Eingabe über lediglich eine Hand erfolgt, die an ein einziges Eingabeelement angreift. Vorzugsweise dient das Eingabegerät 50 sowohl zur Steuerung der robotischen Handhabungseinheit 34 als auch zur Steuerung des Beobachtungsinstruments 38 bzw. zur Steuerung einer Wiedergabe eines durch das Beobachtungsinstrument 38 aufgenommenen Bildes. In diesem Zusammenhang wird erneut auf die DE 10 2015 121 017 A1 verwiesen, die die Verwendung eines Einhand-Eingabegerätes zur Steuerung von Bildaufnahmeparametern und zur Steuerung von Bildwiedergabeparametern zeigt.

Ein weiteres Eingabegerät 52 ist als sogenannter Touch-Monitor, also als Berührbildschirm gestaltet. Demgemäß ist das Eingabegerät 52 für Auswahlentscheidungen, allgemeines Einstellungen und ähnliche Funktionen nutzbar. Es ist grundsätzlich auch möglich, die robotische Handhabungseinheit 34 über das Eingabegerät 52 zu steuern. Das als Touch-Monitor gestaltete Eingabegerät 52 kann etwa genutzt werden, um allgemeine Einstellungen betreffend das Instrument 36 (Beobachtungsinstrument 38) vorzunehmen. Dies umfasst beispielsweise auch die Auswahl des aktuellen Typs des Beobachtungsinstruments 38. Ferner können Betriebsparameter zum Betrieb des Beobachtungsinstruments 38 über das Eingabegerät 52 ausgewählt und/oder eingegeben werden.

Ein weiteres Eingabegerät 54 ist als beispielsweise Fußschalter gestaltet. Der Fußschalter kann durch den Bediener betätigt werden, ohne dass hierfür die Hände benötigt werden. Das als Fußschalter gestaltete Eingabegerät 54 kann insbesondere als Freigabeschalter verwendet werden. Die Gestaltung als Fußschalter ist nicht einschränkend zu verstehen.

Grundsätzlich ist das Eingabegerät 54 dazu vorgesehen, bestimmte Funktionen/Operationen bei Bedarf freizugeben, und zwar nur auf ausdrücklichen Befehl des Bedieners. Anders gesagt kann durch das Eingabegerät verhindert werden, dass bestimmte Funktionen unbewusst ausgelöst werden. Auf diese Weise kann insbesondere die robotische Handhabungseinheit 34 sicher bedient werden. Dies bezieht sich insbesondere auf Bewegungen des Instrumentenhalters 40 (mit dem daran aufgenommenen Instrument 36) in Bezug auf den Patienten 12 oder den Tisch 14. Solche Bewegungen sollen dann möglich sein, wenn ein zusätzliches Freigabesignal über das Eingabegerät 54 vorliegt. Ferner kann das als Freigabeschalter dienende Eingabegerät 54 mit einer Sicherheitssteuerung (Freigabesteuerung) gekoppelt sein.

Ferner zeigt die in Fig. 1 veranschaulichte Ausgestaltung der Handhabungsvorrichtung 10 ein weiteres Eingabegerät 56, welches als Knopf oder Taster gestaltet ist. Das Eingabegerät 56 kann grundsätzlich als Not-Aus-Taster gestaltet sein. Demgemäß kann das Eingabegerät 56 eine aktuelle Aktion der Handhabungsvorrichtung 10, insbesondere der robotischen Handhabungseinheit 34 abbrechen. Es ist jedoch auch vorstellbar, das Eingabegerät 56 ähnlich dem zuvor beschriebenen Eingabegerät 54 als Freigabeschalter zur Aktivierung (Ermöglichung) bestimmter Funktionen auszuführen. Beide Ausführungsformen erhöhen die Sicherheit beim Betrieb der Handhabungsvorrichtung 10.

Ein weiteres Eingabegerät 58 ist in der in Fig. 1 veranschaulichten beispielhaften Ausgestaltung der Handhabungsvorrichtung 10 direkt bei der robotischen Handhabungseinheit 34 vorgesehen. Das Eingabegerät 58 ist am oder nahe dem Instrumentenhalter 40 angeordnet. Das Eingabegerät 58 dient in einem sogenannten Direktsteuermodus einer quasi-direkten Steuerung/Verlagerung der Handhabungseinheit 34 und folglich des Beobachtungsinstruments 38. Mit anderen Worten kann der Bediener das Beobachtungsinstrument 38 im Direktsteuermodus (auch bezeichnet als Direct Drag Mode) durch Ziehen bzw. Schwenken/Drehen des beispielsweise griffartig, pilzartig oder knopfartig gestalteten Eingabegerätes 58 in einfacher Weise quasi-manuell steuern.

Im Direktsteuermodus wird die Handhabungseinheit 34 durch die Steuereinrichtung 44 bzw. durch deren Handhabungssteuereinheit 46 derart betrieben, dass die robotische Handhabungseinheit 34 den Bedienbefehlen unmittelbar folgt. Auf diese Weise ergibt sich für den Bediener der Eindruck, das Beobachtungsinstrument 38 direkt oder nahezu direkt im Raum zu manövrieren. Die Handhabungseinheit 34 folgt der Bewegung, also dem Steuerbefehl, des Bedieners. Sofern die Steuerbewegung durch den Bediener endet, verbleibt die Handhabungseinheit 34 in der aktuell gewählten Position und hält diese und somit auch das Beobachtungsinstrument 34 entsprechend im Raum. Im Direktsteuermodus kann die Handhabungseinheit 34 derart angesteuert werden, dass für den Bediener bei der direkten Einwirkung auf das Eingabegerät 58 eine definierte Kraft zu überwinden ist.

In einer beispielhaften Ausgestaltung ist das Eingabegerät 50 über einen Ausleger 62 mit der Plattform 22 verbunden. Der Ausleger 62 kann verschiedene Glieder aufweisen, welche verstellbar sind. Folglich kann situationsabhängig eine ergonomisch günstige Position für das Eingabegerät 50 eingestellt werden. Vorzugsweise sind die Eingabegeräte 50, 52, 54, 56, 58 signaltechnisch (also etwa über Datenleitungen oder gegebenenfalls per Funk) mittelbar oder unmittelbar mit der Steuereinrichtung 44 gekoppelt. Dies kann eine Kopplung mit der Handhabungssteuereinheit 46 und/oder der Instrumentensteuereinheit 48 umfassen.

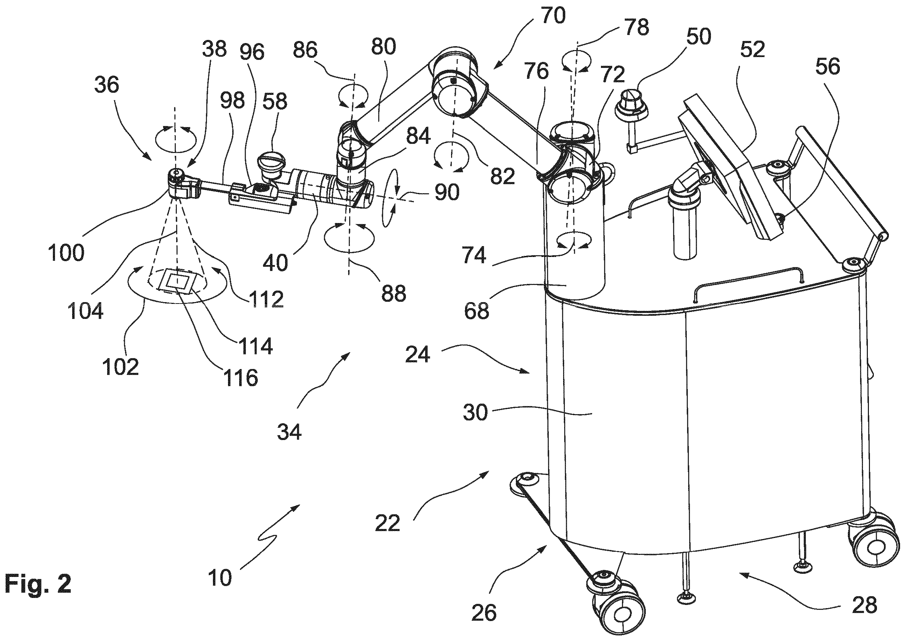

Fig. 1 und Fig. 2 veranschaulichen eine beispielhafte Ausgestaltung der robotischen Handhabungseinheit 34. Die Handhabungseinheit 34 weist ein Grundgestell 68 auf, welches an der als Wagen 24 gestalteten Plattform 22 angeordnet ist. Mit anderen Worten ist die Handhabungseinheit 34 zumindest in dem in Fig. 1 und Fig. 2 gezeigten Ausführungsbeispiel verfahrbar.

Die Handhabungseinheit 34 weist eine kinematische Kette 70 auf, deren Basis das Grundgestell 68 an der Plattform 22 bildet. Die Handhabungseinheit 34 ist als offene kinematische Kette gestaltet. Mit anderen Worten weist die kinematische Kette 70 eine Mehrzahl von Gliedern auf, welche in Reihe angeordnet und miteinander gekoppelt sind.

Die Handhabungseinheit 34 weist ein Karussell 72 auf, welches am Grundgestell 68 aufgenommen/gelagert ist. Das Karussell 72 ist beispielhaft gegenüber dem Grundgestell 68 verdrehbar (um eine vertikale Achse). Demgemäß ist zwischen dem Karussell 72 und dem Grundgestell 68 ein Gelenk 74 vorgesehen. Das Gelenk 74 definiert eine (im Ausführungsbeispiel vertikale) Rotationsachse. Das Grundgestell 68 bildet ein proximales Ende der kinematischen Kette 70 der Handhabungseinrichtung 34. Der Instrumentenhalter 40 bildet ein distales Ende der kinematischen Kette der Handhabungseinrichtung 34.

An das Karussell 72 schließt sich eine Schwinge 76 an, welche über ein Gelenk 78 mit dem Karussell 72 gekoppelt ist, vergleiche Fig. 2. Das Gelenk 78 definiert eine (im Ausführungsbeispiel horizontale) Rotationsachse. Ferner ist ein Arm 80 vorgesehen, der über ein Gelenk 82 mit der Schwinge 76 gekoppelt ist. Das Gelenk 82 definiert eine Rotationsachse. In der kinematischen Kette 70 folgt ein als Hand 84 bezeichnetes Element, welches mit dem Arm 80 über ein Gelenk 86 gekoppelt ist (vgl. Fig. 2). Das Gelenk 86 definiert eine Rotationsachse.

An das als Hand 84 bezeichnete Element schließt sich im Ausführungsbeispiel gemäß den Figuren 1 und 2 der Instrumentenhalter 40 zur Aufnahme des Beobachtungsinstruments 38 an. Der Instrumentenhalter 40 ist über ein Gelenk 88 mit dem als Hand 84 bezeichneten Element gekoppelt. Das Gelenk 88 definiert eine Schwenkachse. Der Instrumentenhalter 40 ist um die durch das Gelenk 88 definierte Achse relativ zur Hand 84 bzw. relativ zum Arm 80 rotierbar. Ferner ist es vorstellbar, dass der Instrumentenhalter 40 um seine Längsachse rotierbar ist, vergleiche das Gelenk 90, welches ebenso eine Rotationsachse definiert. Die Darstellung gemäß Fig. 2 ist nicht einschränkend zu verstehen.

Den Gelenken 74, 78, 82, 86, 88, 90 ist in einer beispielhaften Ausführungsform jeweils ein Antrieb zugeordnet. Bei dem Antrieb handelt es sich beispielsweise um einen Direktantrieb oder Servo-Antriebe. Die Antriebe sind in Fig. 2 nicht explizit dargestellt.

Es versteht sich, dass die Handhabungseinheit 34 auch abweichend von dem in den Figuren 1 und 2 gezeigten Ausführungsbeispiel gestaltet sein kann. Dies betrifft beispielsweise die Anzahl der Glieder der kinematischen Kette 70 und/oder die tatsächlichen Freiheitsgrade bzw. Bewegungsachsen zwischen benachbarten Gliedern. Wesentlich ist, dass die robotische Handhabungseinheit 34 dazu nutzbar ist, das Beobachtungsinstrument 38 in zumindest zwei Freiheitsgraden relativ zum Patienten 12 bzw. zum Tisch 14 zu bewegen. Vorzugsweise ermöglicht die Handhabungseinheit 34 eine Bewegung des Beobachtungsinstruments 38 in vier, fünf, sechs oder gar sieben Freiheitsgraden. Es versteht sich, dass auch robotische Handhabungseinheiten mit mehr oder weniger Gliedern und auch mit abweichenden Bewegungsfreiheitsgraden nutzbar sind. Die Anzahl der beweglichen (im Regelfall schwenkbaren) Achsen wird üblicherweise so gewählt, dass die gewünschten Freiheitsgrade für das Instrument 36 bereitgestellt werden können.

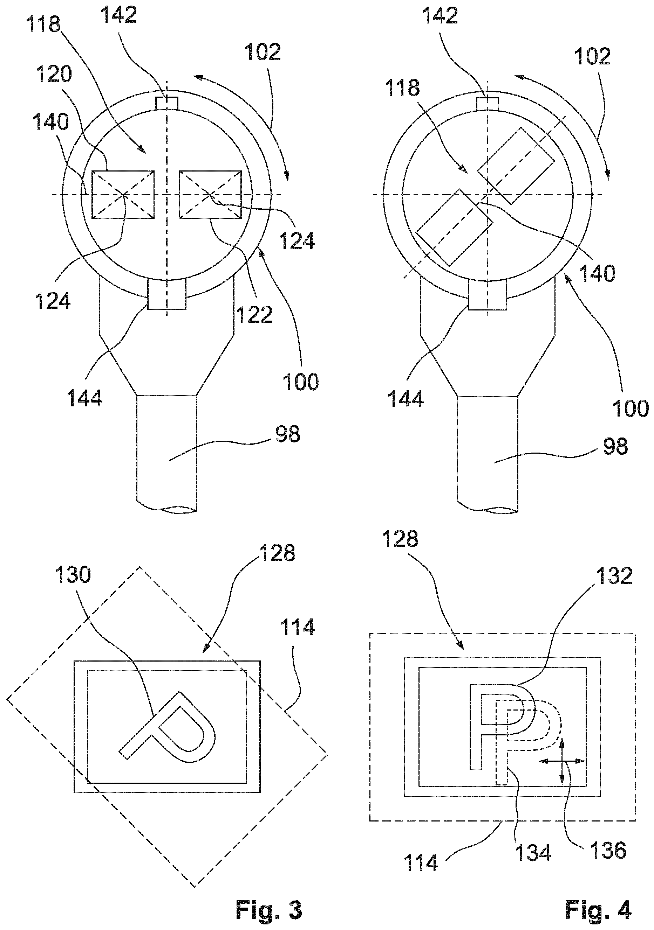

Die Figuren 3 und 4 veranschaulichen anhand vereinfachter schematischer Darstellungen eine beispielhafte Ausgestaltung eines mit 100 bezeichneten Beobachtungskopfes des Beobachtungsinstruments 38. Vergleiche hierzu auch Fig. 2. Das Beobachtungsinstrument 38 weist ein Gehäuse 96 auf. Vom Gehäuse 96 ausgehend erstreckt sich ein Schaft 98 in Richtung auf ein distales Ende des Beobachtungsinstruments 38. Am distalen Ende ist der Beobachtungskopf 100 angeordnet. Die Figuren 3 und 4 veranschaulichen lediglich das distale Ende des Beobachtungsinstruments 38 mit dem Bearbeitungskopf 100.

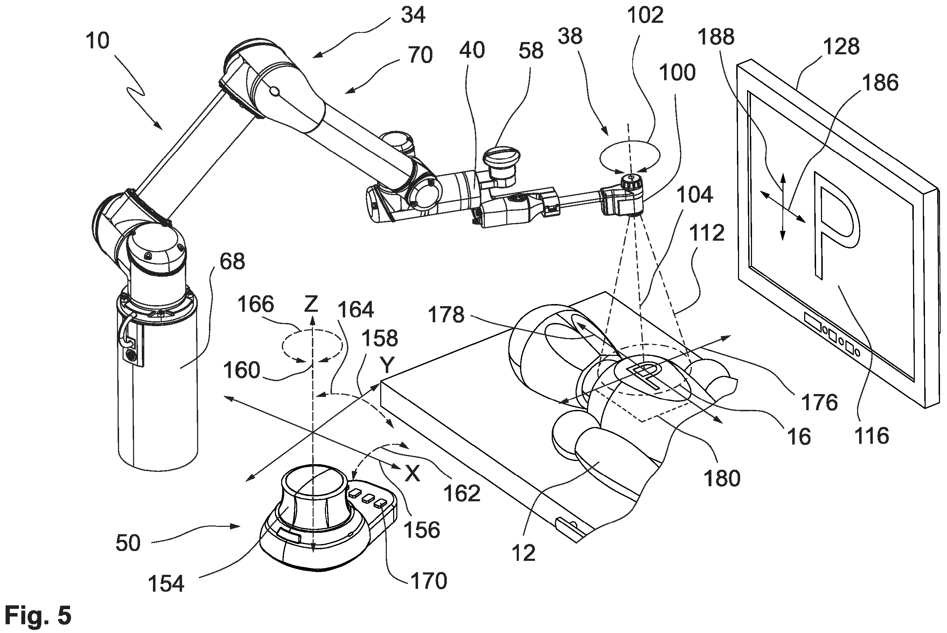

Fig. 3 und Fig. 4 ist ferner entnehmbar, dass zumindest in beispielhaften Ausgestaltungen ein Rotationsfreiheitsgrad (vergleiche den Doppelpfeil 102) für den Beobachtungskopf 100 bzw. für einen darin verbauten Bildaufnehmer vorgesehen ist. Demgemäß ist eine Bildaufrichtung (Bildrotation) in Bezug auf eine optische Achse 104 möglich, vergleiche hierzu auch Fig. 2. Das Beobachtungsinstrument 38 mit dem Beobachtungskopf 100 ist dazu ausgestaltet, ein Sichtfeld 112 (vergleiche Fig. 2 und Fig. 5) zu beobachten und im Sichtfeld 112 einen Bildausschnitt 116 in einem Aufnahmebereich 114 zu erfassen. Dies erfolgt in einer Objektebene bzw. einem Objektfeld 16 (vergleiche Fig. 1). Das Sichtfeld 112 wird durch ein optisches Abbildungssystem des Beobachtungskopfes 100 definiert.

Das Sichtfeld 112 und im Beobachtungskopf 100 verbaute Bildsensoren (ein Sensor oder mehrere Sensoren) definieren den (möglichen) Aufnahmebereich 114. Der Aufnahmebereich 114 kann nicht größer als das Sichtfeld 112 sein. Der Aufnahmebereich 114 wird etwa durch die Größe eines Bildsensors oder mehrerer Bildsensoren sowie die abbildende Optik definiert. Der Bildausschnitt 116 kann grundsätzlich dem Aufnahmebereich 114 entsprechen. Es ist jedoch auch vorstellbar, zumindest in beispielhaften Betriebsmodi, dass der Bildausschnitt 116 bewusst kleiner als der Aufnahmebereich 114 gewählt wird. Dies ist einerseits bei einem Digitalzoom vorstellbar. Ferner kann der Bildausschnitt 116 kleiner als der Aufnahmebereich 114 gewählt werden, um etwaige Abbildungsfehler/Darstellungsfehler im Randbereich des Aufnahmebereichs 114 (also an den Rändern der verbauten Bildsensoren) zu vermeiden oder zumindest zu minimieren.

Zur Erfassung des Bildausschnitts 116 bzw. des Aufnahmebereichs 114 weist das Beobachtungsinstrument 38 einen Bildaufnehmer 118 auf. In dem in den Figuren 3 und 4 veranschaulichten Ausführungsbeispiel ist ein Stereo-Bildaufnehmer 118 verbaut. Demgemäß weist der Bildaufnehmer 118 einen ersten Sensor 120 und einen zweiten Sensor 122 auf. Die Sensoren 120, 122 sind beispielsweise als CCD-Bildsensoren, CMOS-Bildsensoren oder in ähnlicher Weise gestaltet. Die Sensoren 120, 122 weisen jeweils eine Mehrzahl von Erfassungspixeln auf. Es versteht sich, dass der Bildaufnehmer 118 grundsätzlich auch als (Mono-) Bildaufnehmer mit nur einem Beobachtungskanal gestaltet sein kann. Die Bezugszeichen 124 kennzeichnen jeweils ein Zentrum bzw. einen Mittelpunkt der Sensoren 120, 122.

Zur Wiedergabe des aufgenommenen Bildes ist eine Wiedergabeeinheit 128 vorgesehen. Die Wiedergabeeinheit 128 umfasst etwa einen Monitor oder ein ähnliches Display. Die Wiedergabeeinheit 128 ist in beispielhaften Ausgestaltungen zur stereoskopischen Bildwiedergabe ausgebildet. Demgemäß kann die Wiedergabeeinheit 128 als 3D-Monitor gestaltet sein. Es sind Gestaltungen vorstellbar, bei denen ein Monitor über Hilfsmittel (3D-Brillen) betrachtet wird, um den stereoskopischen Effekt zu erzielen. Es sind jedoch auch Gestaltungen vorstellbar, bei denen die Wiedergabeeinheit 128 als Head-Mounted Display (HMD), also beispielsweise als Videobrille gestaltet ist.

Ein Stereo-Bildaufnehmer 118 ermöglicht die stereoskopische Beobachtung, gegebenenfalls sogar eine 3D-Beobachtung. Dies wird durch einen Versatz zwischen den beiden Sensoren 120, 122 ermöglicht, welche an den Versatz zwischen dem rechten und dem linken Auge des Beobachters angepasst ist. Auf diese Weise ergibt sich bei der Betrachtung ein räumlicher Eindruck. Die stereoskopische Beobachtung macht es jedoch erforderlich, dass die beiden Sensoren 120, 122 in einer bestimmten Weise ausgerichtet sind, nämlich entlang eines (künstlichen) Horizonts 140, der an die Lage der Wiedergabeeinheit 128 und mittelbar an die Lage der Augen bzw. der Augenpartie des Beobachters angepasst ist.

In dem in Fig. 3 veranschaulichten Zustand zeigt die Wiedergabeeinheit 128 einen Bildausschnitt 130 in einer ersten Orientierung an. Diese ergibt sich aus der Orientierung des Bildaufnehmers 118 in Bezug auf den Beobachtungskopf 100 sowie insgesamt aus der Orientierung des Beobachtungsinstruments 38 in Bezug auf das Objektfeld 16 beim Patienten 12. Die Darstellung des Bildausschnitts 130 in Fig. 3 veranschaulicht anhand eines Beispiels (Buchstabe P) eine geneigte Orientierung, in der das unmittelbare Verständnis und vor allem die Zuordnung von Richtungen für den Beobachter erschwert ist. Es wäre für den Beobachter wünschenswert, die in Fig. 4 gezeigt Orientierung des Bildausschnitts 132 zu verwenden. Die Orientierung des Bildausschnitts 130 in Fig. 3 resultiert aus der gegebenen Orientierung des Bildaufnehmers 118, vergleiche den Horizont 140.

Um den angezeigten Bildausschnitt 132 in der gewünschten Weise auszurichten, ist es erforderlich, den Bildaufnehmer 118 mit den Sensoren 120, 122 zu drehen, vergleiche die Orientierung des Horizonts 140 des Bildaufnehmers 118 in Fig. 4. Zu diesem Zweck ist der Rotationsfreiheitsgrad 102 vorgesehen, der eine Bildaufrichtung ermöglicht. Die Bildaufrichtung unter Nutzung der Verdrehbarkeit des Bildaufnehmers 118 um die optische Achse 104 in Bezug auf den Beobachtungskopf 100 ist insbesondere für Stereo-Bildaufnehmer von Vorteil. Jedoch können sich auch bei Mono-Bildaufnehmern 118 mit nur einem Beobachtungskanal Vorteile ergeben, etwa in Bezug auf gegebene Abmessungen (zum Beispiel ein Aspektverhältnis) des verwendeten Bildsensors.

In einer beispielhaften Ausführungsform weist der Beobachtungskopf 100 einen Lagesensor/Orientierungssensor 142 zur Erfassung einer Drehlage des Bildaufnehmers 118 in Bezug auf die Beobachtungskopf 100 bzw. den Schaft 98 des Beobachtungsinstruments 38 auf. Auf dieser Basis kann eine gewünschte Ausrichtung des Bildausschnitts 130, 132 in Abhängigkeit von der Ist-Orientierung des Bildaufnehmers 118 eingestellt werden.

Es ist grundsätzlich vorstellbar, den Bildaufnehmer 118 manuell um dessen optische Achse 104 zu rotieren. Es ist in alternativen Ausgestaltungen auch vorstellbar, einen Antrieb 144 zur Rotation des Bildaufnehmers 118 um die optische Achse 104 zu nutzen. Sofern ein Antrieb 144 genutzt wird, kann der Orientierungssensor 142 in den Antrieb 144 integriert werden. Es ist jedoch auch vorstellbar, die Drehlage/Orientierung des Bildaufnehmers 118 von Steuerdaten zur Steuerung des Antriebs 144 abzuleiten. Wenn also dem Antrieb 144 ein bestimmtes Drehinkrement für eine Drehbewegung vorgegeben wird, so ist im Umkehrschluss zumindest die Ziel-Orientierung des Bildaufnehmers 118 bekannt.

Es versteht sich, dass auch eine elektronische/digitale Bildaufrichtung vorstellbar ist, wobei der aufgenommene und angezeigte Bildausschnitt digital gedreht wird. Etwas Derartiges ist jedoch bei der Stereo-Beobachtung unter Beibehaltung der Stereo-Funktionalität kaum realisierbar. Jedoch ist in beispielhaften Ausführungsformen eine digitale Feinjustierung oder Feinausrichtung vorstellbar.

Es versteht sich, dass das Beobachtungsinstrument 38 grundsätzlich auch über die robotische Handhabungseinheit 34 ausgerichtet werden könnte, um den angezeigten Bildausschnitt 130, 132 auszurichten. Dies würde jedoch häufig dazu führen, dass das Beobachtungsinstrument 38 bzw. die Handhabungseinheit 34 im Wege stehen und etwa die freie direkte Sicht auf das Operationsfeld/Objektfeld für den Operateur und/oder Dritte beeinträchtigen könnten. Ferner muss das Operationsfeld in vielen Fällen für weitere Instrumente zugänglich sein. Deshalb wird die robotische Handhabungseinheit 34 regelmäßig derart ausgerichtet, dass diese so wenig wie möglich den Arbeitsablauf stört. Dann muss jedoch unter Umständen der Bildaufnehmer 118 unter Nutzung des Freiheitsgrades 102 rotiert werden, um das Bild in der gewünschten Weise aufzurichten.

Diese Ausrichtung/Aufrichtung durch Rotation des Bildaufnehmers 118 führt jedoch unter Umständen dazu, dass die robotische Handhabungseinheit 34 nicht intuitiv gesteuert werden kann. Wenn nämlich der Beobachter/Bediener beispielsweise über das Eingabegerät 50 Steuerbefehle in Form von Richtungsbefehlen und Wegbefehlen vorgibt, um über die Handhabungseinheit 34 das Beobachtungsinstrument 38 zu verfahren, so orientiert er sich regelmäßig am angezeigten Bildausschnitt 116 (vergleiche auch Bezugszeichen 130, 132 in den Figuren 3 und 4). Die Ausrichtung des Motivs im Bildausschnitt 116 korreliert jedoch häufig nicht mit den Betätigungsachsen (zum Beispiel rechts - links - vorne - hinten) des Eingabegerätes 50. In einem solchen Fall führt also eine Rechtsbewegung am Eingabegerät 50 nicht unbedingt zu einer korrespondierenden Bewegung des angezeigten Bildausschnitts 116 nach rechts.

Fig. 3 und Fig. 4 veranschaulichen ferner eine beispielhafte Ausgestaltung, bei der das angezeigte Bild bzw. der angezeigte Bildausschnitt 116, 132 kleiner als der theoretisch verfügbare Aufnahmebereich 114 der Sensoren 120, 122 ist. Wenn also nur ein Ausschnitt des jeweiligen Aufnahmebereichs 114 angezeigt wird, so ist es theoretisch möglich, den angezeigten Bildausschnitt 116 innerhalb des Aufnahmebereichs 114 zu verschieben. Dies wird in Fig. 4 durch einen verschobenen Bildausschnitt 134 sowie ein mit 136 bezeichnetes Koordinatensystem angedeutet. Ferner ermöglicht dies, wie zuvor schon angedeutet, zumindest in Grenzen einen sogenannten Digital-Zoom.

Die Orientierung des Aufnahmebereichs 114 in Fig. 3 (in Relation zum durch die Wiedergabeeinheit 128 angezeigten Bildausschnitt 116, 130) verdeutlicht, dass auch bei einer digitalen Bildrotation ein größerer Aufnahmebereich 114 von Vorteil ist. Somit kann der angezeigte Bildausschnitt 116, 130 bei dem gegebenen Aspektverhältnis bzw. allgemein bei der gegebenen Gestalt der Wiedergabeeinheit 128 gedreht werden, ohne dass es beispielsweise in den Ecken der Wiedergabeeinheit 128 zu Auslassungen kommt.

Die Möglichkeit, den Bildausschnitt 116, 132 kleiner als den Aufnahmebereich 114 zu wählen, führt zu Situationen, in denen ein aktuelles Zentrum bzw. ein aktueller Mittelpunkt des angezeigten Bildausschnitts 116, 132 nicht dem Mittelpunkt 124 des Sensors 120, 122 entspricht. Dies muss bei dem Betrieb der Handhabungsvorrichtung 10 berücksichtigt werden.

Gemäß einem Aspekt der vorliegenden Offenbarung wird vorgeschlagen, eine Koordinatentransformation zwischenzuschalten, um eine intuitive Steuerung der Handhabungseinheit 34 zum Verfahren des Instruments 36 bzw. Beobachtungsinstruments 38 zu ermöglichen. Dieser Ansatz erlaubt es beispielsweise, sich bei der Steuerung am angezeigten Bildausschnitt 116 zu orientieren, insbesondere an dessen Orientierung.

Dies wird mit ergänzendem Bezug auf Fig. 5 veranschaulicht. Fig. 5 zeigt einen Zustand, in dem der Bildaufnehmer (nicht explizit gezeigt) im Beobachtungskopf 100 des Beobachtungsinstruments 38 derart um die optische Achse 104 ausgerichtet ist, dass der angezeigte Bildausschnitt 116 bei der Wiedergabeeinheit 128 in der gewünschten Orientierung angezeigt ist. Diese Orientierung wird nun der Steuerung der robotischen Handhabungseinheit 34 zugrunde gelegt.

Die Steuerung der Handhabungseinheit 34 erfolgt beispielsweise über ein Betätigungselement 154 des Eingabegerätes 50. Beispielhaft ist das Betätigungselement 154 knopfartig, tellerartig oder puckartig gestaltet. Das Betätigungselement 154 kann jedoch auch ähnlich einem Joystick gestaltet sein. Alternativ kann das Betätigungselement 154 ähnlich einem sogenannten Drehdrücksteller gestaltet sein. Das Betätigungselement 154 weist verschiedene Bewegungsachsen bzw. Eingabeachsen auf. Über diese Eingabeachsen können Steuerbefehle erzeugt werden, indem der Bediener in der gewünschten Weise auf das Betätigungselement 154 einwirkt. Vorzugsweise ist das Betätigungselement 154 als Mehrachs-Betätigungselement gestaltet. Demgemäß ist das Betätigungselement 154 beispielsweise dazu ausgestaltet, Bewegungen entlang mehrerer linearer Achsen 156, 158, 160 zu erfassen. Insgesamt sind beispielsweise sechs Freiheitsgrade denkbar, beispielsweise drei translatorische und drei rotatorische Freiheitsgrade.

Beispielhaft kann die Achse 156 als Translationsachse bezeichnet werden. Die Achse 156 ist beispielhaft einer X-Richtung zugeordnet. Entlang der Achse 156 kann eine Schubbewegung/Linearbewegung induziert werden. Es versteht sich, dass das Betätigungselement 154 entlang der Achse 156 nur in geringem Maße auslenkbar ist. Beispielhaft kann die Achse 158 als Translationsachse bezeichnet werden. Die Achse 158 ist beispielhaft einer Y-Richtung zugeordnet. Entlang der Achse 158 kann eine Schubbewegung/Linearbewegung induziert werden. Es versteht sich, dass das Betätigungselement 154 entlang der Achse 158 nur in geringem Maße auslenkbar ist. Beispielhaft kann die Achse 160 als Hubachse bezeichnet werden. Die Achse 160 ist beispielhaft einer Z-Richtung zugeordnet. Entlang der Achse 160 kann eine Schubbewegung/Linearbewegung induziert werden. Es versteht sich, dass das Betätigungselement 154 entlang der Achse 160 nur in geringem Maße auslenkbar ist.

Dies heißt mit anderen Worten, translatorische Bewegungen des Beobachtungskopfes 100 des Beobachtungsinstruments 38 in einer Ebene (etwa einer X-Y-Ebene) können durch leichte Bewegungen des Betätigungselements 154 entlang der Achsen 156, 158 bewirkt werden.

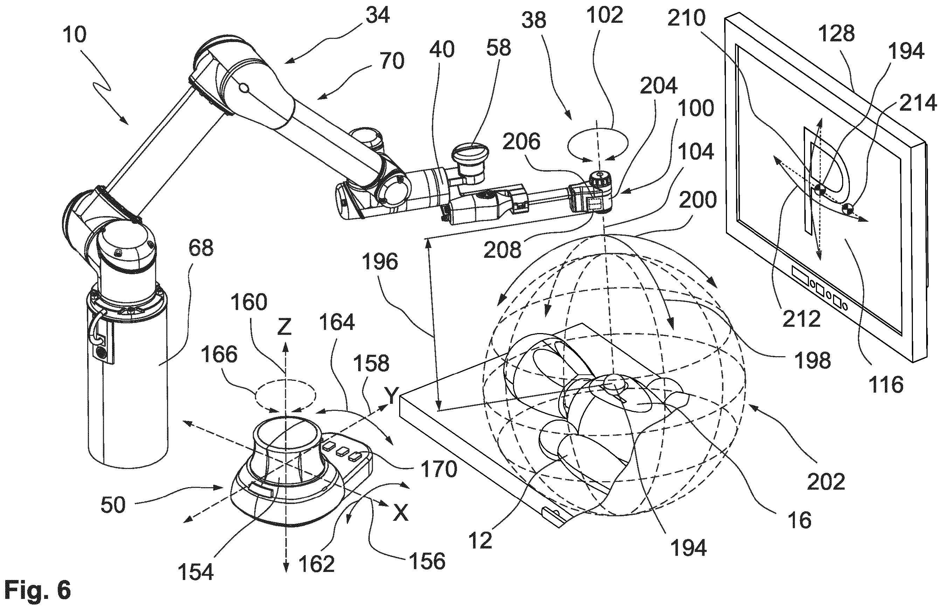

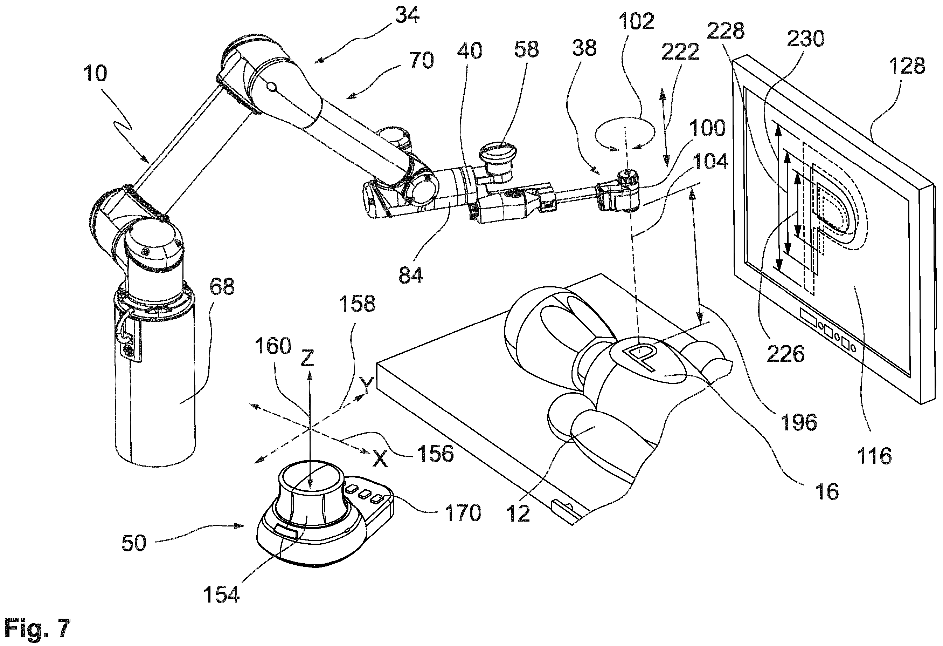

Die Hubachse 160 kann beispielsweise dazu genutzt werden, einen Objektabstand (Bezugszeichen 196 in Fig. 6 und Fig. 7) zwischen dem Beobachtungsinstrument 38 und dem Objektfeld 16 zu verändern. Grundsätzlich ist auch eine Nutzung einer Bewegung entlang der Hubachse 160 (Z-Richtung) zur Steuerung eines Fokusantriebs vorstellbar.

Daneben weist das Betätigungselement 154 gemäß dem in Fig. 5 veranschaulichten Ausführungsbeispiel Schwenkachsen bzw. Rotationsachsen 162, 164, 166 auf. Die Schwenkachse 162 beschreibt Schwenkbewegungen um die Achse 156, also beispielsweise um die X-Achse. Die Schwenkachse 164 beschreibt Schwenkbewegungen um die Achse 158, also beispielsweise um die Y-Achse. Die Schwenkachse oder Rotationsachse 166 beschreibt Schwenkbewegungen/Rotationsbewegungen um die Achse 160, also um die Z-Achse.

Die Schwenkachsen 162, 164 können beispielsweise dazu genutzt werden, dass an der robotischen Handhabungseinheit 34 aufgenommene Beobachtungsinstrument 38 in Bezug auf das Objektfeld 16 zu kippen. Dies erfolgt über eine entsprechende Ansteuerung der Handhabungseinheit 34 in Reaktion auf Schwenkbewegungen um die Schwenkachsen 162, 164, die der Bediener am Betätigungselement 154 vornimmt. In einer beispielhaften Ausgestaltung wird das Beobachtungsinstrument 38 um den Fokuspunkt, also den eingestellten Arbeitsabstand geschwenkt (Pivotbewegung).

Die Rotationsachse 166 kann beispielsweise dazu genutzt werden, einen Fokusantrieb des Beobachtungsinstruments 38 zu steuern. Grundsätzlich ist auch vorstellbar, einen Arbeitsabstand/Objektabstand (Bezugszeichen 196 in Fig. 6 und Fig. 7) zwischen dem Beobachtungsinstrument 38 und dem Objektfeld 16 in Reaktion auf Bedienereingaben (Drehbewegungen) zu verändern. Zwischen diesen Betriebsarten kann unter Nutzung eines Freigabeschalters umgeschaltet werden. Beispielhaft wird die Achse 160 für eine Veränderung des Arbeitsabstands und die Achse 166 für die Steuerung des Fokusantriebs genutzt. Umgekehrt ist es vorstellbar, die Achse 160 für die Steuerung des Fokusantriebs und die Achse 166 für die Veränderung des Arbeitsabstands zu nutzen.

Grundsätzlich ist es vorstellbar, das Betätigungselement 154 in mehreren Raumrichtungen auslenkbar zu gestalten. Auf diese Weise ergibt sich eine eindeutige Bedienung für den Bediener. Es ist jedoch auch vorstellbar, eine Krafteinwirkung auf das Betätigungselement 154 zu erfassen, etwa über geeignete Kraftsensoren. In einem solchen Fall ist das Betätigungselement 154 nicht unbedingt makroskopisch auslenkbar. Stattdessen liegt er eine mikroskopische Auslenkbarkeit vor. Auch auf diese Weise können Bewegungen erfasst sowie den Achsen 156, 158, 160, 162, 164, 166 zugeordnet und auf dieser Basis in Steuerbefehle überführt werden.

Das Eingabegerät 50 weist beispielhaft weitere Betätigungselemente 170 in Form von Tasten oder Knöpfen auf. Auf diese Weise können weitere Funktionen gesteuert werden. Insbesondere können bestimmte Befehle quittiert werden. Ferner ist eine Auswahl eines aktuellen Operationsmodus des Eingabegerätes 50 über eines der Betätigungselemente 170 vorstellbar. Eine weitere Verwendungsmöglichkeit für die Betätigungselemente ist die Speicherung aktueller Positionen der Handhabungseinheit 34 bzw. des Beobachtungsinstruments 38, wobei bedarfsweise die gespeicherte Position ausgehend von einer zwischenzeitlich eingenommenen Position angefahren wird. Sowohl das Speichern einer Position als auch das Anfahren einer zuvor gespeicherten Position kann durch die Betätigungselemente 170 bewirkt werden. Das Anfahren der zuvor gespeicherten Position kann sich einerseits auf die Zielposition beschränken. Alternativ kann das Anfahren der zuvor gespeicherten Position derart erfolgen, dass der zuvor genutzte Bewegungspfad "rückwärts" abgefahren wird.

Im Einklang mit der in Fig. 5 veranschaulichten Beispielkonfiguration erfolgt zur Vereinfachung der Steuerung eine Koordinatentransformation bzw. ein Abgleich der Orientierungen/Koordinatensysteme.

In Fig. 5 veranschaulichen in das Objektfeld 16 projizierte Doppelpfeile 176, 178 ein Koordinatensystem, dass die Orientierung des Bildaufnehmers widerspiegelt. Demgemäß ist es in dem in Fig. 5 gezeigten Betriebsmodus gewünscht, das Beobachtungsinstrument 38 und folglich das Objektfeld 16 in einer Ebene 180 in Reaktion auf Bedienbefehle am Eingabegerät 50 zu verfahren. Eine solche Bewegung in der Ebene 180 erfolgt durch Interpolation und entsprechende Ansteuerung der Glieder der kinematischen Kette 70 der robotischen Handhabungseinheit 34.

In dem an der Wiedergabeeinheit 128 angezeigten Bildausschnitt 116 sind resultierende Bewegungsachsen 186, 188 angedeutet. In dem veranschaulichten beispielhaften Betriebsmodus ist die Achse 156 am Eingabegerät 50 der resultierenden Achse 186 zugeordnet. Ferner ist die Achse 158 am Eingabegerät 50 der resultierenden Achse 188 im Bildausschnitt 116 zugeordnet. Demgemäß wird die robotische Handhabungseinheit 34 derart angesteuert, dass bei einer Rechts-Links-Bewegung am Eingabeelement 154 der angezeigte Bildausschnitt 116 ebenso nach rechts oder nach links verfahren wird. Ferner erfolgt die Ansteuerung der robotischen Handhabungseinheit 34 derart, dass bei einer Vor-Zurück-Bewegung am Eingabeelement 154 der angezeigte Bildausschnitt 116 entlang der angedeuteten Achse 188 hoch oder runter verfahren wird. Eine derartige Bedienung ist intuitiv und kann unter Beobachtung des angezeigten Bildausschnitts 116 an der Wiedergabeeinheit 128 erfolgen.

Dieser Betriebsmodus erfordert jedoch eine Erfassung einer aktuellen Orientierung (gekrümmter Doppelpfeil 102) des Bildaufnehmers im Beobachtungskopf 100 und eine Berücksichtigung dieser Ausrichtung (vergleiche die Doppelpfeile 176, 178) bei der Ansteuerung der robotischen Handhabungseinheit 34. Dies gilt insbesondere bei Verwendung eines Stereo-Bildaufnehmers 118 (vergleiche Fig. 2 und Fig. 3). In Fig. 5 ist beispielsweise der Horizont 140 des Bildaufnehmers 118 (vergleiche erneut Fig. 2 und Fig. 3, vergleiche ebenso den Doppelpfeil 176 in Fig. 5) parallel zur interpolierten Achse 176 ausgerichtet. Diese Ausrichtung wird bei der Interpolation der Bewegungsbahnen für die gewünschten X-Y-Bewegungen in der Ebene 180 berücksichtigt. Mit anderen Worten erfolgt die Koordinatentransformation bei der Umsetzung von Bedienbefehle am Eingabegerät 50 vorzugsweise derart, dass die Achsen 156, 176, 186 hinsichtlich der Steuerbefehle an die Handhabungseinheit 34 parallel zueinander ausgerichtet sind, und dass die Achsen 158, 178, 188 hinsichtlich der Steuerbefehle an die Handhabungseinheit 34 parallel zueinander ausgerichtet sind. Sodann kann in einfacher Weise ein Verschieben des angezeigten Bildausschnitts 116 in der Ebene 180 erfolgen.

Der Bediener kann sich also unabhängig von der äußerlichen Orientierung/Lage der Handhabungseinheit 34 bzw. des Beobachtungsinstruments 38 an der Ausrichtung des Bildausschnitts 116 an der Anzeige der Wiedergabeeinheit 128 orientieren, um den Bildausschnitt 116 über Bedienereingaben an dem zugeordneten Eingabeachsen 156, 158 des Eingabegerätes 50 intuitiv in zumindest zwei Achsen zu steuern.

Zumindest in beispielhaften Ausführungsformen werden während dieses speziellen Verfahrmodus andere Freiheitsgrade des Betätigungselements 154 des Eingabegerätes 50 nicht berücksichtigt, so dass eine ideale oder nahezu-ideale Bewegung in der Ebene 180 ermöglicht ist.

Es versteht sich, dass auch andere Betriebsmodi vorstellbar sind, etwa auch ein räumlicher Modus oder 3D-Modus in dem über das Eingabegerät 50 eine räumliche Steuerung/Bewegung des Beobachtungsinstruments 38 in drei oder mehr Raumachsen (Translationsachsen/Linearachsen und Schwenkachsen/Rotationsachsen) möglich ist.

Fig. 5 veranschaulicht einen Modus, bei dem das Beobachtungsinstrument 38 möglichst parallel mit konstantem Abstand zum beobachteten Objektfeld 16 bewegt wird. Der Objektabstand (vergleiche erneut das Bezugszeichen 196 in Fig. 6 und Fig. 7) bleibt im Wesentlichen gleich.

In Ergänzung hierzu veranschaulicht Fig. 6 einen weiteren Modus, in dem das Beobachtungsinstrument 38 entlang eines gekrümmten Pfades bzw. einer gekrümmten Fläche/Schale in Bezug auf einen Pivotpunkt 194 im Objektfeld 16 bewegt wird. Beispielhaft handelt es sich bei dem Pivotpunkt 194 um ein Zentrum des - einen Teil des Objektfeldes 16 wiedergegebenen - Bildausschnitts 116. Die Bewegung entlang der gekrümmten Schale (beispielsweise Kugelschale oder Kugelhalbschale) erfolgt zumindest in beispielhaften Ausgestaltungen unter Berücksichtigung eines konstanten Objektabstands 196.

Mit anderen Worten kann die Bewegung des Beobachtungsinstruments 38 entlang inter-polierter gekrümmter Achsen 198, 200 erfolgen, die einer sphärischen Fläche 202 (Kugel oder Kugelabschnitt) zugeordnet sind. Beispielhaft, ohne dass dies einschränkend zu verstehen ist, ist die Achse 198 einem 0°-Längengrad und die Achse 200 einem 90°-Längengrad zugeordnet. Die Bewegung entlang/auf der sphärischen Fläche 202 erfolgt unter Wahrung einer Ausrichtung mit der optischen Achse 104 auf den gewählten Pivotpunkt 194. Der Pivotpunkt 194 kann sich aus einem aktuellen Zentrum des beobachteten Bildausschnitts 116 ergeben. Der Pivotpunkt 194 kann jedoch auch außermittig im Bildausschnitt 116 angeordnet und gewählt werden. Der Pivotpunkt 194 kann grundsätzlich auch als Fokuspunkt bezeichnet werden.

In einer beispielhaften Ausführungsform weist das Beobachtungsinstrument 38 beim Beobachtungskopf 100 eine Beobachtungsoptik 204 auf. Beispielhaft ist der Beobachtungsoptik 204 ein Fokusantrieb 206 zur Fokusverstellung zugeordnet. Der Fokusantrieb 206, dient dazu, einen Fokusabstand der Beobachtungsoptik 204 an den gewählten Arbeitsabstand/Objektabstand 196 anzupassen, so dass das aktuell beobachtete Objektfeld 16 hinreichend scharf abgebildet wird. Die Steuerung des Fokusantriebs 206 kann manuell und/oder automatisch erfolgen.

Das Beobachtungsinstrument 38 weist zumindest in beispielhaften Ausführungsformen ferner eine Messeinrichtung 208 zur Ermittlung des Objektabstands 196 auf. Auf diese Weise kann der aktuelle Objektabstand 196 bestimmt werden. Bei der in Fig. 6 veranschaulichten Bewegung des Beobachtungsinstruments 38 entlang der gekrümmten Bahn bzw. Fläche 202 soll der Objektabstand 196 konstant gehalten werden. Dafür muss jedoch zumindest in einigen Ausführungsbeispielen der Objektabstand 196 zunächst noch ermittelt werden. Dies erfolgt über die Messeinrichtung 208.

In einer alternativen Ausgestaltung kann die Steuereinrichtung 44 den Objektabstand 196 mittelbar über aktuelle Betriebsparameter der Beobachtungsoptik 204 bzw. des Fokusantriebs 206 ermitteln. Mit anderen Worten lässt also ein bestimmter Zustand der Beobachtungsoptik 204 auf einen bestimmten Objektabstand 196 schließen.

Beispielhaft erfolgt die Steuerung der Fahrbewegung entlang der gekrümmten Achsen 198, 200 unter Nutzung der Schwenkachsen 162, 164 des Eingabegerätes 50. Mit anderen Worten kann etwa ein Verschwenken des Betätigungselements 154 um die X-Achse 156 (Schwenkachse 162) eine Verfahrbewegung entlang der gekrümmten Achse 198 steuern. Demgemäß kann etwa ein Verschwenken des Betätigungselements 154 um die Y-Achse 158 (Schwenkachse 164) eine Verfahrbewegung entlang der gekrümmten Achse 200 steuern. In Fig. 6 sind resultierende Bewegungen des angezeigten Bildausschnitts 116 an der Wiedergabeeinheit 128 durch gekrümmte Doppelpfeile 210, 212 angedeutet. Es versteht sich, dass die Wiedergabeeinheit 128 im Normalfall einen ebenen Bildschirm aufweist. Insofern sind die Pfeile 210, 212 lediglich aus Veranschaulichungsgründen gekrümmt.

Mit anderen Worten erlaubt der in Fig. 6 gezeigte Modus der Handhabungsvorrichtung 10 ein Umkreisen des Zentrums des Bildausschnitts 116 mit dem Beobachtungsinstrument 38, wobei das Beobachtungsinstrument 38 mit seiner optischen Achse 104 auf das Zentrum ausgerichtet bleibt. Ähnlich einem Planeten kann das Beobachtungsinstrument 38 das Zentrum umkreisen, wobei die optische Achse 104 beispielsweise radial auf das Zentrum ausgerichtet bleibt.

Erneut gilt, dass gerade bei einem Stereo-Bildaufnehmer 118 (Fig. 3 und Fig. 4) die aktuelle Ausrichtung des Bildaufnehmers 118 (Horizont 140) bestimmt und etwa mittels Koordinatentransformation bei der Steuerung berücksichtigt wird, so dass sich der Bediener intuitiv am angezeigten Bildausschnitt 116 orientieren kann. Unter Nutzung der Schwenkachsen 162, 164 erfasste Steuerimpulse führen zu richtungsgleichen Bewegungen des angezeigten Bildausschnitts 116 entlang der resultierenden Achsen/Pfade 210, 212. Die Bedienung wird deutlich vereinfacht.

Es ist grundsätzlich vorstellbar, den Pivotpunkt 194 auch im Zentrum des angezeigten Bildausschnitts 116 zu verorten. Dies kann damit einhergehen, dass der Pivotpunkt 194 schlussendlich auch mit dem Zentrum 124 des Sensors 120, 122 des Bildaufnehmers 118 (vergleiche Fig. 3) zusammenfällt. Diese Weise hat man eine doppelt zentrale Ausrichtung.

Wie vorstehend bereits in Zusammenhang mit Fig. 4 erläutert, kann es auch Situationen geben, in denen das Zentrum des angezeigten Bildausschnitts 116 nicht mit dem Zentrum 124 des Sensors 120, 122 übereinstimmt. In einem solchen Fall ist es gleichwohl vorstellbar, dass der Pivotpunkt 194 im Zentrum des angezeigten Bildausschnitts 116 gewählt wird. Demgemäß liegt ein Versatz zwischen dem Pivotpunkt 194 und dem tatsächlichen Zentrum 124 des Sensors 120, 122 vor. Dieser Versatz wird von der Steuereinrichtung 44, insbesondere von der Handhabungssteuereinrichtung 46, bei der Bewegungsteuerung des Beobachtungsinstruments 38 entlang der gekrümmten Bahn 198, 200 berücksichtigt. Gleichwohl ist das Ziel der Steuerung, den gewählten Pivotpunkt 194 im Zentrum des angezeigten Bildausschnitts 116 zu halten.

Es ist jedoch auch vorstellbar, dass ein Pivotpunkt 214 gewählt wird, der bewusst nicht im Zentrum des angezeigten Bildausschnitts 116 liegt. Es gibt also zwischen dem Zentrum des angezeigten Bildausschnitts 116 und dem gewählten außermittigen Pivotpunkt 214 einen Versatz. Gedanklich wird also ein außermittiger Ankerpunkt als Zentrum der Bewegung des Beobachtungsinstruments auf der gekrümmten Fläche 202 gewählt. Die Steuereinrichtung 44, insbesondere die Handhabungssteuereinrichtung 46, kann dazu ausgestaltet sein, diesen an der Anzeige der Wiedergabeeinheit 128 sichtbaren Versatz während der Bewegung beizubehalten. Mit anderen Worten ist die optische Achse 104 des Beobachtungsinstruments 38 in diesem Modus bewusst nicht auf das Drehzentrum, also den außermittigen Pivotpunkt 214 ausgerichtet.

Es versteht sich, dass die in Fig. 5 und Fig. 6 gezeigten Bedienmodi kombiniert werden können, wenn der Bediener das Betätigungselement 154 sowohl translatorisch (Achsen 156, 158) als auch rotatorisch (Achsen 162, 164) bewegt. Es ist jedoch auch vorstellbar, die beiden Bedienmodi voneinander zu trennen. Demgemäß gibt es einen Parallelverschiebemodus, einen Schwenkmodus, sowie einen kombinierten Modus. Die entsprechenden Modi können beispielhaft über die Betätigungselemente 170 am Eingabegerät 50 oder anderweitig ausgewählt werden.

Fig. 7 veranschaulicht die Nutzung des Eingabegerätes 50 zur Steuerung weiterer Funktionen des optischen Beobachtungsinstruments 38. Beispielhaft kann die Hubachse 160 des Eingabegerätes 50 genutzt werden, um eine Veränderung des Objektabstands 196 herbeizuführen, vergleiche die interpolierte Achse 222. Über diese Funktion kann eine Vergrößerung (Detailerhöhung) bzw. Erhöhung des Abbildungsmaßstabs bewirkt werden, wenn der Beobachtungskopf 100 mit dem Bildaufnehmer 118 näher an die Objektebene/das Objektfeld 16 heranfährt.

Das Resultat einer Veränderung des Arbeitsabstands/Objektabstands 196 wird durch die Doppelpfeile 226, 228, 230 im angezeigten Bildausschnitt 116 veranschaulicht. Wenn der Arbeitsabstand verringert wird, erscheint das wiedergegebene Bild größer. Wenn der Arbeitsabstand erhöht wird, erscheint das wiedergegebene Bild kleiner. Auf diese Weise kann - jedenfalls vom Resultat her betrachtet - eine Zoom-Funktion bewirkt werden. Dies kann über Manipulationen am Betätigungselement 154 bewirkt werden. Dies kann einerseits ein Drücken oder Ziehen entlang der Hubachse 160 (Z-Achse) umfassen. Es ist jedoch auch vorstellbar, diese Funktion über ein Verdrehen des Betätigungselements 154 um die Z-Achse 160 (Rotationsachse 166) herbeizuführen.

Es ist zumindest in beispielhaften Ausführungsformen erforderlich, auch die Gegenstandsweite der optischen Einheit des Beobachtungskopfes 100 anzupassen, damit das Bild bei dem gewählten Objektabstand auch scharf erscheint. Auch hierfür kann einer der Bewegungsfreiheitsgrade (vergleiche die Achsen 160, 166) des Betätigungselements 154 des Eingabegerätes 50 zur Steuerung eines Fokusantriebs genutzt werden.

In alternativen Ausführungsformen ist bei dem Beobachtungskopf 100 eine optische Einheit mit variabler Brennweite verbaut, so dass ein optischer Zoom realisierbar ist. In alternativen Ausführungsformen ist zumindest in Grenzen ein sogenannter Digital-Zoom möglich. Dies ist insbesondere dann der Fall, wenn der wiedergegebene Bildausschnitt 116 kleiner als der theoretisch mögliche Aufnahmebereich 114 des Bildaufnehmers bzw. kleiner als das Sichtfeld der optischen Baugruppe ist. Dann kann der erfasste und/oder wiedergegebene Bildausschnitt 116 innerhalb der durch den Aufnahmebereich 114 vorgegebenen Grenzen zumindest ein Stück weit variiert werden, um eine vergrößerte Detaildarstellung oder verkleinerte Übersichtsdarstellung zu ermöglichen.

Ferner ist es vorstellbar, den Digital-Zoom mit alternativen Maßnahmen zur Bereitstellung vergrößerter/verkleinerter Bildausschnitte zu koppeln, um über ein und dasselbe Eingabegerät 50 eine intuitive Steuerung einer solchen Vergrößerungsfunktion zu ermöglichen. Der Modus, in dem das Eingabegerät 50 genutzt wird, um durch eine Bewegung des Beobachtungsinstruments 38 entlang der interpolierten Achse 222 zur Veränderung des Objektabstands 196 herbeizuführen, kann grundsätzlich gleichzeitig mit den in Fig. 5 und Fig. 6 beschriebenen Modi verwendet werden. Es ist jedoch auch vorstellbar, die einzelnen Modi voneinander zu trennen, um eine eindeutige Bedienung zu ermöglichen.

Ferner ist es in einer beispielhaften Ausgestaltung vorstellbar, eine Verfahrgeschwindigkeit der robotischen Handhabungseinheit und folglich des daran aufgenommenen Beobachtungsinstruments 38 in Abhängigkeit von einer gewählten Zoom-Stufe bzw. in Abhängigkeit von einem gewählten Arbeitsabstands/Objektabstand 196 zu wählen. Demgemäß kann das Beobachtungsinstrument 38 langsamer verfahren werden, wenn eine vergrößerte Darstellung (hoher Zoom-Faktor, geringer Objektabstand bzw. großer Abbildungsmaßstab) gewählt ist. Auf diese Weise kann der sich ändernde Bildausschnitt 116 noch gut für den Beobachter erfasst werden. Umgekehrt kann das Beobachtungsinstrument 38 schneller verfahren werden, wenn eine verkleinerte Darstellung (kleiner Zoom-Faktor, großer Objektabstand bzw. kleiner Abbildungsmaßstab) gewählt ist. Dies ist möglich, da der angezeigte Bildausschnitt 116 einen größeren Bereich des Objektfeldes 16 abdeckt.

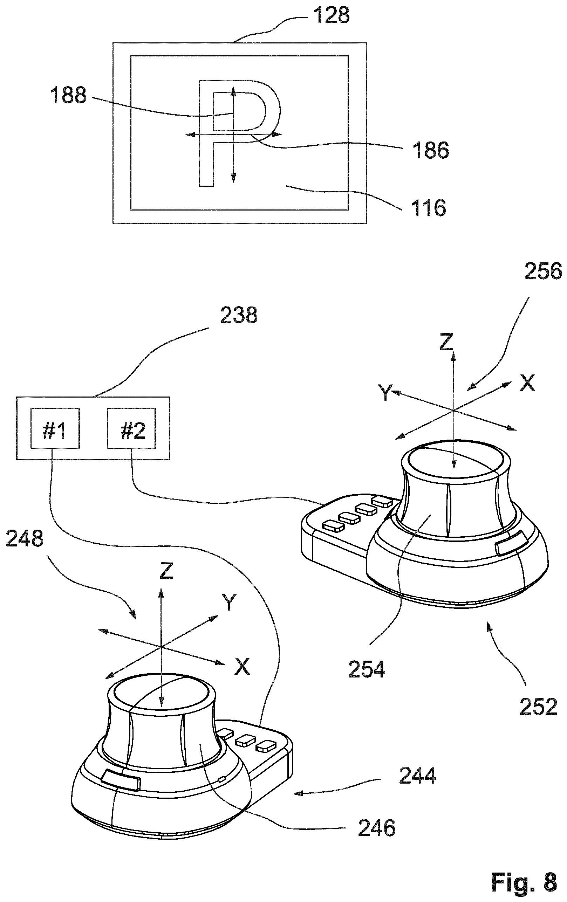

Fig. 8 veranschaulicht in Zusammenschau mit Fig. 1, dass eine Mehrzahl von Eingabegeräten 244, 252 über eine Schnittstelle 238 mit der Steuereinrichtung 44, also etwa der Handhabungssteuereinheit 46 oder der Instrumentensteuereinheit 48 gekoppelt werden kann. In Fig. 8 handelt es sich bei den Eingabegeräten 244, 252 jeweils um Einhand-Eingabegeräte mit einem Eingabeelement 246, 254, welches in diversen Raumachsen betätigbar ist, um Steuerbefehle zu erzeugen. Vergleiche hierzu die den Eingabeelementen 246, 254 zugeordneten Koordinatensysteme 248, 256. Eine Mehrzahl von Eingabegeräten 244, 252 kann erforderlich sein, um verschiedenen Bedienern in der Operationsumgebung Manipulationen zu ermöglichen. Dies kann einerseits eine Steuerung der robotischen Handhabungseinheit 34 betreffen. Ferner kann dies eine Steuerung des Beobachtungsinstruments 38 betreffen, etwa zur Steuerung von Bildaufnahmeparametern und/oder Bildwiedergabeparametern.

Es versteht sich, dass die Steuereinrichtung 44 mit der Schnittstelle 238 eine geeignete Aktivierung/Priorisierung/Hierarchie nutzt, um in eindeutiger Weise zu definieren, welches der Eingabegeräte 244, 252 aktuell verwendet wird. Auf diese Weise kann beispielsweise ein primäres Eingabegerät 244 und ein sekundäres Eingabegerät 252 definiert werden, wobei das primäre Eingabegerät 244 höher priorisiert ist. Demgemäß wird das sekundäre Eingabegerät 252 deaktiviert bzw. werden dessen Befehle ignoriert, wenn das primäre Eingabegerät 244 genutzt wird. Andere Maßnahmen zur Definition des aktuell genutzten Eingabegerätes sind vorstellbar.

Die medizinische Handhabungsvorrichtung 10 kann grundsätzlich über verschiedenartige Eingabegeräte angesteuert werden, vergleiche die Eingabegeräte 50, 52, 54, 56 und 58 in Fig. 1. Fig. 8 zeigt zwei Eingabegeräte 244, 252 des gleichen Typs. Dies ist jedoch nicht einschränkend zu verstehen.

Unabhängig von der aktuellen Position und Orientierung der Eingabegeräte 244, 252 können sich deren Benutzer bei der Steuerung der robotischen Handhabungseinheit 34 am aktuellen Bildausschnitt 116 der Wiedergabeeinheit 128 orientieren. Mit anderen Worten werden die Koordinatensysteme 248, 256 der Eingabegeräte 244, 252 mit den resultierenden Bewegungsachsen 186, 188 des angezeigten Bildausschnitts 116 in Übereinstimmung gebracht.

Ferner ist es vorstellbar, unterschiedliche Wiedergabeeinheiten 128, etwa verschiedene Monitore oder HMDs für die verschiedenen Benutzer vorzusehen. Dies kann Situationen umfassen, in denen die Benutzer unterschiedliche Orientierungen (Drehorientierung) des jeweils angezeigten Bildausschnitts 116 auf der ihnen zugeordneten Wiedergabeeinheit nutzen. Sodann kann das jeweilige Eingabegerät 244, 252 bzw. dessen Koordinatensystems 248, 256 mit der jeweiligen Orientierung des Bildausschnitts in Übereinstimmung gebracht werden, um den Bildausschnitt intuitiv verschieben zu können. Dies kann die Bedienung für verschiedene Bediener deutlich vereinfachen. Gerade im medizinischen Umfeld ist es vorstellbar, dass verschiedene Personen an einer medizinischen Prozedur beteiligt sind. Demgemäß ist es vorstellbar, die Verantwortung für die Bedienung der Handhabungsvorrichtung 10 bzw. der robotischen Handhabungseinheit 34 im zeitlichen Ablauf einer medizinischen Prozedur zwischen den Beteiligten zu wechseln.

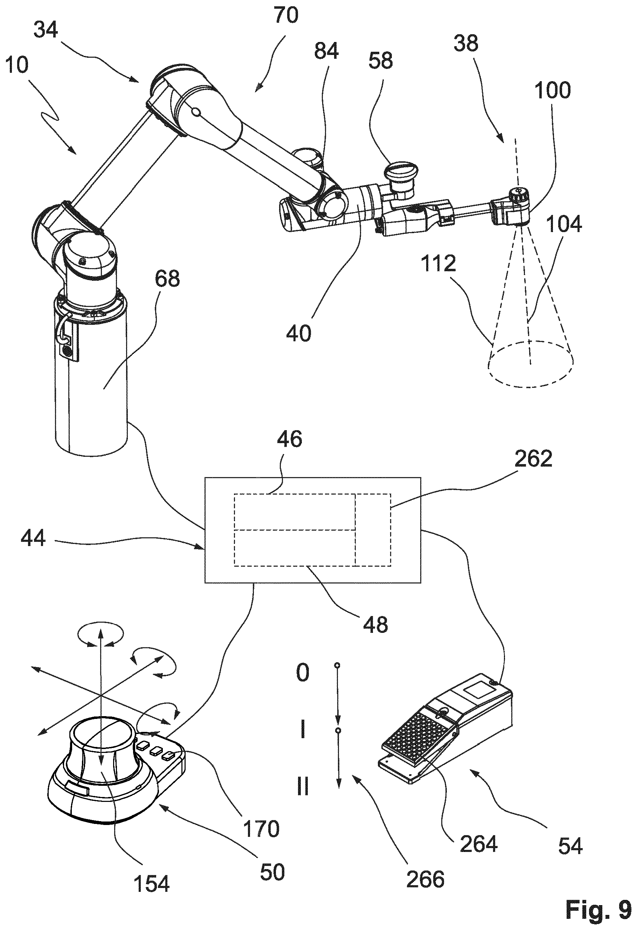

Fig. 9 veranschaulicht in Zusammenschau mit Fig. 1 einen Freigabeprozess zur Erhöhung der Sicherheit bei der Bedienung der Handhabungsvorrichtung 10, insbesondere der robotischen Handhabungseinheit 34. Zu diesem Zweck ist eine Sicherheitseinrichtung 262 vorgesehen, die auch als Freigabesteuerung bezeichnet werden kann. Die Sicherheitseinrichtung 262 kann als Bestandteil der Steuereinrichtung 44 ausgebildet sein, vergleiche die schematische Darstellung der Steuereinrichtung 44 mit der Handhabungssteuereinheit 46 und der Instrumentensteuereinheit 48 in Fig. 9.

Über das Eingabegerät 50 kann die robotische Handhabungseinheit 34 angesteuert werden, so dass Glieder der kinematischen Kette 70 bewegt werden, um das Beobachtungsinstrument 38 zu verfahren. Dies kann im Falle von Irrtümern oder Fehlbedienungen beträchtliche Auswirkungen haben. Demgemäß ist in beispielhaften Ausführungsformen ein zusätzlicher Freigabeprozess unter Nutzung eines Freigabeschalters vorgesehen. Beispielhaft handelt es sich bei dem Freigabeschalter um das Eingabegerät 54 bzw. um dessen Eingabeelement 264. Das Eingabeelement 264 ist beispielsweise als Fußschalter ausgebildet. Der Freigabeschalter hat eine Doppelfunktion, da er zum einen die Bewegung der robotische Handhabungseinheit 34 freigibt und zum anderen zum Hin- und Herschalten zwischen der Steuerung der Beobachtungseinheit 38 und der robotische Handhabungseinheit 34 nutzbar ist.

Es versteht sich, dass grundsätzlich auch das Eingabegerät 56 (vergleiche Fig. 1), welches an der Plattform 22 angeordnet ist, als Freigabeschalter nutzbar ist. Demgemäß kann die Sicherheitseinrichtung 262 derart gestaltet sein, dass eine Steuerung der Handhabungseinheit 34 nur dann möglich ist, wenn ein entsprechender Modus über das Eingabegerät 54 aktiviert ist.

Die Sicherheitseinrichtung 262 stellt sicher, dass nur bewusste Manipulationen der gegenwärtigen Position des Beobachtungsinstruments 38 möglich sind. Fehlbedienungen/unbewusste Steuerungen können vermieden werden.

In beispielhaften Ausführungsformen ist das Eingabegerät 54 mit der Sicherheitseinrichtung 262 fest verdrahtet. Mit anderen Worten kann es sich bei der Sicherheitseinrichtung 262 um eine diskrete Sicherheitseinrichtung handeln, welche nicht nur mittels Software bei der Steuereinrichtung 44 implementiert ist. Auf diese Weise ist die Sicherheitseinrichtung 262 unabhängig gestaltet. Manipulationen werden erschwert. Eine feste Ankopplung des Eingabegerätes 54 (fest verdrahtet bzw. "hard wired") erschwert Manipulationen desselben.

Das Eingabegerät 54 bzw. dessen Eingabeelement 264 weist/weisen zumindest zwei Schaltstellungen auf. Eine erste Schaltstellung (Stufe 0) entspricht beispielsweise einem unbetätigten Zustand. In diesem Zustand kann das Eingabegerät 50 nicht genutzt werden, um die Handhabungseinheit 34 zu steuern und zu bewegen. Das Eingabegerät 50 ist deaktiviert, zumindest im Hinblick auf die Steuerung der Handhabungseinheit 34. Eine zweite Schaltstellung (Stufe I) kann einen Zustand herbeiführen, in dem das Eingabegerät 50 aktiviert ist, so dass Befehle am Eingabegerät 50 von der Steuereinrichtung 44 verarbeitet werden, um die Handhabungseinheit 34 zu steuern und zu bewegen.

Zur weiteren Erhöhung der Sicherheit ist zumindest in beispielhaften Ausgestaltungen eine dritte Stufe (Stufe II) vorgesehen, welche auch als Panik-Modus/Panik-Stufe bezeichnet werden kann. Die zweite Stufe (Stufe I) ist bei dieser Ausgestaltung zwischen der ersten Stufe (Stufe 0) und der dritten Stufe (Stufe II) vorgesehen. Dies heißt mit anderen Worten, der Bediener muss eine bestimmte Mindestkraft aufbringen, um das Eingabeelement 264 des Freigabeschalters aus der ersten Stufe (Deaktivierungszustand) in die zweite Stufe (Aktivierungszustand) zu bringen. Diese Betätigungskraft darf jedoch eine definierte Maximalkraft nicht überschreiten. Beim Überschreiten der Maximalkraft wird nämlich das Eingabeelement 264 des Freigabeschalters aus der zweiten Stufe (Aktivierungszustand) in die dritte Stufe (Deaktivierungszustand oder Panik-Zustand) gebracht. Es muss also eine definierte Betätigungskraft aufgebracht werden, die in einem Bereich zwischen einer Minimalkraft und einer Maximalkraft liegt, um die robotische Handhabungseinheit 34 über das Eingabegerät 50 steuern zu können.

Diese Gestaltung erhöht die Sicherheit weiter. Nämlich dann, wenn der Freigabeschalter in Form des Eingabegerätes 54 unbeabsichtigt mit hoher Kraft betätigt wird, wird nicht zwangsläufig auch das Eingabegerät 50 für Bedienbefehle freigegeben. Stattdessen wird der Aktivierungszustand/Freigabezustand (zweite Stufe bzw. Stufe I) durchlaufen bzw. übersprungen und das Eingabegerät 54 in die dritte Stufe (Stufe II) versetzt.

Es versteht sich, dass der Freigabeschalter auch anderweitig gestaltet sein kann. So ist es vorstellbar, den Freigabeschalter bewusst nur mit einer ersten Stufe (Stufe 0) und einer zweiten Stufe (Stufe I) zu betreiben. Weitere Betätigungselemente können vorgesehen sein, etwa zusätzliche Bedienelemente, welche zusammen mit dem Freigabeschalter betätigt werden müssen. In der in Fig. 9 gezeigten Ausgestaltung ist der Freigabeschalter als Eingabegerät 54 in Form eines Fußschalters gestaltet. Es versteht sich, dass auch handbetätigte Schalter als Freigabeschalter nutzbar sind.

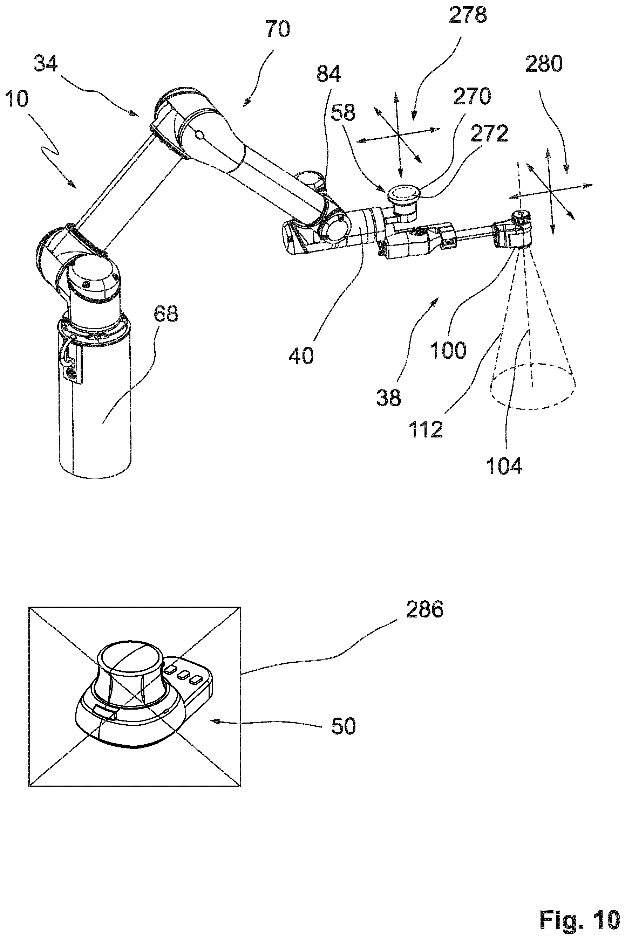

Fig. 10 veranschaulicht einen weiteren beispielhaften Betriebsmodus der Handhabungsvorrichtung 10, insbesondere der Handhabungseinheit 34. Der in Fig. 10 veranschaulichte Betriebsmodus kann auch als Direktsteuermodus bezeichnet werden. Im Direktsteuermodus erfolgt keine Steuerung der robotischen Handhabungseinheit 34 über das Eingabegerät 50, vergleiche den mit 286 bezeichneten durchgestrichenen Block in Fig. 10.

Stattdessen erfolgt im Direktsteuermodus die Steuerung der Handhabungseinheit 34 direkt durch eine Manipulation beziehungsweise einen Angriff an einem Element der kinematischen Kette 70 der Handhabungseinheit 34. Zu diesem Zweck ist in dem in Fig. 10 gezeigten Ausführungsbeispiel das Eingabegerät 58 vorgesehen, welches ein Eingabeelement 270 in Form eines Griffs oder Knopfs aufweist. Der Bediener kann das Eingabeelement 270 ergreifen und im Raum bewegen. Das Eingabegerät 58 mit dem Eingabeelement 270 ist beispielhaft am Instrumentenhalters 40 der robotischen Handhabungseinheit 34 angeordnet, also in unmittelbarer Nähe des aufgenommenen Beobachtungsinstruments 38. Das Eingabegerät 58 kann auch als Direktsteuereingabegerät bezeichnet werden.

Zumindest in einer beispielhaften Ausführungsform weist das Eingabegerät 58 beim Eingabeelement 270 einen Sensor 272 auf, der beispielsweise eine Annäherung oder ein Vorhandensein der Hand des Bedieners erfasst. Auf diese Weise kann die Steuerung der Handhabungseinheit 34 über das Eingabegerät 58 im Direktsteuermodus freigegeben werden.

Es ist vorstellbar, für die Freigabe des Direktsteuermodus unter Nutzung eines Sensors 272 beim Eingabeelement 270 des Eingabegerätes 58 auf eine zusätzliche Freigabe (vergleiche das als Freigabeschalter genutzte Eingabegerät 54 in Fig. 1 und Fig. 9) zu verzichten. Dies ist deshalb vorstellbar, weil der Bediener über das Eingabegerät 58 direkt oder nahezu direkt Einfluss auf die Position und Orientierung des am Instrumentenhalter 40 aufgenommenen Beobachtungsinstruments 38 nehmen kann. In einem solchen Fall ist die Bedienung eindeutig und unmittelbar möglich.

Im Direktsteuermodus werden Kraft- und Wegimpulse, die der Bediener auf das Eingabeelement 270 des Eingabegerät 58 aufbringt, von der Handhabungssteuereinheit 46 (vergleiche Fig. 1) erfasst und ausgewertet, wobei Antriebe der Elemente der robotischen Handhabungseinheit 34 derart angesteuert werden, dass die robotische Handhabungseinheit 34 mit dem Instrumentenhalter 40 und dem daran aufgenommenen Beobachtungsinstrument 38 der induzierten Bewegung am Eingabegerät 58 folgt. Es versteht sich, dass im Direktsteuermodus sowohl translatorische Bewegungen als auch rotatorische Bewegungen/Schwenkbewegungen des an der Handhabungseinheit 34 aufgenommenen Beobachtungsinstruments 38 bewirkt werden können.

Die Erfassung der Bedienimpulse kann etwa durch Überwachung der verschiedenen Achsantriebe der kinematische Kette 70 der Handhabungseinheit 34 erfolgen. Die Bedienimpulse sind in den Achsantrieben spürbar und können folglich erfasst werden. Alternativ können den Achsantrieben entsprechende Sensoren zugeordnet werden.

Alternativ oder zusätzlich ist es vorstellbar, das Eingabegerät 58 ähnlich dem Eingabegerät 50 mit eigenen Bewegungsfreiheitsgraden und entsprechenden Sensoren zu versehen, um Auslenkungen zu erfassen. Es auch vorstellbar, Kraftsensoren/Deformationssensoren beim Eingabegerät 58 bzw. bei dessen Eingabeelement 270 vorzusehen, um zu erfassen, wie der Bediener die Handhabungseinheit 34 mit dem Beobachtungsinstrument 38 bewegen möchte.

Die Steuereinrichtung 44, insbesondere die Handhabungssteuereinheit 46, steuert im Direktsteuermodus die Handhabungseinheit 34 derart, dass diese den Bewegungsimpulsen des Bedieners am Direktsteuer-Eingabegerät 58 folgt, und dass dann, wenn der Bediener nicht mehr auf das Eingabegerät 58 einwirkt, die aktuelle Position und/oder Orientierung beibehalten wird. Auf diese Weise kann der Bediener das Beobachtungsinstrument 38 quasi-manuell bewegen, wobei eine unmittelbare und direkte Rückkopplung erfolgt.

In Fig. 10 ist dem Eingabeelement 270 des Eingabegerätes 58 ein Eingabe-Koordinatensystem 278 zugeordnet. Ein hierzu ausgerichtetes Koordinatensystem 280 veranschaulicht die resultierende interpolierte Bewegung zum Verfahren des Beobachtungsinstruments 38 über Bedienimpulse am Eingabegerät 58. Das Beobachtungsinstrument 38 folgt den Bedienimpulsen am Eingabegerät 58 aufgrund der Nähe zwischen dem Eingabegerät und dem Beobachtungsinstrument 38 unmittelbar.

Die Handhabungssteuereinheit 46 kann derart betrieben werden, dass der Bediener im Direktsteuermodus einen bestimmten, aber nicht zu großen Widerstand (Bremsmoment) bei der Betätigung am Eingabegerät 58 verspürt. Dies erlaubt eine feinfühlige Bewegungs- und Positionsvorgabe im Direktsteuermodus.

Es ist ferner vorstellbar, im Direktsteuermodus durch die Steuereinrichtung 44, insbesondere durch deren Handhabungssteuereinheit 46, den durch den Bediener manuell über das Eingabegerät 58 gesteuerten Bewegungspfad der Handhabungseinrichtung 34 aufzuzeichnen und bedarfsweise "rückwärts" abzufahren. Auf diese Weise kann die Steuereinrichtung 44, insbesondere deren Handhabungssteuereinheit 46, eine Rückkehrfunktion bereitstellen. Das Gerät kann folglich die Startposition oder eine andere Position, welche gespeichert wurde, ausgehen von der gegenwärtigen Position anfahren. Dies ist grundsätzlich auch in anderen Steuermodi vorstellbar, nicht nur im Direktsteuermodus. Das Speichern und Aufrufen gewählter Funktionen kann beispielsweise über die Betätigungselemente 170 am Eingabegerät 50 oder über andere Betätigungselemente gesteuert werden.

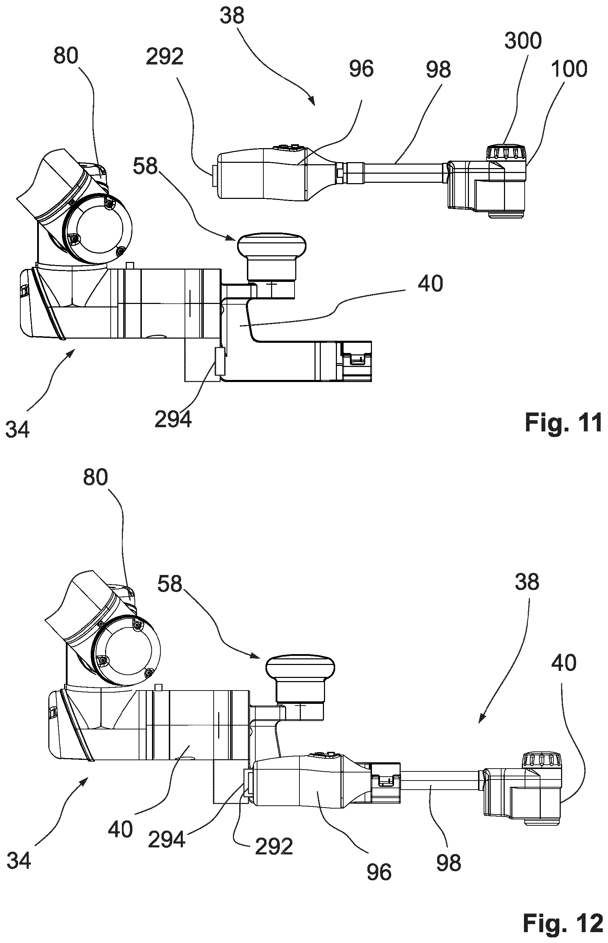

Die Figuren 11 und 12 veranschaulichen die Befestigung des Beobachtungsinstruments 38 am Instrumentenhalter 40 der Handhabungseinheit 34. In Fig. 11 ist das Beobachtungsinstrument 38 vom Instrumentenhalter 40 gelöst. In Fig. 12 ist das Beobachtungsinstrument 38 am Instrumentenhalter 40 befestigt. Neben der mechanischen Kopplung zwischen dem Beobachtungsinstrument 38 und dem Instrumentenhalter 40 ist ferner eine signaltechnische Kopplung über Schnittstellen 292, 294 vorgesehen. Eine Schnittstelle des Beobachtungsinstruments ist mit 292 bezeichnet. Eine Schnittstelle auf Seiten der Handhabungseinheit 34 ist mit 294 bezeichnet. Demgemäß kann gemeinsam mit der mechanischen Kopplung auch eine signaltechnische Kopplung erfolgen.

In Zusammenhang mit der Befestigung des Beobachtungsinstruments 38 kann folglich die Steuereinrichtung 44 der Handhabungsvorrichtung 10 über die Schnittstellen 292, 294 ermitteln, um welchen Instrumententyp es sich bei dem Beobachtungsinstrument 38 handelt. Eine solche Identifikation kann für eine instrumententyp-spezifische Grundeinstellung (Parametersatz) der Handhabungseinheit 34 genutzt werden. Dies kann etwa eine Berücksichtigung gegebener Abmessungen des Beobachtungsinstruments bei der Steuerung der robotischen Handhabungseinheit 34 betreffen. Ferner ist es vorstellbar, über die Schnittstellen 292, 294 Informationen betreffend einen Drehantrieb 300 für den Bildaufnehmer (in Fig. 11 und 12 nicht explizit dargestellt) am Beobachtungskopf 100 auszutauschen. Ferner ist es vorstellbar, Informationen betreffend eine aktuelle Orientierung/Drehlage des Bildaufnehmers zu erlangen.

Über die Schnittstellen 292, 294 können ferner Bildinformationen übertragen werden, also etwa Bildsignale des überwachten Bildausschnitts bzw. Aufnahmebereichs. Zusätzlich werden Informationen übertragen, die für den Betrieb der robotischen Handhabungseinheit 34 nutzbar sind.

Es ist vorstellbar, dass das Beobachtungsinstrument 38 eine Identifikationsinformation (ID) enthält, die über die Schnittstelle 292, 294 abgefragt werden kann. Demgemäß könnte sodann die Steuereinrichtung 44 auf Basis dieser Informationen einen Parametersatz betreffend das Beobachtungsinstrument 38 abfragen, etwa bei einer Datenbank. Ferner ist es vorstellbar, dass das Beobachtungsinstrument 38 selbst diese Informationen über die Schnittstelle 292, 294 bereitstellt.

Die in den Figuren 11 und 12 veranschaulichte Gestaltung betrifft den Informationsaustausch zwischen dem Beobachtungsinstrument 38 und einerseits der Handhabungssteuereinheit 46 und andererseits der Instrumentensteuereinheit 48. Entsprechende Signale/Informationen werden unter Zwischenschaltung der Handhabungseinheit 34 übertragen.

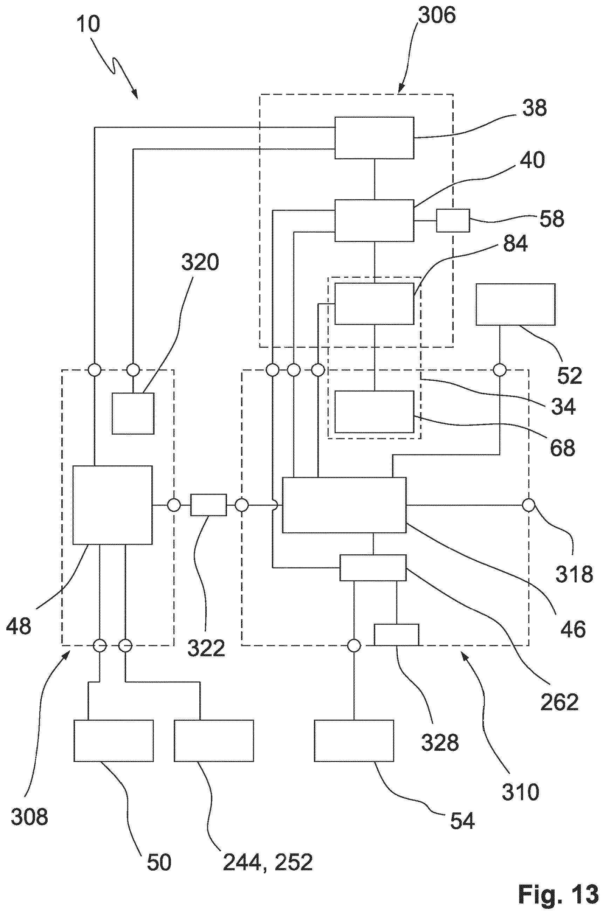

Fig. 13 zeigt anhand einer schematischen Darstellung ein Blockschaltbild zur Veranschaulichung einer beispielhaften funktionalen Ausgestaltung/Systemarchitektur einer Handhabungsvorrichtung 10. In struktureller Hinsicht kann die in Fig. 10 gezeigte Handhabungsvorrichtung 10 grundsätzlich der anhand der Figuren 1 bis 12 veranschaulichten Gestaltung der Handhabungsvorrichtung 10 entsprechen.

Die Handhabungsvorrichtung 10 weist einen Beobachtungsabschnitt 306, einen Instrumentensteuerungsabschnitt 308 und einen Handhabungsabschnitt 310 auf. Es handelt sich bei den Abschnitten 306, 308, 310 um funktional abgegrenzte Abschnitte. Es handelt sich nicht unbedingt um strukturell abgegrenzte Abschnitte. Der Beobachtungsabschnitt 306 umfasst das am Halter 40 aufgenommene Beobachtungsinstrument 38. Dem Beobachtungsabschnitt 306 ist ferner das Eingabegerät 58 für den Direktsteuermodus zugeordnet. Das Eingabegerät 58 weist beispielhaft einen Sensor zur Aktivierung des Direktsteuermodus auf.

Eine mechanische Verbindung des Beobachtungsabschnitts 306 mit dem Handhabungsabschnitt 310 erfolgt über die robotische Handhabungseinheit 34. Dort ist beispielsweise die Hand 84 vorgesehen, welche den Instrumentenhalter 40 trägt, vergleiche Fig. 2. Die Handhabungseinheit 34 umfasst ferner eine Basis bzw. ein Gestell 68, welches dem Handhabungsabschnitt 310 zugeordnet ist. Der Handhabungsabschnitt 310 umfasst ferner die Handhabungssteuereinheit 46 zur Steuerung der Handhabungseinheit 34. Der Handhabungseinheit 46 ist eine Schnittstelle 318 zur Energieversorgung vorgesehen. Die Schnittstelle 318 kann auch zum Informationsaustausch und/oder zur Medienversorgung dienen.

Darüber hinaus ist dem Handhabungsabschnitt 310 - in struktureller Hinsicht - auch das Eingabegerät 52 zugeordnet, welches beispielhaft als Touch-Monitor gestaltet ist. Das Eingabegerät 52 ist mit der Handhabungssteuereinheit 46 und folglich auch mit der (globalen) Steuereinrichtung 44 gekoppelt. Der Handhabungsabschnitt 310 umfasst ferner die Sicherheitseinrichtung 262, welche auch als Freigabesteuerung bezeichnet werden kann. Die Sicherheitseinrichtung 262 ist beispielsweise mit dem als Freigabeschalter gestalteten Eingabegerät 54 gekoppelt. Signaltechnisch ist die Sicherheitseinrichtung 262 beispielsweise mit der Handhabungssteuereinheit 46 gekoppelt, um Betriebsmodi der Handhabungssteuereinheit 46 und folglich der Handhabungseinheit 34 zu aktivieren oder zu sperren.

In dem Ausführungsbeispiel gemäß Fig. 13 ist die Sicherheitseinrichtung 262 ferner mit einem Feststell-Sensor 328 gekoppelt, der überwacht, ob die die Handhabungseinheit 34 tragende Plattform 22 bzw. der Wagen 24 gebremst und gesichert ist. Dies ist zumindest in beispielhaften Ausgestaltungen auch eine Bedingung für eine Freigabe der Bewegung der Handhabungseinheit 34.

Der Instrumentensteuerungsabschnitt 308 umfasst im Wesentlichen die Instrumentensteuereinheit 48, also die CCU/Konsole zur Überwachung und Steuerung des Beobachtungsinstruments 38. Demgemäß ist die Instrumentensteuereinheit 48 über zumindest eine Signalleitung mit dem Beobachtungsinstrument 38 gekoppelt. Ferner ist im Ausführungsbeispiel gemäß Fig. 13 eine Lichtquelle 320 vorgesehen, welche zu Beleuchtungszwecken mit dem Beobachtungsinstrument 38 gekoppelt ist.

Fig. 13 veranschaulicht ferner Leitungen zum Signalaustausch bzw. Informationsaustausch zwischen dem Beobachtungsabschnitt 306, dem Instrumentensteuerungsabschnitt 308 und dem Handhabungsabschnitt 310

Ferner ist zumindest ein Eingabegerät 50, 244, 252 über eine geeignete Schnittstelle mit der Instrumentensteuereinheit 48 gekoppelt. Es ist möglich, eine Mehrzahl von Eingabegeräten 50, 244, 252 vorzusehen, so dass verschiedene Bediener die Handhabungsvorrichtung 10 steuern können. Die Instrumentensteuereinheit 48 und das zumindest eine Eingabegerät 50, 244, 252 sind derart gestaltet, dass diese auch in einem handgeführten Modus des Beobachtungsinstruments 38 zur Steuerung verwendbar sind. Mit anderen Worten koordiniert die Instrumentensteuereinheit 48 die Bildaufnahme und gegebenenfalls die Bildwiedergabe. Ebenso ist das zumindest eine Eingabegerät 50, 244, 252 zur Steuerung von Bildaufnahmeparametern und Bildwiedergabeparametern verwendbar.

Es ist jedoch gemäß der in Fig. 13 veranschaulichten Ausgestaltung vorgesehen, das zumindest eine mit der Instrumentensteuereinheit 48 gekoppelte Eingabegerät 50, 244, 252 zusätzlich auch zur Steuerung der robotischen Handhabungseinheit 34 zu nutzen. Zu diesem Zweck ist die Instrumentensteuereinheit 48 mit der Handhabungssteuereinheit 46 verbunden, beispielsweise über eine Schnittstelle 322 zum Informationsaustausch. Die Schnittstelle 322 kann auch als Netzwerk-Schnittstelle oder Datenbus-Schnittstelle bezeichnet werden.

Diese Konfiguration führt dazu, dass in einem Modus, in dem das oder die Eingabegeräte 50, 244, 252 nutzbar ist/sind, um die robotische Handhabungseinheit 34 zur Bewegung des daran aufgenommenen Beobachtungsinstruments 38 zu steuern, die Instrumentensteuereinheit 48 die entsprechenden Steuersignale nicht selbst umfänglich verarbeitet, sondern diese über die Schnittstelle 322 an die Handhabungssteuereinheit 46 weiterleitet bzw. durchleitet. Ein solcher Modus wird beispielsweise über das als Freigabeschalter 54 gestaltete Eingabegerät unter Nutzung der Sicherheitseinrichtung 262 freigegeben.

Ein Vorteil dieser Gestaltung ist, dass die Instrumentensteuereinheit 48 weiterhin unabhängig und selbstständig zur Steuerung des Beobachtungsinstruments 38 verwendbar ist, etwa in einem handgehaltenen/handgeführten Modus. Dies gilt auch für mögliche Eingabegeräte 50, die direkt mit der Instrumenten Steuereinheit 48 gekoppelt sind. Dies ist auch ohne Handhabungseinheit 34 sowie deren Handhabungssteuereinheit 46 möglich.

Gleichwohl können die erweiterten Nutzungsumfänge durch die Bereitstellung der robotischen Handhabungseinheit 34 unter Nutzung ein und desselben Eingabegerätes 50 gesteuert werden. Es ist also nicht unbedingt notwendig, für die Steuerung der Handhabungseinheit 34 ein zusätzliches Eingabegerät 50 vorzusehen. Stattdessen kann das Eingabegerät 50 in verschiedenen Modi zur Instrumentensteuerung und zur Handhabungssteuerung genutzt werden.

Dies ist insbesondere dann vorstellbar, wenn das Eingabegerät 50 ein Mehrachs-Eingabegerät ist. Derartige Eingabegeräte (vergleiche etwa eine sogenannte 3D-Maus) eignen sich gut für beide Steuermodi. Es ergibt sich der Vorteil, dass ein und dieselbe Anordnung für einen handgehaltenen Betrieb des Beobachtungsinstruments 38 und einen durch die robotische Handhabungseinheit 34 unterstützten Betrieb nutzbar ist. Es ist nicht unbedingt notwendig, zweimal in das Beobachtungsinstrument 38 und die diesem zugeordnete Instrumentensteuereinheit 48 zu investieren. Gleichwohl wird eine einfache und sichere Bedienung der erweiterten Anordnung der Handhabungsvorrichtung 10 mit der robotischen Handhabungseinheit 34 gewährleistet.

Ein weiterer Aspekt der vorliegenden Offenbarung betrifft die Nutzung von aktuellen Betriebsparametern bzw. allgemeinen Parametern des Beobachtungsinstruments 38 durch die Steuereinrich-tung 44, insbesondere durch deren Handhabungssteuereinheit 46, für die Steuerung der robotischen Handhabungseinheit 34. Insbesondere sind beispielhafte Ausgestaltungen vorstellbar, bei denen die Fahrgeschwindigkeit der robotischen Handhabungseinheit 34 von Betriebsparametern des Beobachtungsinstruments 38 abhängig gemacht wird. Dies kann beispielsweise einen aktuellen Objektabstand betreffen. Bei einem großen Objektabstand kann eine hohe Verfahrgeschwindigkeit und bei einem kleinen Objektabstand eine kleine Verfahrgeschwindigkeit zur Bewegung des Beobachtungsinstruments 38 gewählt werden.

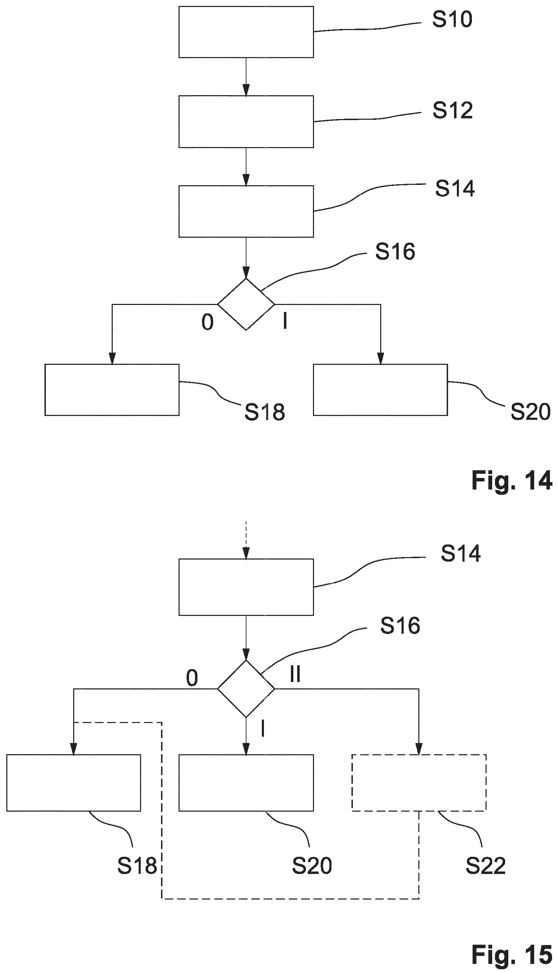

Mit Bezugnahme auf Fig. 14 wird anhand eines schematischen Blockdiagramms eine beispielhafte Ausführungsform eines Verfahrens zur Steuerung einer Handhabungsvorrichtung veranschaulicht. Bei der Handhabungsvorrichtung kann es sich um eine medizinische Handhabungsvorrichtung handeln. Es kann sich jedoch auch um eine nicht-medizinische Handhabungsvorrichtung handeln.

Das Verfahren umfasst einen Schritt S10, der die Bereitstellung eines Instruments und dessen Anordnung an einem Instrumentenhalter an einer robotischen Handhabungseinheit umfasst. Bei dem Instrument handelt es sich beispielhaft um ein Beobachtungsinstrument. Es folgt ein Schritt S12, der die Steuerung der Handhabungsvorrichtung mit einem Eingabegerät umfasst, wobei sich die Steuerung auf die Steuerung der robotischen Handhabungseinheit und/oder des Instruments bezieht. Mit anderen Worten gibt es verschiedene Betriebsmodi für die Steuereinrichtung bzw. das Eingabegerät. Beispielhaft umfasst die Steuereinrichtung eine Instrumentensteuereinheit zur Steuerung des Instruments und eine Handhabungssteuereinheit zur Steuerung der robotischen Handhabungseinheit. Das Eingabegerät ist sowohl zur Steuerung der robotischen Handhabungseinheit als auch zur Steuerung des Instruments nutzbar.

In einem weiteren Schritt S14 wird ein Freigabeschalter überwacht. Bei dem Freigabeschalter handelt es sich beispielsweise um einen Fußschalter. Andere Gestaltungen sind denkbar. Der Freigabeschalter kann verschiedene Stellungen/Zustände aufweisen.

In einem Schritt S16 wird festgestellt, ob sich der Freigabeschalter in einem ersten Zustand 0 oder einem zweiten Zustand I befindet. Der erste Zustand 0 entspricht einem nicht aktivierten Zustand. Der zweite Zustand I entspricht einem aktivierten Zustand. Beispielsweise kann der Bediener eine bestimmte Betätigungskraft aufbringen, um den Freigabeschalter vom ersten Zustand 0 in den zweiten Zustand I zu überführen.

Sofern sich der Freigabeschalter im ersten Zustand 0 befindet, folgt ein Schritt S18. Demgemäß befindet sich das Eingabegerät in einem ersten Betriebsmodus. Im ersten Betriebsmodus kann das Instrument gesteuert werden. Im ersten Betriebsmodus ist jedoch keine Steuerung der robotischen Handhabungseinheit zur Bewegung des Instruments vorgesehen. Dies führt dazu, dass im ersten Betriebsmodus Relativbewegungen zwischen dem Instrument und einem Zielobjekt, etwa einem Beobachtungsobjekt, reduziert oder vermieden werden.

Wird jedoch im Schritt S16 festgestellt, dass sich der Freigabeschalter im zweiten Zustand I befindet, so folgt ein Schritt S20. Im Schritt S20 ist das Eingabegerät in einem zweiten Betriebsmodus betreibbar. Im zweiten Betriebsmodus kann das Eingabegerät folglich genutzt werden, um die robotische Handhabungseinheit zur Bewegung des Instruments anzusteuern. Dies ist jedoch nur nach expliziter Freigabe durch Betätigung des Freigabeschalters möglich. Auf diese Weise kann der zweite Betriebsmodus nur über eine zusätzliche Freigabe desselben aktiviert werden. Dies reduziert die Wahrscheinlichkeit einer unbewussten Aktivierung des zweiten Betriebsmodus.

Fig. 15 veranschaulicht anhand eines schematischen Blockdiagramms eine weitere beispielhafte Ausgestaltung eines Verfahrens zur Steuerung einer Handhabungsvorrichtung. Das in Fig. 15 veranschaulichte Verfahren basiert auf der in Fig. 14 gezeigten Gestaltung. Hinsichtlich der Schritte S10 bis S14 wird daher auf die vorstehenden Erläuterungen in Zusammenhang mit Fig. 14 verwiesen.

Die in Fig. 15 gezeigte Ausgestaltung nutzt einen Freigabeschalter, der drei Zustände/Stellungen 0, I und II aufweist. Demgemäß wird im Schritt S16 festgestellt, ob sich der Freigabeschalter im ersten Zustand 0, im zweiten Zustand I oder im dritten Zustand II befindet. Der erste Zustand 0 entspricht einem nicht aktivierten Zustand. Der zweite Zustand I entspricht einem aktivierten Zustand. Der dritte Zustand II wird erreicht, wenn sich die Betätigungskraft des Bedieners auf den Freigabeschalter über eine definierte Grenze erhöht. Auf diese Weise kann die Gefahr von Fehlbedienungen weiter verringert werden. Nämlich dann, wenn der Freigabeschalter unbeabsichtigt mit hoher Kraft betätigt wird, so geht der Freigabeschalter ausgehend vom ersten Zustand 0 über den zweiten Zustand I in den dritten Zustand II über. Mit anderen Worten muss der Bediener die Betätigungskraft in einem bestimmten Bereich halten, um den zweiten Zustand I des Freigabeschalters herbeizuführen und beizubehalten. Mit anderen Worten ist der Freigabeschalter dreistufig gestaltet, wobei sich die zweite Stufe (Aktivierung des zweiten Betriebsmodus) zwischen der ersten Stufe und der dritten Stufe befindet.

Sofern im Schritt S16 festgestellt wird, dass sich der Freigabeschalter im ersten Zustand 0 befindet, folgt ein Schritt S18. Im Schritt S18 befindet sich das Eingabegerät im ersten Betriebsmodus. Sofern im Schritt S16 festgestellt wird, dass sich der Freigabeschalter im zweiten Zustand I befindet, folgt ein Schritt S20. Im Schritt S20 befindet sich das Eingabegerät im zweiten Betriebsmodus.

Sofern im Schritt S16 festgestellt wird, dass sich der Freigabeschalter im dritten Zustand II befindet, folgt ein Schritt S22. Der Schritt S22 kann grundsätzlich als Not-Aus Funktion gestaltet sein. Demgemäß sind zumindest Teilfunktionen der Handhabungsvorrichtung deaktiviert, wenn sich der Freigabeschalter im dritten Zustand befindet. Alternativ ist es vorstellbar, dass der dritte Zustand II des Freigabeschalters ähnlich dem ersten Zustand 0 behandelt wird. Demgemäß befindet sich das Eingabegerät im ersten Betriebsmodus (vergleiche die optionale, gestrichelte Linie zwischen den Schritten S22 und S18).

Die Verwendung des Freigabeschalters erhöht die Bediensicherheit. Die Gefahr von Fehlbedienungen lässt sich verringern. Relativbewegungen des Instruments gegenüber dem Zielobjekt/Patienten können nach erfolgter Freigabe mit demjenigen Eingabegerät gesteuert werden, welches für die Steuerung instrumenteneigener Funktionen vorgesehen ist.

## Patentansprüche

1. Medizinische Handhabungsvorrichtung, die Folgendes aufweist:
- einen Instrumentenhalter (40) zur Aufnahme eines Instruments (36), insbesondere eines Beobachtungsinstruments (38),
- eine robotische Handhabungseinheit (34), die den Instrumentenhalter (40) trägt,
- eine Steuereinrichtung (44) mit einer Handhabungssteuereinheit (46) zur Steuerung der robotischen Handhabungseinheit (34) und einer Instrumentensteuereinheit (48) zur Steuerung des Instruments (36),
wobei die Steuereinrichtung (44) eine Schnittstelle (238) für zumindest ein Eingabegerät (50) aufweist, und
- ein mit der Steuereinrichtung (44) koppelbares Eingabegerät (50), das mit der Schnittstelle (238) gekoppelt ist,
wobei das Eingabegerät (50) in einem ersten Betriebsmodus zur Steuerung des Instruments (36) und in einem zweiten Betriebsmodus zur Steuerung der robotischen Handhabungseinheit (34) nutzbar ist,
wobei die Steuereinrichtung (44) mit dem Instrument (36) und mit der robotischen Handhabungseinheit (34) koppelbar ist,
wobei die Steuereinrichtung (44) dem Eingabegerät (50) und dem am Instrumentenhalter (40) aufgenommenen Instrument (36) zwischengeschaltet ist, und
wobei die Steuereinrichtung (44) dazu ausgebildet ist, im zweiten Betriebsmodus über das Eingabegerät (50) erfasste Steuerbefehle für die robotische Handhabungseinheit (34) der Handhabungssteuereinheit (46) über die Instrumentensteuereinheit (48) bereitzustellen, weiterhin aufweisend
- einen als Fußschalter gestalteten Freigabeschalter (54) zur Aktivierung des zweiten Betriebsmodus, in dem die robotische Handhabungseinheit (34) in Reaktion auf Eingabebefehle am Eingabegerät (50) motorisch verfahrbar ist, um das Instrument (36) motorisch zu bewegen,
wobei der Freigabeschalter (54) zwei Zustände (0, I) aufweist, wobei ein erster Zustand (0) ein Ursprungszustand ist, und wobei ein zweiter Zustand (I) ein Aktivierungszustand für den zweiten Betriebsmodus ist,
wobei der Freigabeschalter (54) einen dritten Zustand (II) aufweist, wobei der Freigabeschalter (54) bei Aktivierung ausgehend vom ersten Zustand (0) durch Aufbringen einer Betätigungskraft zunächst in den zweiten Zustand (I) und danach bei erhöhter Betätigungskraft in den dritten Zustand (II) überführbar ist, und
wobei sowohl im ersten Zustand (0) als auch im dritten Zustand (II) des Freigabeschalters (54) die Steuerung der robotischen Handhabungseinheit (34) gesperrt ist.

2. Handhabungsvorrichtung nach Anspruch 1, wobei die Steuereinrichtung (44) signaltechnisch dem Eingabegerät (50) und der robotischen Handhabungseinheit (34) zwischengeordnet ist.

3. Handhabungsvorrichtung nach einem der Ansprüche 1 oder 2, wobei der Freigabeschalter (54) direkt mit einer Handhabungssteuereinheit (46) verbunden ist, die der robotischen Handhabungseinheit (34) zugeordnet ist.

4. Handhabungsvorrichtung nach einem der Ansprüche 1 bis 3, wobei die Steuerbefehle für die robotische Handhabungseinheit (34) im zweiten Betriebsmodus über die Instrumentensteuereinheit (48) übermittelt werden, welche die Steuerbefehle weiterleitet.

5. Handhabungsvorrichtung nach einem der Ansprüche 1 bis 4, wobei das Eingabegerät (50) als Mehrachs-Eingabegerät (50) gestaltet ist und Bedienbewegungen in Form von Schubbewegungen oder Schwenkbewegungen in zumindest zwei Achsen (156, 158, 160, 162, 164, 166) erfasst, wobei die Bedienbewegungen in Steuerbefehle für die robotische Handhabungseinheit (34) überführt werden, und vorzugsweise wobei die robotische Handhabungseinheit (34) eine mehrgliedrige Kinematik (70) mit einer Mehrzahl von Koppelgliedern (72, 76, 80, 84) umfasst, wobei die Steuereinrichtung (44) dazu ausgebildet ist, die Bewegungsvorgaben mittels Interpolation in Steuerbefehle für Bewegungsachsen der Koppelglieder (72, 76, 80, 84) zu überführen.

6. Handhabungsvorrichtung nach einem der Ansprüche 1 bis 5, wobei die Steuereinrichtung (44) dazu ausgebildet ist, eine aktuelle Position und Orientierung des Instruments (36) zu speichern und bedarfsweise aufzurufen, und wobei die Steuereinrichtung (44) vorzugsweise dazu ausgebildet ist, eine Mehrzahl von vorgegebenen Positionen und Orientierungen des Instruments (36) abzuspeichern, um diese bedarfsweise aufzurufen.

7. Handhabungsvorrichtung nach einem der Ansprüche 1 bis 6, wobei die Steuereinrichtung (44) dazu ausgestaltet ist, die Handhabungseinheit (34) derart anzusteuern, dass das Instrument (36) durch interpolierte Bewegung der Handhabungseinheit (34) um eine virtuelle Kippachse verschwenkbar ist, die parallel zum Bildaufnehmer (118) angeordnet ist.

8. Handhabungsvorrichtung nach einem der Ansprüche 1 bis 7, wobei die Steuereinrichtung (44) dazu ausgestaltet ist, die robotische Handhabungseinheit (34) in einem Direktsteuermodus zu betreiben, um das Instrument (36) im Raum zu bewegen und auszurichten, wobei Bedienbefehle an der robotischen Handhabungseinheit (34) durch Einwirkung auf ein dem Instrument benachbartes Element (40) der Handhabungseinheit (34) erzeugbar sind, und wobei die Handhabungssteuereinheit (46) dazu ausgestaltet ist, die robotische Handhabungseinheit (34) derart anzusteuern, dass das Instrument (36) der induzierten Bewegung folgt.

9. Handhabungsvorrichtung nach Anspruch 8, wobei ein Bedienelement (270) zur Steuerung der robotischen Handhabungseinheit (34) im Direktsteuermodus vorgesehen ist, und wobei das Bedienelement (270) einen Sensor (272) zur Erzeugung eines Freigabesignals für den Direktsteuermodus aufweist.

10. Handhabungsvorrichtung nach einem der Ansprüche 1 bis 9, wobei die robotische Handhabungseinheit (34) auf einem Wagen (24) montiert ist, und wobei eine Ansteuerung der robotischen Handhabungseinheit (34) zur Bewegung des Instruments (36) nur dann ermöglicht ist, wenn der Wagen (24) blockiert ist.

11. Handhabungsvorrichtung nach einem der Ansprüche 1 bis 10, wobei die Schnittstelle (238) mit einer Mehrzahl von Eingabegeräten (244, 252) koppelbar ist, über die die robotische Handhabungseinheit (34) steuerbar ist, wobei die Eingabegeräte unterschiedlich priorisierbar (244, 252) sind.

12. Nicht-chirurgisches Verfahren zur Steuerung einer Handhabungsvorrichtung, die eine robotische Handhabungseinheit (34) mit einem Instrumentenhalter (40) und einem daran aufgenommenen Instrument (36) umfasst, wobei das Verfahren die folgenden Schritte aufweist:
- Bereitstellung einer robotischen Handhabungseinheit (34) mit einem Instrumentenhalter (40) und einem Eingabegerät (50),
- Bereitstellung des Instruments (36) am Instrumentenhalter,
wobei das Eingabegerät (50) in einem ersten Betriebsmodus zur Steuerung des Instruments (36) und in einem zweiten Betriebsmodus zur Steuerung der robotischen Handhabungseinheit (34) nutzbar ist, und wobei die Steuerung des Instruments (36) und der robotischen Handhabungseinheit (34) unter Nutzung des Eingabegerätes (50) über eine Steuereinrichtung (44) erfolgt, mit der das Eingabegerät (50) koppelbar ist,
- Aktivierung des zweiten Betriebsmodus, in dem die robotische Handhabungseinheit (34) in Reaktion auf Eingabebefehle am Eingabegerät (50) motorisch verfahrbar ist, um das Instrument (36) motorisch zu bewegen, durch Betätigung eines als Fußschalter gestalteten Freigabeschalters (54),
wobei der Freigabeschalter (54) zwei Zustände (0, I) aufweist, wobei ein erster Zustand (0) ein Ursprungszustand ist, und wobei ein zweiter Zustand (I) ein Aktivierungszustand für den zweiten Betriebsmodus ist,
wobei der Freigabeschalter (54) einen dritten Zustand (II) aufweist, wobei der Freigabeschalter (54) bei Aktivierung ausgehend vom ersten Zustand (0) durch Aufbringen einer Betätigungskraft zunächst in den zweiten Zustand (I) und danach bei erhöhter Betätigungskraft in den dritten Zustand (II) überführbar ist,
wobei sowohl im ersten Zustand (0) als auch im dritten Zustand (II) des Freigabeschalters (54) die Steuerung der robotischen Handhabungseinheit (34) gesperrt ist,
wobei die Steuereinrichtung (44) mit dem Instrument (36) und mit der robotischen Handhabungseinheit (34) koppelbar ist,
wobei die Steuereinrichtung (44) dem Eingabegerät (50) und dem am Instrumentenhalter (40) aufgenommenen Instrument (36) zwischengeschaltet ist, und
wobei die Steuereinrichtung (44) dazu ausgebildet ist, im zweiten Betriebsmodus über das Eingabegerät (50) erfasste Steuerbefehle für die robotische Handhabungseinheit (34) der Handhabungssteuereinheit (46) über die Instrumentensteuereinheit (48) bereitzustellen.

13. Verfahren nach Anspruch 12, wobei der Freigabeschalter (54) in Abhängigkeit von einer aufgebrachten Betätigungskraft im ersten Zustand (0), zweiten Zustand (I) und dritten Zustand (II) betreibbar ist, wobei im ersten Zustand (0) und im dritten Zustand (II) der zweite Betriebsmodus deaktiviert ist, und wobei beim Schalten des Freigabeschalters (54) der zweite Zustand (I) dem ersten Zustand (0) und dem dritten Zustand (II) zwischengeordnet ist.

## Claims

1. A medical handling device, comprising:
- an instrument holder (40) for holding an instrument (36), in particular an observation instrument (38),
- a robotic handling unit (34), which supports the instrument holder (40),
- a control device (44) comprising a handling control unit (46) for controlling the robotic handling unit (34) and an instrument control unit (48) for controlling the instrument (36),
wherein the control device (44) comprises an interface (238) for at least one input device (50), and
- an input device (50) that is arranged to be coupled to the control device (44) and coupled to the interface (238),
wherein the input device (50) is operable in a first operating mode for controlling the instrument (36) and in a second operating mode for controlling the robotic handling unit (34),
wherein the control device (44) is arranged to be coupled to the instrument (36) and to the robotic handling unit (34),
wherein the control device (44) is interposed between the input device (50) and the instrument (36) supported by the instrument holder (40), and
wherein the control device (44) is adapted to provide in the second operating mode control commands, which have been detected via the input device (50), for the robotic handling unit (34) to the handling control unit (46) via the instrument control unit (48),
further comprising
- an enabling switch (54) arranged as a foot switch for activating the second operating mode, in which the robotic handling unit (34) is movable in a motor-driven manner in response to input commands at the input device (50) to move the instrument (36) in a motor-driven manner, ,
wherein the enabling switch (54) comprises two states (0, I), wherein a first state (0) is an initial state, and wherein a second state (I) is an activation state for the second operating mode,
wherein the enabling switch (54) comprises a third state (II), wherein the enabling switch (54), when activated, can be transferred starting from the first state (0) by applying an actuating force firstly into the second state (I) and then with an increased actuating force into the third state (II), and
wherein the control of the robotic handling unit (34) is blocked both in the first state (0) and in the third state (II) of the enabling switch (54).

2. The handling device according to claim 1, wherein the control device (44) is interposed between the input device (50) and the robotic handling unit (34) in terms of signals.

3. The handling device according to any one of claims 1 or 2, wherein the enabling switch (54) is directly connected to a handling control unit (46) that is associated with the robotic handling unit (34).

4. The handling device according to any one of claims 1 to 3, wherein in the second operating mode the control commands for the robotic handling unit (34) are transmitted via the instrument control unit (48), which forwards the control commands.

5. The handling device according to any one of claims 1 to 4, wherein the input device (50) is arranged as a multi-axis input device (50) and detects operating movements in the form of travel motions or pivot motions in at least two axes (156, 158, 160, 162, 164, 166), wherein the operating movements are converted into control commands for the robotic handling unit (34), and preferably wherein the robotic handling unit (34) comprises a multi-link kinematics (70) with a plurality of coupling links (72, 76, 80, 84), wherein the control device (44) is adapted to convert the movement instructions by interpolation into control commands for movement axes of the coupling links (72, 76, 80, 84).

6. The handling device according to any one of claims 1 to 5, wherein the control device (44) is adapted to store a current position and orientation of the instrument (36) and to recall it as required, and wherein the control device (44) is preferably adapted to store a plurality of predetermined positions and orientations of the instrument (36) in order to recall them as required.

7. The handling device according to any one of claims 1 to 6, wherein the control device (44) is adapted to control the handling unit (34) in such a way that the instrument (36) is pivotable about a virtual pivot axis, which is arranged parallel to the image capturing unit (118), by interpolated movement of the handling unit (34).

8. The handling device according to any one of claims 1 to 7, wherein the control device (44) is adapted to operate the robotic handling unit (34) in a direct control mode in order to move and align the instrument (36) in space, wherein operating commands can be generated at the robotic handling unit (34) by acting on an element (40) of the handling unit (34), which is adjacent to the instrument, and wherein the handling control unit (46) is adapted to control the robotic handling unit (34) in such a way that the instrument (36) follows the induced movement.

9. The handling device according to claim 8, wherein an operating element (270) is provided for controlling the robotic handling unit (34) in the direct control mode, and wherein the operating element (270) comprises a sensor (272) for generating an enabling signal for the direct control mode.

10. The handling device according to any one of claims 1 to 9, wherein the robotic handling unit (34) is mounted on a cart (24), and wherein the control of the robotic handling unit (34) for moving the instrument (36) is only enabled when the cart (24) is blocked.

11. The handling device according to any one of claims 1 to 10, wherein the interface (238) can be coupled to a plurality of input devices (244, 252), via which the robotic handling unit (34) can be controlled, wherein the input devices can be prioritized differently (244, 252).

12. A non-surgical method for controlling a handling device comprising a robotic handling unit (34) having an instrument holder (40) and an instrument (36) mounted thereon, the method comprising the steps of
- providing a robotic handling unit (34) comprising an instrument holder (40) and an input device (50),
- providing the instrument (36) at the instrument holder,
wherein the input device (50) is operable in a first operating mode for controlling the instrument (36) and in a second operating mode for controlling the robotic handling unit (34), and wherein the control of the instrument (36) and the robotic handling unit (34) is performed by using an input device (50) via a control device (44), to which the input device (50) can be coupled,
- activating the second operating mode, in which the robotic handling unit (34) is movable in a motor-driven manner in response to input commands at the input device (50), to move the instrument (36) in a motor-driven manner, by actuating an enabling switch (54) that is arranged as a foot switch,
wherein the enabling switch (54) comprises two states (0, I), wherein a first state (0) is an initial state, and wherein a second state (I) is an activation state for the second operating mode,
wherein the enabling switch (54) comprises a third state (II), wherein the enabling switch (54), when activated, can be transferred starting from the first state (0) by applying an actuating force firstly into the second state (I) and then with an increased actuating force into the third state (II),
wherein the control of the robotic handling unit (34) is blocked both in the first state (0) and in the third state (II) of the enabling switch (54),
wherein the control device (44) is arranged to be coupled to the instrument (36) and to the robotic handling unit (34),
wherein the control device (44) is interposed between the input device (50) and the instrument (36) supported by the instrument holder (40), and
wherein the control device (44) is adapted to provide in the second operating mode control commands, which have been detected via the input device (50), for the robotic handling unit (34) to the handling control unit (46) via the instrument control unit (48).

13. The method according to claim **12,** wherein the enabling switch (54) can be operated in the first state (0), the second state (I) and the third state (II) depending on an applied actuating force, wherein the second operating mode is deactivated in the first state (0) and in the third state (II), and wherein the second state (I) is interposed between the first state (0) and the third state (II) when the enabling switch (54) is switched.

## Revendications

1. Dispositif de manipulation médical qui comporte :
- un support d'instrument (40) pour recevoir un instrument (36), en particulier un instrument d'observation (38),
- une unité de manipulation robotisée (34) qui porte le support d'instrument (40),
- un dispositif de commande (44) comprenant une unité de commande de manipulation (46) pour commander l'unité de manipulation robotisée (34) et une unité de commande d'instrument (48) pour commander l'instrument (36),
le dispositif de commande (44) comportant une interface (238) destinée à au moins un dispositif d'entrée (50), et
- un dispositif d'entrée (50) pouvant être couplé au dispositif de commande (44), qui est couplé à l'interface (238),
le dispositif d'entrée (50) pouvant être utilisé dans un premier mode de fonctionnement pour commander l'instrument (36) et dans un deuxième mode de fonctionnement pour commander l'unité de manipulation robotisée (34),
le dispositif de commande (44) pouvant être couplé à l'instrument (36) et à l'unité de manipulation robotisée (34),
le dispositif de commande (44) étant connecté entre le dispositif d'entrée (50) et l'instrument (36) reçu sur le support d'instrument (40), et
le dispositif de commande (44) étant conçu, dans le deuxième mode de fonctionnement, pour fournir à l'unité de commande de manipulation (46), par l'intermédiaire de l'unité de commande d'instrument (48), des instructions de commande détectées par l'intermédiaire du dispositif d'entrée (50) et destinées à l'unité de manipulation robotisée (34), comportant en outre
- un commutateur de validation (54) conçu sous forme d'interrupteur à pied pour l'activation du deuxième mode de fonctionnement, dans lequel l'unité de manipulation robotisée (34) peut être déplacée au moyen d'un moteur en réaction à des instructions d'entrée sur le dispositif d'entrée (50) afin de déplacer l'instrument (36) au moyen d'un moteur,
le commutateur de validation (54) présentant deux états (0, I), un premier état (0) étant un état d'origine, et un deuxième état (I) étant un état d'activation pour le deuxième mode de fonctionnement,
le commutateur de validation (54) présentant un troisième état (II), le commutateur de validation (54) pouvant, lors de l'activation, être amené du premier état (0) au deuxième état (I) par application d'une force d'actionnement, puis au troisième état (II) par une force d'actionnement accrue, et
la commande de l'unité de manipulation robotisée (34) étant verrouillée dans le premier état (0) ainsi que dans le troisième état (II) du commutateur de validation (54).

2. Dispositif de manipulation selon la revendication 1, dans lequel le dispositif de commande (44) est interposé, du point de vue de l'échange de signaux, entre le dispositif d'entrée (50) et l'unité de manipulation robotisée (34).

3. Dispositif de manipulation selon l'une quelconque des revendications 1 ou 2, dans lequel le commutateur de validation (54) est directement relié à une unité de commande de manipulation (46) qui est associée à l'unité de manipulation robotisée (34).

4. Dispositif de manipulation selon l'une quelconque des revendications 1 à 3, dans lequel les instructions de commande destinées à l'unité de manipulation robotisée (34) sont transmises, dans le deuxième mode de fonctionnement, par l'intermédiaire de l'unité de commande d'instrument (48), laquelle retransmet les instructions de commande.

5. Dispositif de manipulation selon l'une quelconque des revendications 1 à 4, dans lequel le dispositif d'entrée (50) est configuré sous forme de dispositif d'entrée multiaxes (50) et détecte des mouvements de commande sous la forme de mouvements de poussée ou de mouvements de pivotement suivant au moins deux axes (156, 158, 160, 162, 164, 166), les mouvements de commande étant convertis en instructions de commande destinées à l'unité de manipulation robotisée (34), et de préférence, dans lequel l'unité de manipulation robotisée (34) comprend une cinématique multiéléments (70) ayant une pluralité d'éléments de couplage (72, 76, 80, 84), le dispositif de commande (44) étant conçu pour convertir, au moyen d'une interpolation, les instructions de mouvement en instructions de commande pour les axes de mouvement des éléments de couplage (72, 76, 80, 84).

6. Dispositif de manipulation selon l'une quelconque des revendications 1 à 5, dans lequel le dispositif de commande (44) est conçu pour stocker une position et une orientation actuelles de l'instrument (36) et les rappeler en cas de besoin, et de préférence, dans lequel le dispositif de commande (44) est conçu pour mémoriser une pluralité de positions et d'orientations prédéfinies de l'instrument (36) afin de les rappeler en cas de besoin.

7. Dispositif de manipulation selon l'une quelconque des revendications 1 à 6, dans lequel le dispositif de commande (44) est configuré pour commander l'unité de manipulation (34) de telle sorte que l'instrument (36) soit pivotant autour d'un axe de basculement virtuel agencé parallèlement au capteur d'image (118), par un mouvement interpolé de l'unité de manipulation (34).

8. Dispositif de manipulation selon l'une quelconque des revendications 1 à 7, dans lequel le dispositif de commande (44) est configuré pour faire fonctionner l'unité de manipulation robotisée (34) dans un mode de commande directe afin de déplacer et d'orienter l'instrument (36) dans l'espace, des instructions de commande pouvant être générées pour l'unité de manipulation robotisée (34) en agissant sur un élément (40) de l'unité de manipulation (34) qui est adjacent à l'instrument, et l'unité de commande de manipulation (46) étant configurée pour commander l'unité de manipulation robotisée (34) de telle sorte que l'instrument (36) suive le mouvement induit.

9. Dispositif de manipulation selon la revendication 8, dans lequel il est prévu un élément de commande (270) pour commander l'unité de manipulation robotisée (34) dans le mode de commande directe, et dans lequel l'élément de commande (270) comporte un capteur (272) pour générer un signal de validation destiné au mode de commande directe.

10. Dispositif de manipulation selon l'une quelconque des revendications 1 à 9, dans lequel l'unité de manipulation robotisée (34) est montée sur un chariot (24), et dans lequel une commande de l'unité de manipulation robotisée (34) pour le mouvement de l'instrument (36) n'est rendue possible que si le chariot (24) est bloqué.

11. Dispositif de manipulation selon l'une quelconque des revendications 1 à 10, dans lequel l'interface (238) peut être couplée à une pluralité de dispositifs d'entrée (244, 252) par l'intermédiaire desquels l'unité de manipulation robotisée (34) peut être commandée, les dispositifs d'entrée pouvant être hiérarchisés de manière différente (244, 252).

12. Procédé non chirurgical pour commander un dispositif de manipulation qui comprend une unité de manipulation robotisée (34) dotée d'un support d'instrument (40) et d'un instrument (36) reçu sur celui-ci, le procédé comprenant les étapes suivantes :
- la mise à disposition d'une unité de manipulation robotisée (34) comprenant un support d'instrument (40) et un dispositif d'entrée (50),
- la mise à disposition de l'instrument (36) sur le support d'instrument,
le dispositif d'entrée (50) pouvant être utilisé dans un premier mode de fonctionnement pour commander l'instrument (36) et dans un deuxième mode de fonctionnement pour commander l'unité de manipulation robotisée (34), et la commande de l'instrument (36) et de l'unité de manipulation robotisée (34) s'effectuant en utilisant le dispositif d'entrée (50) par l'intermédiaire d'un dispositif de commande (44) auquel le dispositif d'entrée (50) peut être couplé,
- l'activation du deuxième mode de fonctionnement, dans lequel l'unité de manipulation robotisée (34) peut être déplacée au moyen d'un moteur en réaction à des instructions d'entrée sur le dispositif d'entrée (50) afin de déplacer l'instrument (36) au moyen d'un moteur, par actionnement d'un commutateur de validation (54) configuré sous forme d'interrupteur à pied,
le commutateur de validation (54) présentant deux états (0, I), un premier état (0) étant un état d'origine, et un deuxième état (I) étant un état d'activation pour le deuxième mode de fonctionnement,
le commutateur de validation (54) présentant un troisième état (II), le commutateur de validation (54) pouvant, lors de l'activation, être amené du premier état (0) au deuxième état (I) par application d'une force d'actionnement, puis au troisième état (II) par une force d'actionnement accrue,
la commande de l'unité de manipulation robotisée (34) étant verrouillée dans le premier état (0) ainsi que dans le troisième état (II) du commutateur de validation (54),
le dispositif de commande (44) pouvant être couplé à l'instrument (36) et à l'unité de manipulation robotisée (34),
le dispositif de commande (44) étant connecté entre le dispositif d'entrée (50) et l'instrument (36) reçu sur le support d'instrument (40), et
le dispositif de commande (44) étant conçu, dans le deuxième mode de fonctionnement, pour fournir à l'unité de commande de manipulation (46), par l'intermédiaire de l'unité de commande d'instrument (48), des instructions de commande détectées par l'intermédiaire du dispositif d'entrée (50) et destinées à l'unité de manipulation robotisée (34).

13. Procédé selon la revendication 12, dans lequel le commutateur de validation (54) peut être actionné en fonction d'une force d'actionnement appliquée dans le premier état (0), le deuxième état (I) et le troisième état (II), dans lequel, dans le premier état (0) et dans le troisième état (II), le deuxième mode de fonctionnement est désactivé, et dans lequel, lors de la commutation du commutateur de validation (54), le deuxième état (I) est interposé entre le premier état (0) et le troisième état (II).
